# EUROPEAN PATENT SPECIFICATION

(11) **EP 0 514 534 B1**
(45) Date of publication and mention of the grant of the patent: **01.04.1998**
(21) Application number: 92902641.7
(22) Date of filing: 06.12.1991
(51) Int. Cl.: C12Q 1/68

(54) **METHODS AND REAGENTS FOR HLA DRBETA DNA TYPING**
VERFAHREN UND REAGENZIEN FÜR DIE KARAKTERISIERUNG VON HLA-DRBETA-DNS
PROCEDES ET REACTIFS POUR TYPER L'ADN DE HLA DRBETA

(30) Priority: 06.12.1990 US 623098
(43) Date of publication of application: 25.11.1992
(73) Proprietor: F. HOFFMANN-LA ROCHE AG, 4002 Basel (CH)
(72) Inventor: ERLICH, Henry, A., Oakland, CA 94602 (US); BEGOVICH, Ann, B., El Cerrito, CA 94350 (US); BUGAWAN, Teodorica, San Leandro, CA 94579 (US); GRIFFITH, Robert, L., Belmont, CA 94002 (US); SCHARF, Stephen, J., Berkeley, CA 94709 (US); APPLE, Raymond, J., San Francisco, CA 94116 (US)
(74) Representative: Notegen, Eric-André
(86) International application number: US9109294
(87) International publication number: WO9210589

(56) References cited:
- EP-A- 0 237 362
- WO-A-89/04875
- WO-A-89/11547
- IMMUNOGENETICS vol. 30, September 1989, NEW YORK US pages 208 - 213; L. FUGGER ET AL.: 'Typing for HLA-DPB1*03 and HLA-DPB1*06 using allele-specific DNA in vitro amplification and allele-specific oligonucleotide probes. Detection of "new" DPB1*06 variants'

## Description

### Background of the Invention

### Field of the Invention

The present invention provides methods and reagents for DNA typing HLA DRbeta (DRB) nucleic acids. The invention enables one to type homozygous or heterozygous samples from a variety of sources, including samples comprising RNA or cDNA templates, and to detect allelic variants not distinguishable by present serological, cellular, or biochemical methods. The present typing system facilitates typing tissue for transplantation, determining individual identity, and identifying disease susceptible individuals. The invention therefore has applications in the field of medicine generally and medical research and diagnostics specifically, the field of forensic science, and the field of molecular biology.

### Description of Related Art

The HLA Class II proteins HLA DR, HLA DQ, and HLA DP are encoded by genes in the major histocompatibility complex (MHC) region on the short arm of human chromosome 6. The Class II proteins are heterodimeric glycoproteins consisting of an approximately 34 kD alpha chain and an approximately 29 kD beta chain. The Class II proteins are expressed on the cell surface of macrophages, B-cells, activated T-cells, and other cell types and are involved in binding and presenting antigen to helper T-lymphocytes. See the article entitled "Structure, function, and genetics of the Human Class II molecules" by Giles and Capra, 1985, Adv. Immunol. 37:1. In addition, the Class II proteins influence specific immune responsiveness by determining the repertoire of expressed T-cell receptors in mature T-lymphocytes. For a general review of the HLA Class II genes and proteins, see the article entitled "Structure, sequence and polymorphism in the HLA D region" by Trowsdale et al., 1985, Immunol. Rev. 85:5.

The Class II alpha and beta chains are encoded by separate genes, and the DP, DQ, and DR genes are located in separate regions of the MHC. Methods for HLA DP typing using a panel of probes specific for variant segments of DPalpha and DPbeta genes have been disclosed in WO 89/11547. Fugger et al. (Immunogenetics 30, 208-213 (1989) have reported on the typing for HLA-DPB1*03 and HLA-DPB1*06 using allele specific DNA in vitro amplification and allele specific oligonucleotide probes and the detection of "new" DPB1*06 variants. In the DR region, a single DRA locus, or gene, encodes the non-polymorphic DRalpha chain, but five different DRB loci, termed DRB1, DRB2 (now known as DRB6), DRB3, DRB4, and DRB5, encode the polymorphic DRbeta chain. Some loci are present only on certain haplotypes (such as DRB5 on the DR2 haplotype); in addition, the number of expressed DRB genes also varies between haplotypes. See the article entitled "HLA-DRbeta genes vary in number between different DR-specificities, whereas the number of DQbeta genes is constant" by Bohme et al . 1985, J. Immunol. 135:2149.

The number of distinct DRB 1 alleles identified is continually increasing. The 1989 report from the WHO Nomenclature Committee for factors of the HLA system identified 34 distinct DRB 1 alleles; these alleles are thought to express the serological DR specificities DR1 to DRw18. (see the article entitled "Nomenclature for factors of the HLA system, 1989" by the WHO Nomenclature Committee, 1990, Immunogenetics 31:131-140). By the 1990 report, the number of DRB 1 alleles recognized had risen to 45 (see the article entitled "Nomenclature for factors of the HLA system, 1990" by the WHO Nomenclature Committee, 1991, Immunogenetics 33:301-309). The present invention provides the sequences of several newly discovered alleles.

The alleles of the DRB2 locus (now termed the DRB6 locus), which are present on DR1, DR2, and DRw10 haplotypes, are apparently not expressed. See the article entitled "Analysis of isotypic and allotypic sequence variation in the HLA DRB region using the in vitro enzymatic amplification of specific DNA segments" by Erlich et al., 1989, in Immunobiology of HLA (Dupont ed., Springer-Verlag, New York).

The alleles of the DRB3 locus, which is thought to encode the supertypic specificity DRw52 (DRw52a, DRw52b, and DRw52c), are present on the DR3, DRw6, DRw11, DRw12, DRw13, DRw14, DRw17, and DRw18 haplotypes.

The DRB4 locus, which has a single allele, encodes the DRw53 supertypic specificity and is present only on the DR4, DR7, and DRw9 haplotypes. See the article entitled "Structural relationships between the DRbetal and DRbeta2 subunits in DR4, 7, and w9 haplotypes and the DRw53 (MT3) specificity" by Matsuyama et al., 1986, J. Immunol. 137:934.

The alleles of the DRB5 locus are present only on DR2 haplotypes. See the article entitled "Analysis of isotypic and allotypic sequence variation in the HLA DRB region using the in vitro enzymatic amplification of specific DNA segments," supra.

Polymorphism of the Class II DR antigens (proteins) is currently typed with allosera obtained from multiparous women in a microcytotoxicity assay on purified B lymphocytes. In addition, cellular typing protocols capable of greater specificity and based on either the specificity of alloreactive T-cell clones or the proliferative response of T-cell cultures to stimulation by homozygous typing cells (HTCs) have been developed.

These cellular-based analyses define the Dw specificities that further subdivide many of the serologically defined antigens, e.g., the five Dw subtypes of DR4. See the article entitled "Sequence polymorphism of HLA DRbeta I alleles relating to T cell recognized determinants" by Cairns et al., 1985, Nature 317:166. Both the serological and cellular assay procedures, however, are difficult and time-consuming. HLA DR DNA typing protocols based on restriction fragment length polymorphisms (RFLP) have also been developed. See U.S. Patent No. 4,582,788. However, these RFLP-based analyses require large amounts of high molecular weight DNA, are labor intensive, and the limited number of informative restriction enzymes in turn limits the results obtained.

The advent of the polymerase chain reaction (PCR) has facilitated the analysis and manipulation of complex genomic DNA. The PCR process enables one to amplify a specific sequence of nucleic acid starting from a very complex mixture of nucleic acids and is more fully described in U.S. Patent Nos. 4,683,195; 4,683,202; 4,889,818; and 4,965,188, and European Patent Publication Nos. 237,362 and 258,017.

The PCR process has also facilitated typing the Class II HLA DNA of an individual. Scientists have studied the polymorphic second exon of DRB loci in genomic DNA by designing oligonucleotide primers and using those primers to amplify the sequences of interest. See the article entitled "Sequence analysis of the HLA DRB and HLA DQB loci from three Pemphigus vulgaris patients" by Scharf et al., 1988, Hum. Immunol. 22:61.

When the PCR primers contain restriction enzyme recognition sequences, the amplified DNA can be cloned directly into sequencing vectors, and the nucleotide sequence of the amplification product can be readily determined. See the article entitled "Direct cloning and sequence analysis of enzymatically amplified genomic sequences" by Scharf et al., 1986, Hum. Immunol. 233:1076.

The amplified DNA can also be studied by detection methods that employ sequence-specific oligonucleotide (SSO) probes. See the article entitled "Analysis of enzymatically amplified beta-globin and HLA DQalpha DNA with allele-specific oligonucleotide probes" by Saiki et al., 1986, Nature 324:163.

Despite these advances, the complexity of the HLA DRbeta genes has prevented the development of an informative and efficient means for determining the HLA DRbeta DNA type of an individual. The present invention meets the need for an efficient, informative DRbeta DNA typing method by providing novel processes and reagents. These novel processes and reagents have in turn led to the discovery of previously unknown DRbeta alleles, which can also be typed and identified by the present method.

The present typing system can be used to type cDNA synthesized from mRNA and to type and study the expression of DRB genes in tissues, transgenic systems, disease states, and cells lines. Cells that do not express the DR antigens or show unusual seroreactivity, such as tumor cells, can be readily typed. Moreover, samples from unusual sources, e.g., ancient DNAs or forensic samples, can be typed, even when the DNA sample is degraded or when only very small quantities are available for analysis.

Because PCR can amplify a fragment of target DNA over a million-fold, and because the present system can employ PCR-generated nucleic acid, radioactively labeled probes are not necessary, and nonisotopic SSOs covalently coupled to horseradish peroxidase (HRP) provide sufficient sensitivity for detection. The presence of the specifically bound HRP-labeled probes of the invention can be detected in a simple dot-blot format by chromogenic dye or chemiluminescent substrates in a matter of minutes.

### Summary of the Invention

The present invention provides amplification and detection methods, generic and allele or group specific amplification primers, nonisotopic sequence specific oligonucleotide (SSO) probes, including probes for identifying previously unknown alleles at the DRB 1 locus, and kits for practicing the methods, that together provide a rapid, simple and precise system for typing the HLA DRB alleles, including those that cannot be distinguished by serological methods.

In one aspect, the present invention provides a method for determining the DRbeta DNA type of nucleic acid in a sample, which method comprises: (a) amplifying any nucleic acids in the sample that contain a DRbeta gene second exon using the primer pair GH46 (SEQ ID NO:67) and GH50 (SEQ ID NO:68); (b) hybridizing said nucleic acid amplified in step (a) to a first panel of oligonucleotide probes under conditions such that said probes hybridize only to exactly complementary sequences greater than ten nucleotides in length; (c) amplifying a specific subset of nucleic acids in the sample that contain DRbeta gene second exon sequences using the primer pairs GH46 (SEQ ID NO:67) and CRX37 (SEQ ID NO:73) or GH46 (SEQ ID NO:67) and DRB30 (SEQ ID NO:107); (d) hybridizing said nucleic acid amplified in step (c) with a second panel of oligonucleotide probes under conditions such that said probes hybridize only to exactly complementary sequences greater than ten nucleotides in length; and (e) determining from the pattern of probe hybridization in steps (b) and (d) the DRbeta alleles from which the DRbeta DNA in said sample originates. In the preferred method of the present invention steps (a) and (c) are carried out by a polymerase chain reaction. The preferred first and second panel of oligonucleotide probes hybridize to DRbeta gene second exon sequences encoding amino acid residues 9 to 16, amino acid residues 25 to 34, amino acid residues 67 to 74, and amino acid residue 86.

In another aspect the present invention provides kits for performing the above mentioned method by using a polymerase chain reaction.

### Brief Description of the Figures

Figure 1 shows the results of DRB generic and DRB 1 specific amplification and detection; see Example 2.

Figure 2 shows the results of DRB1 specific amplification and detection; see Example 2.

Figure 3 shows the results of HLA DRB DNA typing of the classical serological types DR1 through DR10; see Example 3.

Figure 4 shows the results of HLA DRB DNA typing to subtype cell lines with the DR4 serological specificity; see Example 5.

Figure 5 shows the results of HLA DRB DNA typing to subtype the DR3, DR5, DRw6, and DRw8 specificities; see Example 6.

Figure 6 shows the tabulation of probe hybridization results to determine DRB type; see Example 6.

Figure 7 shows the results of HLA DRB DNA typing of a number of different cell lines; see Examples 6 and 7.

Figure 8 shows the HLA DRB DNA subtyping of DR3 cell lines; see Example 7.

Figure 9 shows the tabulation of probe hybridization results to determine the HLA DRB DNA type of heterozygous and other unusual samples; see Example 7.

Figure 10 shows the tabulation of probe hybridization results to determine the HLA DRB DNA type; see Example 9.

Figure 11, 12, and 13 show the tabulation of probe hybridization results to determine DRB allele type; see Example 9.

### Detailed Description of the Invention

The present invention provides an HLA DRB typing system and sequence specific oligonucleotide probes (SSOs) for analyzing DRB alleles. The invention can be used to type heterozygous samples from a variety of sources, including cDNA templates, and can be used to detect allelic variants not distinguishable by serological methods. This typing system can utilize a dot-blot format that is simple and rapid to perform, produces detectable signals in minutes, and will prove valuable for tissue typing and determining individual identity and disease susceptibility.

The number of distinct DRB1 alleles identified is continually increasing. The 1989 WHO Nomenclature Committee report listed 34 DRB 1 alleles; this set of alleles is herein referred to as the 1989 allele set. The 1990 WHO Nomenclature Committee report listed 45 alleles; this set of alleles is herein referred to as the 1990 allele set. In addition, the present invention provides the sequences of several newly discovered alleles.

The diversity of DRB loci, the most highly polymorphic of the HLA Class II loci, and the large number of alleles at these loci in the population make difficult the process of identifying the particular DRB loci and alleles from which a nucleic acid in a sample originates. The present invention allows one to make this determination with great specificity and so can be used to identify the particular individual from whom a sample was taken. This discrimination power in turn leads to the applications of the invention in the field of forensic science.

Because PCR (or other amplification processes) can be used to amplify very small amounts of DNA (or degraded DNA), the present invention can be used to type HLA DRB DNA from unusual sources, such as a buccal swab, a single hair, and even DNA from preserved ancient specimens. With the latter samples, analysis of the alleles from prehistoric sources, e.g., early hominids, is possible. For purposes of the present invention, "amplification" is defined by any process that increases the amount of target nucleic acid in a sample by means of nucleic acid replication or transcription.

The present method can be used for DRB DNA typing of cells that do not express the DRB genes. The method is also suitable, however, for DRB DNA typing of cDNA synthesized from DRB mRNA. The latter method facilitates the study of the expression of HLA DRB in various cell lines or tissues and can be used to determine if there is an association between HLA DRB expression and susceptibility to transformation, autoimmunity, or other health conditions.

The research potential of the present invention should in no way obscure the immediate clinical applications. The genes and gene products of the MHC play a central role in the immunological state of an individual, and particular MHC gene products are associated with disease resistance and susceptibility. Because the present invention allows the determination of the MHC DRB gene products in a sample, the invention also has applications in the field of medicine, particularly for medical diagnostic methods.

The discriminating power of this system will be valuable in typing potential transplantation donors, where very precise HLA DRB matching appears to be critical in minimizing risk of rejection or graft versus host disease. See the article entitled "Mixed lymphocyte reactions for individuals with phenotypic identity for specific HLA B, DR determinants: The role of linkage disequilibrium and of specific DR and other Class II determinants" by Pollack et al., 1983, J. Clin. Immunol. 3:341. Disease susceptibility studies have shown that single nucleotide differences in the DRB alleles can be medically significant. See the articles entitled "Specific HLA-DQB and HLA-DRB 1 alleles confer susceptibility to Pemphigus vulgans" by Scharf et al., 1989, Proc. Natl. Acad. Sci. USA 86:6215; and "Identification of HLA-Dw14 genes in DR4+ Rheumatoid Arthritis" by Nepom et al., 1986, Lancet page 1002.

In addition to the above benefits, the present invention also provides methods for identifying previously unknown DR alleles, and related primers, probes, and methods for the identification of any DRB allele. Unusual patterns of SSO probe hybridization using this typing system identify new alleles, as exemplified by the DNA typing of the "Beguit" cell line (see Example 7). This cell line showed an unusual pattern of probe hybridization at the DRB 1 locus, revealing a previously unreported sequence, now designated DRB1*1103 (SEQ ID NO: 27).

In similar fashion, analysis by the present method of the DRB 1 alleles in a patient with Lyme disease revealed a new pattern of probe hybridization. DRB 1 allele sequences were cloned from genomic DNA of the patient and sequenced. The sequence of one DRB 1 allele of the patient is different from any previously reported DRB 1 allele and was originally designated as DR'LY10' (also DRB1*LY10). The other allele in the patient was DRB1*0402. The DR'LY10' allele has regions of sequences similar to DRB1*0801, *0802, *0803, and *1201 at the 5'-end and to *1401 at the 3'-end. The present invention provides probes for distinguishing the DR'LY10' allele from other DRB alleles. The DR'LY10' allele is now designated as DRB1*1404 (SEQ ID NO: 40).

Other newly discovered alleles of the DRB 1 locus are DRB1*1305 (SEQ ID NO: 36), originally designated as DR'PEV', DRB1*1303 (SEQ ID NO: 34), and DRB1*1105 (SEQ ID NO: 29), originally designated as DRB1*BUGS. Each was discovered when analysis by the methods of the present invention revealed a new pattern of probe hybridization. Subsequent sequence analysis of each allele revealed the sequence variation causing the novel hybridization pattern. The present invention provides primers and probes for distinguishing the newly discovered alleles from other DRB alleles.

The present invention also provides kits for making practice of the present DRB typing method more convenient. One type of kit includes both amplification and typing reagents. Another kit contains only one or more DRB probes of the invention. In either kit, the probes can be labeled or unlabeled or attached to a solid support. The primers, if present in the kit, can also be labeled to facilitate detection, i.e., to bind a signal development reagent or for immobilization. The kits can also contain reagents that facilitate detection of probe hybridization, i.e., the chromogenic substrate TMB and streptavidin-linked horseradish peroxidase. In brief, the reagents useful in practicing the present method can be packaged in any configuration that promotes utilization of the invention.

The nucleotide sequence of the second exon of each allele along with the encoded amino acid sequence are provided in the Sequence Listing section. Table 1 and 2, below, presents equivalent nucleotide sequence information for the alleles in the 1989 allele set, but in a manner which facilitates comparison. Table 1 also shows the nucleic acid sequence and the encoded amino acid sequence in both three and one letter codes for allele DRB*0101. Similarly, Table 3 presents equivalent amino acid sequence information, but using one letter amino acid codes. The sequence identification number of each allele is shown below. All alleles with the exception of DRB1*1503, DRB1*0303, and DRB1*1105 are listed in the 1990 WHO Nomenclature Committee report, supra (1990 allele set). For the sequence of DBR1*1503, see also Demopolos et al., 1991, Human Immunology 30:41-44.

The nucleotide sequences of alleles which do not appear in Tables 1, 2, and 3 are provided in Table 9 below in addition to the Sequence Listing section.

| Allele | SEQ ID NO: | Allele | SEQ ID NO: |
|---|---|---|---|
| DRB1*0101: | SEQ ID NO:1 | DRB1*1201: | SEQ ID NO:30 |
| DRB1*0102: | SEQ ID NO:2 | DRB1*1202: | SEQ ID NO:31 |
| DRB1*0103: | SEQ ID NO:3 | DRB1*1301: | SEQ ID NO:32 |
| DRB1*0301: | SEQ ID NO:4 | DRB1*1302: | SEQ ID NO:33 |
| DRB1*0302: | SEQ ID NO:5 | DRB1*1303: | SEQ ID NO:34 |
| DRB1*0303: | SEQ ID NO:6 | DRB1*1304: | SEQ ID NO:35 |
| DRB1*0401: | SEQ ID NO:7 | DRB1*1305: | SEQ ID NO:36 |
| DRB1*0402: | SEQ ID NO:8 | DRB1*1401: | SEQ ID NO:37 |
| DRB1*0403: | SEQ ID NO:9 | DRB1*1402: | SEQ ID NO:38 |
| DRB1*0404: | SEQ ID NO:10 | DRB1*1403: | SEQ ID NO:39 |
| DRB1*0405: | SEQ ID NO:11 | DRB1*1404: | SEQ ID NO:40 |
| DRB1*0406: | SEQ ID NO:12 | DRB1*1405: | SEQ ID NO:41 |
| DRB1*0407: | SEQ ID NO:13 | DRB1*1501: | SEQ ID NO:42 |
| DRB1*0408: | SEQ ID NO:14 | DRB1*1502: | SEQ ID NO:43 |
| DRB1*0409: | SEQ ID NO:15 | DRB1*1503: | SEQ ID NO:44 |
| DRB1*0410: | SEQ ID NO:16 | DRB1*1601: | SEQ ID NO:45 |
| DRB1*0411: | SEQ ID NO:17 | DRB1*1602: | SEQ ID NO:46 |
| DRB1*0701: | SEQ ID NO:18 | DRB2*0101: | SEQ ID NO:47 |
| DRB1*0801: | SEQ ID NO:19 | DRB3*0101: | SEQ ID NO:48 |
| DRB1*0802: | SEQ ID NO:20 | DRB3*0201: | SEQ ID NO:49 |
| DRB1*0803: | SEQ ID NO:21 | DRB3*0202: | SEQ ID NO:316 |
| DRB1*0804: | SEQ ID NO:22 | DRB3*0301: | SEQ ID NO:50 |
| DRB1*0901: | SEQ ID NO:23 | DRB4*0101: | SEQ ID NO:51 |
| DRB1*1001: | SEQ ID NO:24 | DRB5*0101: | SEQ ID NO:52 |
| DRB1*1101: | SEQ ID NO:25 | DRB5*0102: | SEQ ID NO:53 |
| DRB1*1102: | SEQ ID NO:26 | DRB5*0201: | SEQ ID NO:54 |
| DRB1*1103: | SEQ ID NO:27 | DRB5*0202: | SEQ ID NO:55 |
| DRB1*1104: | SEQ ID NO:28 | | |
| DRB1*1105: | SEQ ID NO:29 | | |

In Tables 1, 2, and 3, below, all sequences except the recently identified "PEV" and "Ly10" alleles are listed in the 1989 WHO HLA nomenclature report, supra, and the DRB1*0101 nucleotide sequence serves as the consensus sequence. The inferred amino acid sequence for the consensus sequence is written in one and three letter code above the nucleotide sequence. Sequence homology is indicated by dashed lines, and letters indicate polymorphic bases. The designated SSO probes and primers written at the right end of the alignments in the tables are the same sense as, or complementary to, the regions of sequences boxed. Where two names appear at the end of an alignment, the left-most name refers to the left-most box, the right-most name refers to the right-most box. The probe CRX12 hybridizes to the region shown in all DRB alleles.

Table 1, in three parts designated A, B, and C, shows the nucleotide sequence of 35 DRB1 alleles corresponding to the DR specificities DR1 to DRw18.

Table 2 shows the nucleotide sequence alignments for the alleles of the DRB2, DRB3, DRB4, and DRB5 loci. The major regions of sequence polymorphism for the DRB1 alleles are localized to amino acid positions 9-16, 25-34, 67-74, and 86; the remainder of the second exon sequence is relatively invariant.

Table 3 shows the inferred amino acid sequence alignment encoded by the DRB alleles. Analysis of the amino acid sequences reveals a complex but restricted pattern of polymorphism with particular polymorphic sequences found in several different alleles. However, some polymorphic sequences are unique for each allele. The DR1, DR2, DR4, DR7, DRw9, and DRw10 alleles each have unique polymorphic sequences at the first hypervariable region (positions 9 to 16) of the DRB 1 locus that can be used to determine the serological DR specificities by SSO typing. By contrast, the DR3, DRw11, and DRw6 alleles share a polymorphic epitope, "YSTS," and cannot be distinguished by a probe for this region alone but can be distinguished by the polymorphisms at other positions in each allele. Similarly, DRw8 and DRw12 also cannot be distinguished in this region.

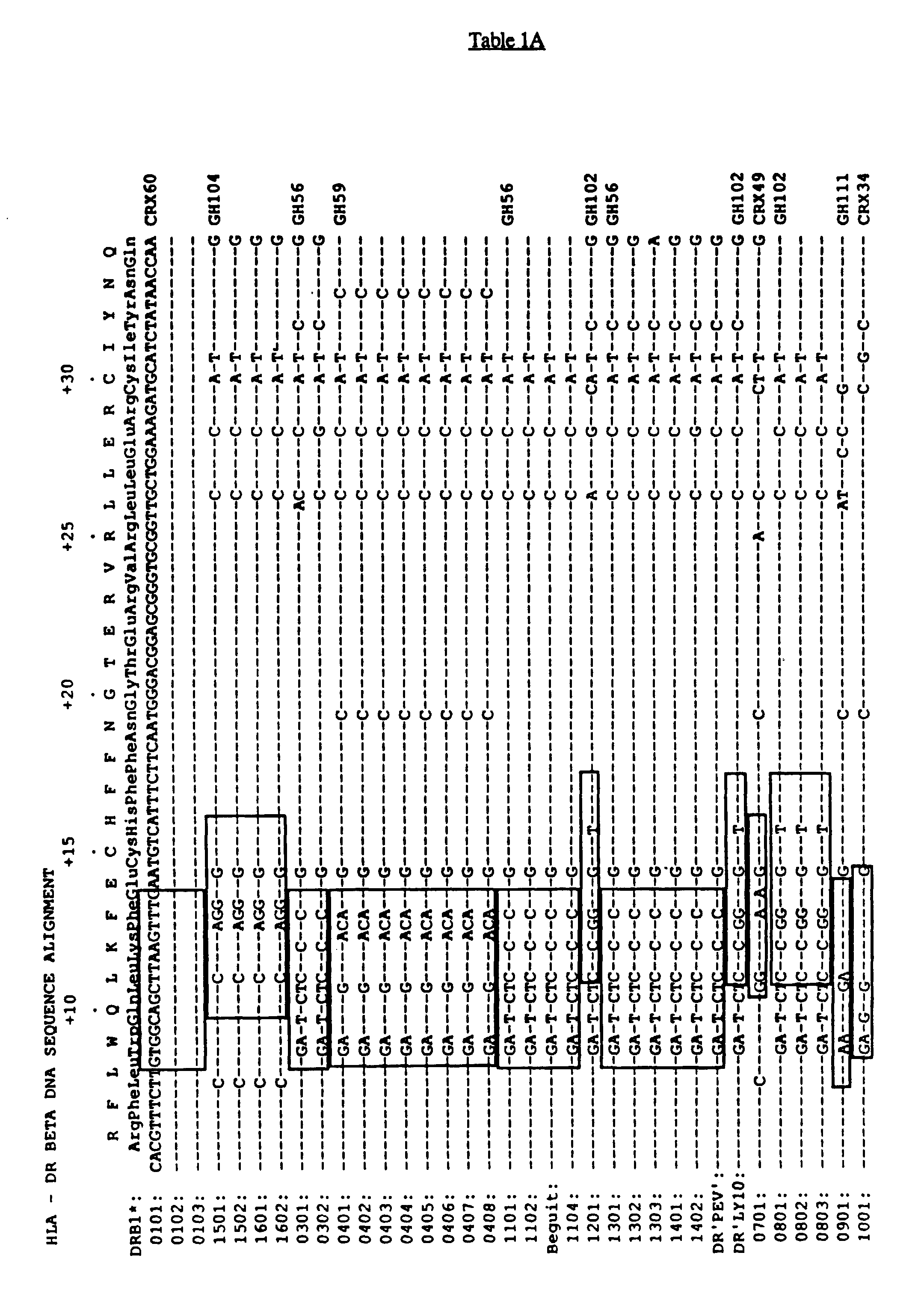

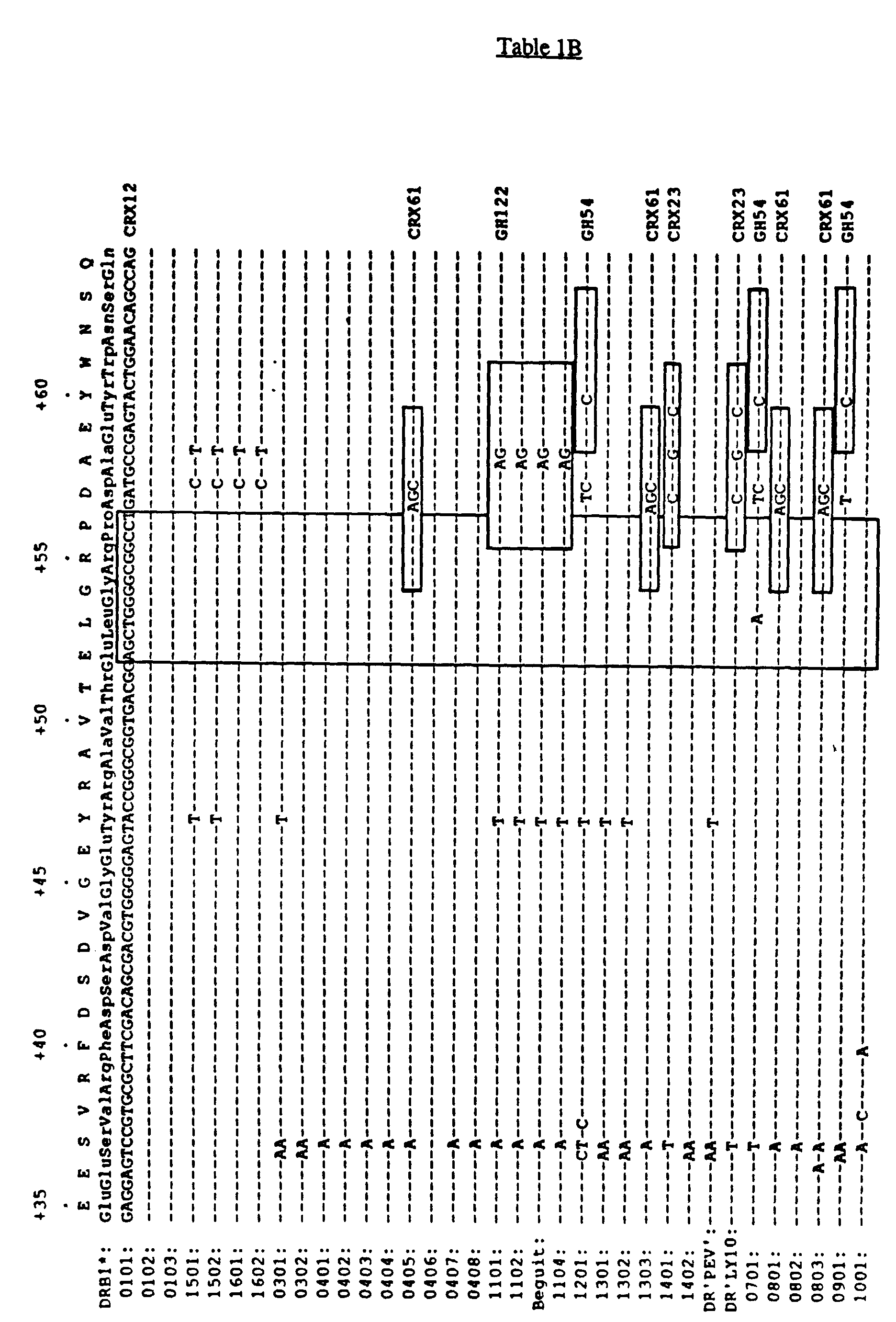

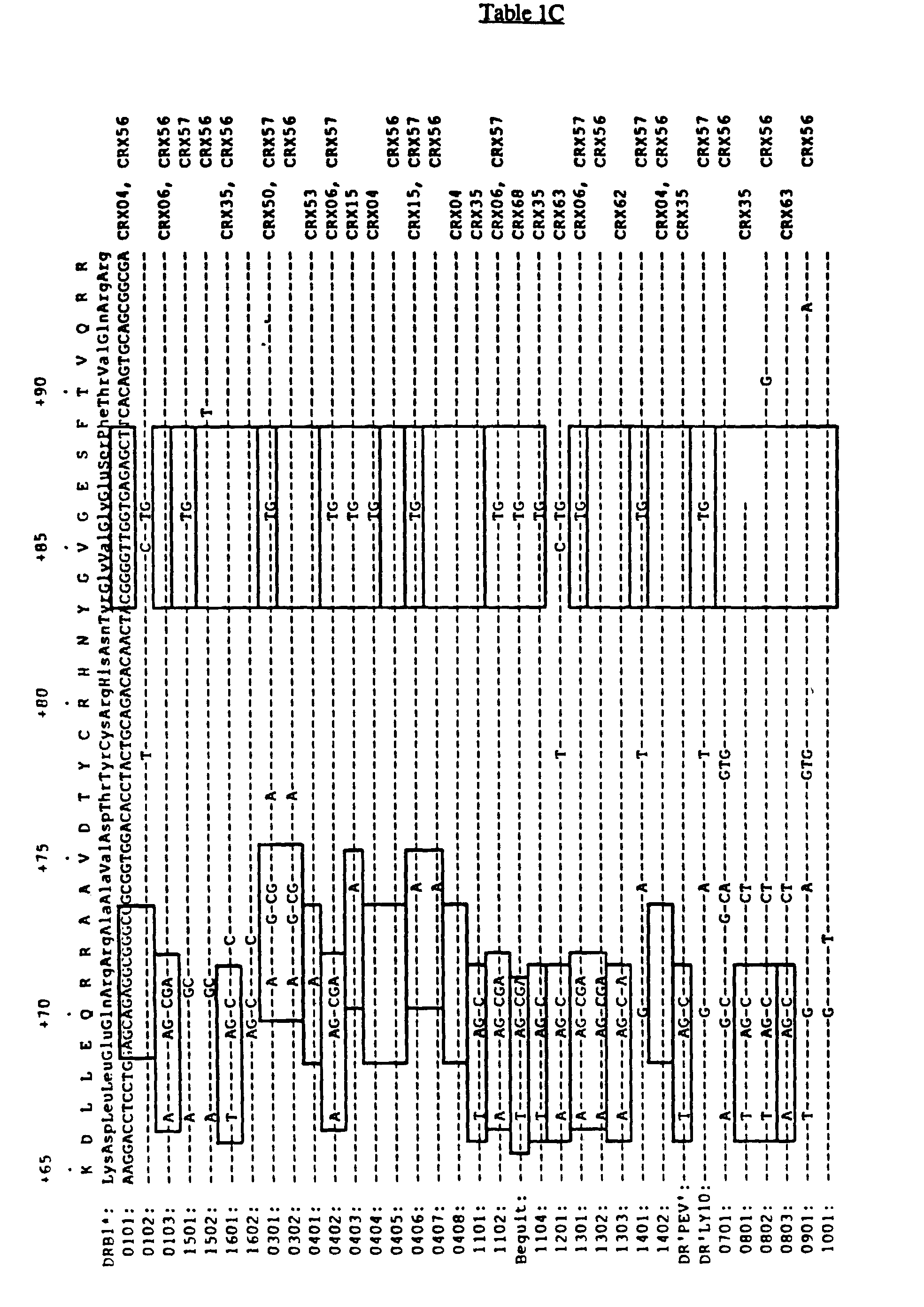

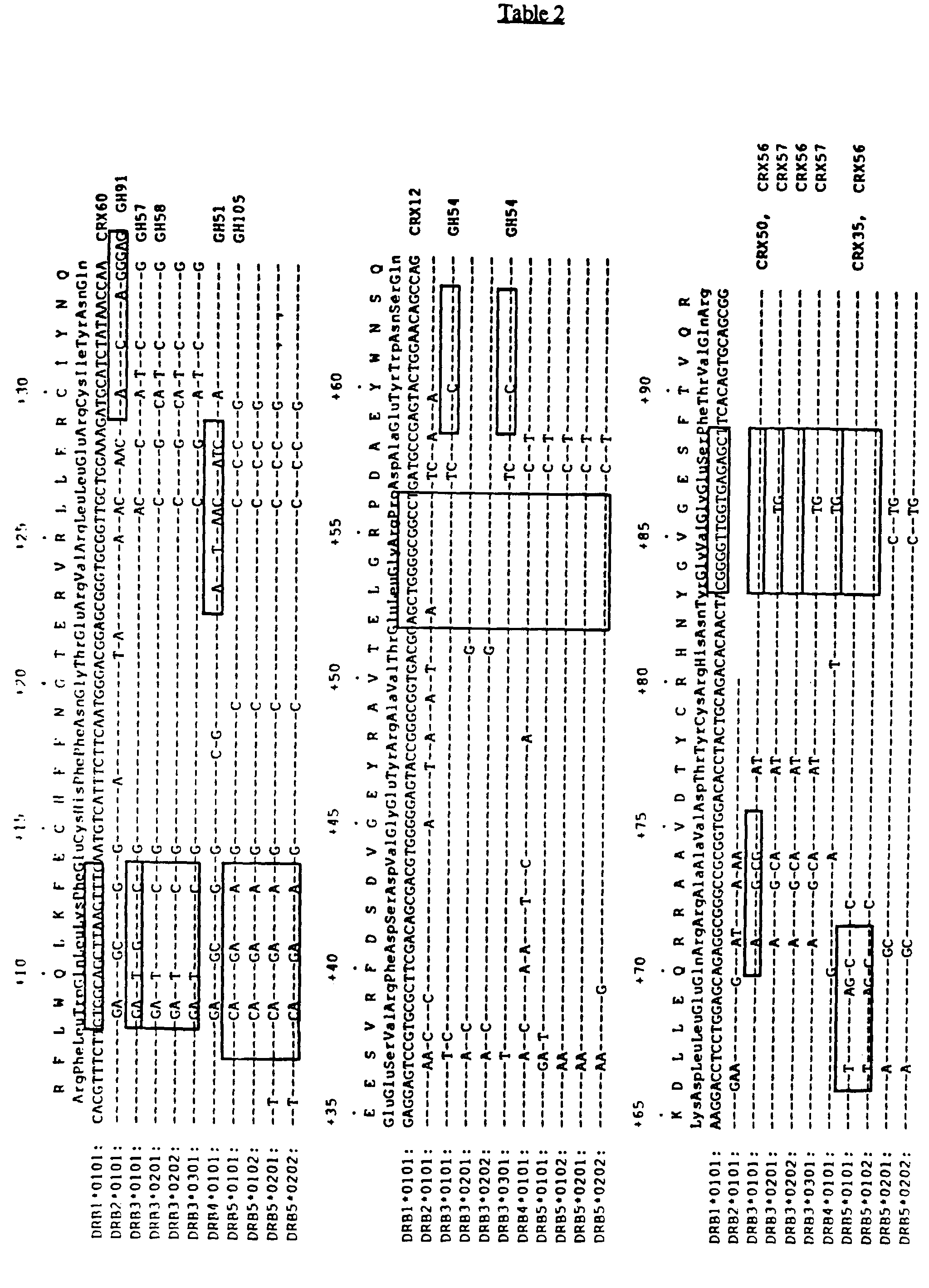

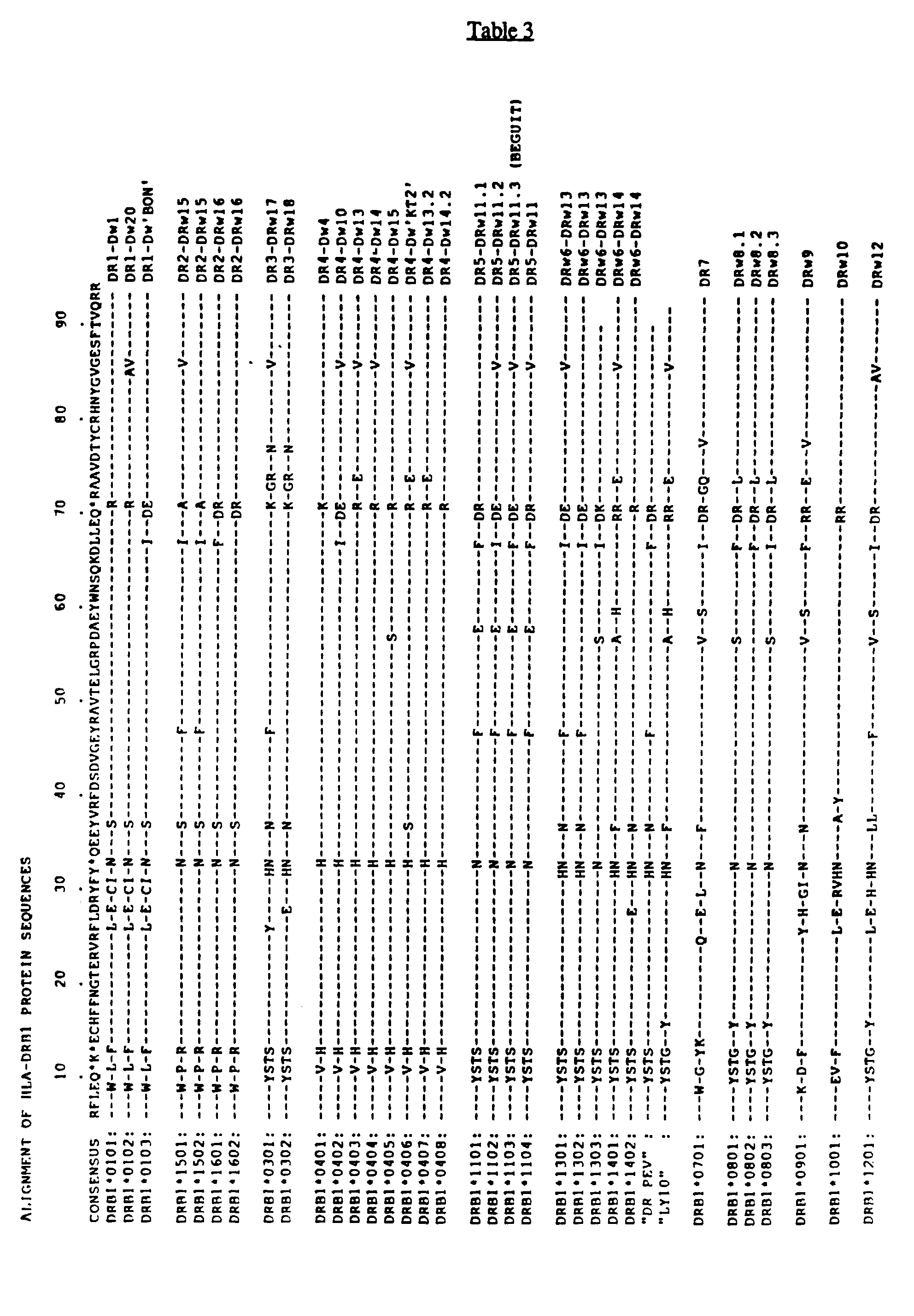

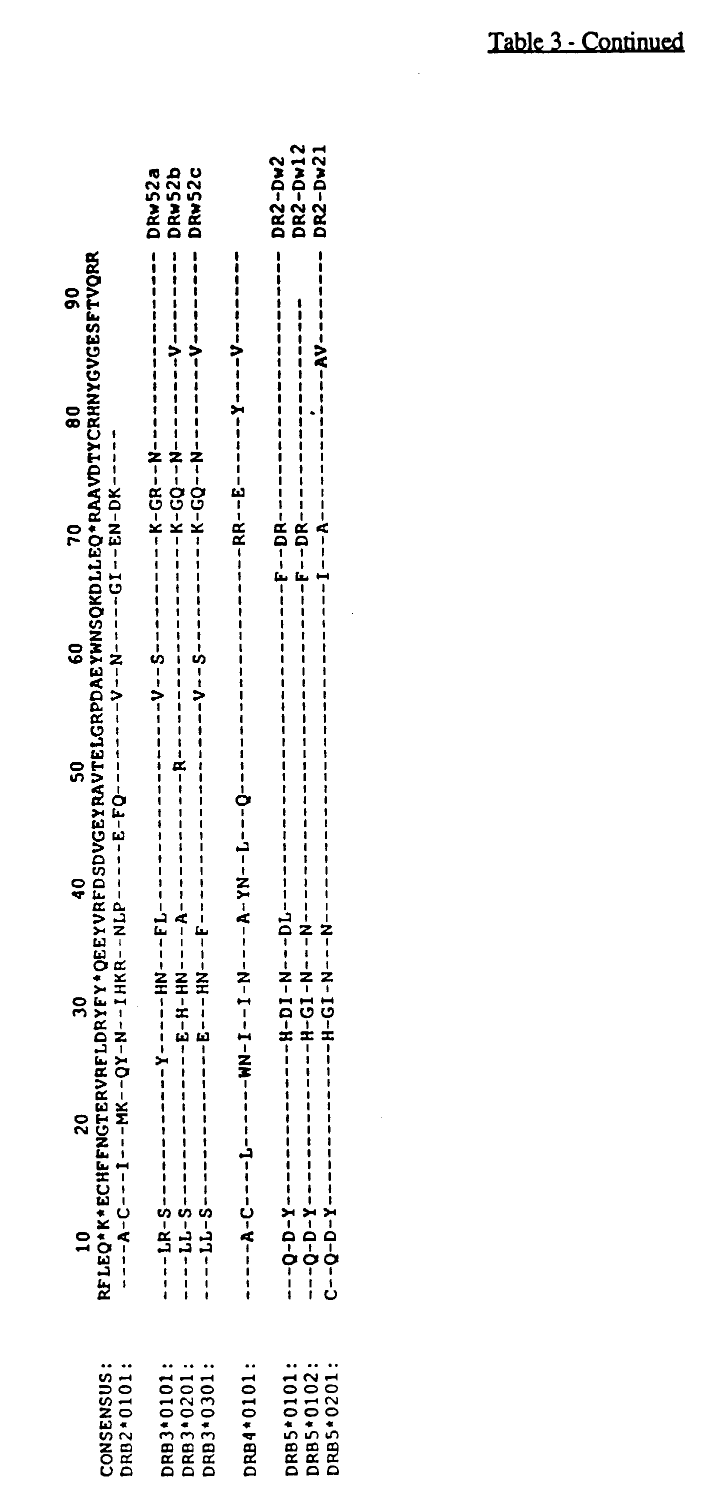

The classical serologically defined DR types (except DR3 and DRw6) can be distinguished by amplifying with the generic DRB primers GH46 (SEQ ID NO: 67) and GH50 (SEQ ID NO: 68) and analyzing the amplification products with the first panel of probes, shown in Table 4. The allele specificities shown in Table 4 are with respect to the 1989 allele set. For purposes of the present invention, "generic primers" are PCR primers that hybridize to DRB gene second exon sequences and can be used to amplify any allele of any DRB locus. Because of the hybridization site of GH50, one cannot probe for polymorphisms at position 86 (referring to the amino acid sequence) of the second exon when the product of amplification was generated with primer GH50. An alternate primer that can be used to generate products than can be analyzed for position 86 polymorphisms is DRB151 (SEQ ID NO: 227),

5'-CCGAATTCGCCGCTGCACTGTGAAGCT-3'.

**Table 4**

| First Panel of HLA DRB Typing SSO Probes HLA DRB | | | | |
|---|---|---|---|---|
| Probe | SEQ ID NO: | Epitope | Alleles(DRB1*) | Wash (SSPE. °C.) |
| CRX60 | SEQ ID NO: 79 | "W-L-F" | 0101,0102,0103 | 0.4X, 42 |
| GH105 | SEQ ID NO: 91 | "Q-D-Y" | DRB5 | 0.1X, 42 |
| GH104 | SEQ ID NO: 90 | "W-P-R" | 1501, 1502, 1601, 1602 | 0.2X, 42 |
| GH59 | SEQ ID NO: 87 | "V-H" | 0401-0408 | 0.2X, 42, 20' |
| CRX06 | SEQ ID NO:61 | "I-DE" | 0103,0402,1102, 1301, 1302 | 0.1X,42 |
| GH122 | SEQ ID NO: 93 | "E" | 1101, 1102, 1103, 1104 | 0.2X, 42 |
| CRX23 | SEQ ID NO: 66 | "A-H" | 1401, DR "LY10" | 0.1X, 42 |
| CRX35 | SEQ ID NO: 71 | "F-DR" | 1601, 1101, 1104, DR "PEV", 0801, 0802 | 0.2X, 42 |
| CRX49 | SEQ ID NO: 74 | "G-YK" | 0701, 0702 | 1.0X, 42 |
| GH102 | SEQ ID NO: 89 | "YSTG" | 0801,0802,0803, 1201, DR "LY10", 1404 | 0.1X, 42 |
| GH111 | SEQ ID NO: 92 | "K-D-F" | 0901 | 0.4X, 42 |
| CRX34 | SEQ ID NO: 70 | "EV" | 1001 | 0.4X, 42 |
| CRX04 | SEQ ID NO: 60 | "R" | 0101, 0102, 0403, 0404, 0405, 0406, 0407, 0408, 1402 | 0.1X, 42 |
| GH56 | SEQ ID NO: 86 | "YSTS" | 0301, 0302, 1101-1104, 1301-1303, 1401, 1402, DR "PEV" | 0.2X, 42 |
| CRX68 | SEQ ID NO: 84 | "F-DE" | 1103 | 0.2X, 42 |
| CRX12 | SEQ ID NO: 63 | DRB "ALL" | All HLA-DRB alleles | 0.2X, 42 |

The probes shown in Table 4 are hybridized and then washed for 15 minutes at 42°C, except for GH59, which is washed for 20 minutes at 42°C. All SSPE wash solutions contain 0.1% SDS. Each probe is conjugated to HRP at the 5'-end.

This PCR/SSO DRbeta typing system is useful for "subtyping" the serologically defined DR haplotypes. For example, the cell line "KOSE" is homozygous for DRw6 when typed serologically; however, PCR/SSO DRbeta typing reveals that "KOSE" has two different DRw6 alleles, DRB1*1302 and DRB1*1401.

Samples that may be DR3 or DRw6 can be distinguished by amplifying with the DRB 1 specific primers GH46 and CRX37 and analyzing the amplification products with the probes GH125 and CRX50 from the second panel of probes, shown in Table 5. The allele specificities shown in Table 5 are with respect to the 1989 allele set.

**Table 5**

| Second Panel of HLA DRB Typing SSO Probes HLA DRB | | | | |
|---|---|---|---|---|
| Probe | SEQ ID NO: | Epitope | Alleles(DRB1*) | Wash (SSPE. °C.) |
| GH125^{a} | SEQ ID NO: 94 | "Y" | 0301 | 0.2X, 50 |
| CRX50^{a} | SEQ ID NO: 75 | "K-GR" | 0301, 0302 | 0.2X, 50 |
| CRX53^{a} | SEQ ID NO: 76 | "K" | 0401 | 0.5X, 55 |
| CRX15^{a} | SEQ ID NO: 64 | "R-E" | 0403, 0406, 0407 | 0.4X, 55 |
| CRX62 | SEQ ID NO: 81 | "I-DK" | 1303 | 0.2X, 42 |
| CRX63 | SEQ ID NO: 82 | "I-DR" | 0803, 1201 | 0.2X, 42 |
| CRX61 | SEQ ID NO: 80 | "S" | 0405, 1303, 0801, 0803 | 0.1X, 42 |
| GH54^{a} | SEQ ID NO: 85 | "V-S" | 0701, 0901, 1201 | 0.2X, 42 |
| CRX56^{a} | SEQ ID NO: 86 | "G" | 0101, 0103, 0302, 0401, 0405, 0407, 0408, 1101, 1302, 1303, 0701, 0901, 1001, 1402, DR "PEV", 0801, 0802, 0803, 1502, 1601,1602 | 0.2X, 42 |
| CRX57^{a} | SEQ ID NO: 78 | "V" | 0301, 0402, 0403, 0404, 0406, 1102, 1103, 1104, 1301, DR "LY10," 1401, 1501 | 0.1X, 42 |
| CRX12 | SEQ ID NO: 63 | DRB "ALL" | All HLA DRB alleles | 0.2X, 42 |

For the probes shown in Table 5, all SSPE wash solutions contain 0.1% SDS. In addition, probes marked "a" require DRB 1 specific amplification with primers GH46/CRX37. CRX15 is hybridized at 50°C instead of 42°C. Each probe is conjugated to HRP at the 5'-end. The probes CRX56 and CRX57 do not completely base-pair with the epitope "G" and epitope "V" alleles, respectively, because the probes contain one fewer G residue than the alleles. Probes modified to include the G residue missing in CRX56 and CRX57 are within the scope of the present invention.

For purposes of the present invention, "DRB 1 specific" primers are primer pairs that comprise at least one primer that hybridizes to an intron sequence flanking the second exon of the DRB1 gene, which primer will not hybridize to the other DRB genes. Table 6 shows the nucleotide sequence of the DRB1 and DRB3 introns of the DR3 haplotype, from the last 10 codons prior to the start of the 3' downstream intron. The sequence for CRX37 (SEQ ID NO: 73) is underlined; the solid arrow indicates the direction of extension of the primer. The asterisks indicate sequence differences between the DRB1 and DRB3 intron sequences. The segments of the 3' downstream introns are listed in the Sequence Listing as SEQ ID NO: 314 (DRB1) and SEQ ID NO: 315 (DRB3); sequences upstream of the intron are in the allele sequences.

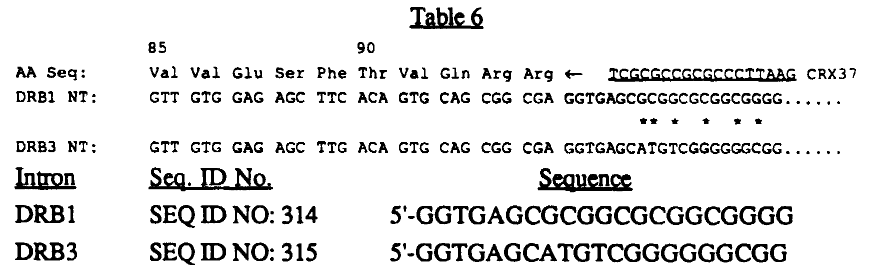

The DR3, DR4, and DRw6 haplotypes appear to be evolutionarily distinct from DR2, DR7, and DR9 haplotypes. There may be differences in the intron sequences of these alleles such that the primer is too mismatched to prime extension. Because the DRB1 alleles may have diverged in evolution prior to gene duplication, it may prove difficult to find DRB1 specific primer sequences for amplifying the DRB1 gene second exon from all DR haplotypes.

The HLA DRB 1 alleles can be distinguished with greater specificity by amplifying with the generic and DRB 1 specific primers and analyzing with both panels (Tables 4 and 5) of probes as required. The alleles of the 1989 allele set that cannot be distinguished with the specific probes in Tables 4 and 5 are the four DR2 subtypes DRB1*1501, *1502, *1601, and *1602 and the DR4 subtypes DRB1*0403 and DRB1*0406.

The DRB1 specific PCR primers are capable of reproducibly amplifying the DRB1 sequences from all but the DR2, DR7, and DR9 haplotypes. The primer CRX37 was designed from intron nucleotide sequences from DR3, DR4, and DRw6 haplotypes. See the article entitled "Sequence Analysis of HLA Class II Genes from Insulin-Dependent Diabetic Individuals" by Horn et al., 1988, Hum. Immunol. 1:249.

The present invention provides a number of allele specific and group specific primers in addition to the DRB1 specific primer pair GH46/CRX37. These primers are shown below. For the sequence of AB60, see Todd et al., 1987, Nature 329:599.

AB54 SEQ ID NO: 56 5'-GGGGATCCTGGAGCAGGTTAAACA-3'

AB60 SEQ ID NO: 57 5'-CCGAATTCCGCTGCACTGTGAAGCTCTC-3'

AB82 SEQ ID NO: 58 5'-GGGGATCCTGGAGTACTCTACGTC-3'

AB83 SEQ ID NO: 59 5'-GGGGATCCTGTGGCAGCCTAAGAGG-3'

For example, DR4 specific amplification can be achieved with PCR primer pair AB54/AB60 (PCR profile: 35 cycles of: ramp to 94°C; 30 seconds denaturing at 94°C; 30 seconds annealing and extending at 65°C). DR3, DR5, and DR6 group specific amplification can be achieved with PCR primer pair AB82/AB60 (35 cycles of: ramp to 96°C; 30 seconds denaturing at 96°C; 30 seconds annealing and extending at 65°C). DR2 specific amplification can be achieved with PCR primer pair AB83/AB60 (35 cycles of: ramp to 96°C; 30 seconds denaturing at 96°C; 30 seconds annealing and extending at 70°C).

Specific alleles can be detected after group specific amplification with the epitope specific probes described herein; for instance, DRB1*1601 can be detected after DR2 specific amplification with the AB83/AB60 primer pair with an "F-DR" epitope specific probe.

Detection of alleles of the DR2 haplotype can be difficult, but such difficulty can be overcome by the group specific primers and the typing methods of the present invention. In the generic primer amplification of DRB alleles from DR2 haplotypes, both the DRB 1 and DRB5 loci are amplified. Although one could use an "F" (at position 47) epitope specific DNA probe to determine the serologic DRw15 and DRw16 subtypes, "F' occurs also in 0301, some DRw13 alleles, and in all DRw11 and DRw12 alleles; other methods can provide more complete discrimination. Thus, a group specific primer designed to hybridize to the sequence encoding the epitope "I--A" will amplify the DRB1*1501 and DRB1*1502 alleles, and the amplified product will hybridize with a "WPR" epitope specific DNA probe. The primer DRB150 (SEQ ID NO: 226) (5'-TGTCCACCGCGGCCCGCGCCT-3') is a primer designed to perform such an allele specific amplification (with GH46). The DRB5*0201 and DRB5*0202 alleles will also amplify with a group (or epitope) specific "I--A" primer, but as these alleles occur only with the DRB*1601 and DRB1*1602 alleles, the result of probe hybridization will be epitope "QDY" positive and "WPR" negative.

In addition, one should note that because the DRB1 specific primers exemplified herein do not amplify DR2 alleles, the DR2 specific amplification described above can be carried out in the same reaction tube and at the same time as the DRB1 specific amplification. Other allele and group specific primers and amplification methods are described in the Examples.

The extensive allelic diversity at the HLA DRB loci, like that of the other Class II beta genes, is localized primarily to the second exon. In general, the pattern of second exon sequence polymorphism, when present in the population at a particular DRB locus, is a patchwork, with specific polymorphic segments found in a variety of different alleles. In principle, such shared epitopes among different alleles could reflect either common ancestry, gene conversion, or convergent evolution. For purposes of oligonucleotide typing, however, this patchwork pattern of polymorphism means that many alleles cannot be identified by hybridization to a single oligonucleotide but can be identified by a unique pattern of hybridization with a panel of probes.

The sequence-specific oligonucleotide probes of the invention, called "SSOs," when employed in the present methods under the appropriate hybridization and wash conditions, are extremely specific, capable of distinguishing single nucleotide polymorphisms. The SSOs of the invention, unlike the probes used in RFLP methods, can be used to determine not only if alleles are different but also where and how the alleles differ.

In a preferred embodiment, these probes comprise the same sequence as, or a sequence complementary to, the regions of DNA of each allele boxed in Tables 1 and 2. For example, the probe CRX60 is specific for the DNA encoding the amino acids "W-L-F" and can hybridize uniquely to the DR1 allele. The probe CRX49 is specific for the amino acids encoding "G-YK" and hybridizes to the DR7 allele; other examples will be apparent upon consideration of the Tables.

While a single probe (i.e., GH56) can be used to distinguish DR3, DRw11, and DRw6 from the other DR serotypes, additional probes are required to distinguish between DR3, DRw11, and DRw6. The GH56 + GH122 probe combination, which hybridizes to DNA encoding the "YSTS" and "E" epitopes (codons 10 to 13 and 58) on DRw11, can be used to distinguish DRw11 from DR3 and DRw6. Likewise, the probe combinations GH56 + CRX06 corresponding to the "YSTS" and "I-DE" (codon 67 to 71) epitopes can be used to distinguish DRw6 (DRB1*1301 and DRB1*1302 but not DRB1*1303, which has the epitope "I--DK" but is still DRw6) from DRw11 and DR3.

The combinations of the SSO probes shown in Table 4 used to determine the majority of the serological DR types are shown in Table 7.

**Table 7**

| Combinations of SSO Probes to Determine Serological DR Types | |
|---|---|
| DR Type | Probe(s) |
| DR1 Dw1 | CRX60 + CRX04 |
| DR1 Dw "BON" | CRX60 + CRX06 |
| DR2 | GH105 |
| DR4 Dw4 | GH59 + CRX53 |
| DR4 Dw10 | GH59 + CRX06 |
| DR4 Dw13, 14, 15 | GH59 + CRX04 |
| DR3, w11, w6 | GH56 |
| DRw11.1 | GH56 + GH122 + CRX35 |
| DRw11.2 | GH56 + GH122 + CRX06 |
| DRw12, DRw8.3 | GH102 + CRX63 |
| DRw13 (1301, 1302) | GH56 + CRX06 |
| DRw14 Dw16 | GH56 + CRX04 |
| DRw14 Dw9 | GH56 + CRX23 |
| DR7 | CRX49 |
| DRw8.1, w 8.2 | GH102 + CRX35 |
| DR9 | GH111 |
| DRw10 | CRX34 |
| DRw12, all DRw8 | GH102 |

Further subdivision of the DR serotypes (Table 4) requires the use of probes shown in Table 5, as shown in Table 8, below.

**Table 8**

| Combinations of SSO Probes to Distinguish 31 of 34 HLA DRB1 Alleles (1989 set) | | | |
|---|---|---|---|
| DRB1* | Probe(s) | DRB1* | Probe(s) |
| 0101 | CRX60 + CRX04 + CRX56 | 1103 | GH122 + GH56 + CRX57 + CRX68 |
| 0102 | CRX60 + CRX04 | 1104 | GH122 + GH56 + CRX35 + CRX57 |
| 0103 | CRX60 + CRX06 + CRX56 | 1201 | GH102 + GH54 + CRX63 |
| DR2 | GH105 | 1301 | GH56 + CRX06 + CRX57 |
| 0301 | GH56 + GH125 + CRX50 + CRX57 | 1302 | GH56 + CRX06 + CRX56 |
| 0302 | GH56 + CRX50 + CRX56 | 1303 | GH56 + CRX62 + CRX61 + CRX56 |
| 0401 | GH59 + CRX53 + CRX56 | 1401 | GH56 + CRX23 + CRX57 |
| 0402 | GH59 + CRX06 + CRX57 | 1402 | GH56 + CRX04 + CRX56 |
| 0403, 0406 | GH59 + CRX15 + CRX57 + CRX04 | DR"PEV" | GH56 + CRX35 + CRX56 |
| 0404 | GH59 + CRX04 + CRX57 | 0701 | CRX49 + GH54 + CRX56 |
| 0405 | GH59 + CRX04 + CRX61 + CRX56 | 0801 | GH102 + CRX61 + CRX35 + CRX56 |
| 0407 | GH59 + CRX15 + CRX56 + CRX04 | 0802 | GH102 + CRX35 + CRX56 |
| 0408 | GH59 + CRX04 + CRX56 | 0803 | GH102 + CRX61 + CRX63 + CRX56 |
| 1101 | GH122 + GH56 + CRX35 + CRX56 | 0901 | GH111 + GH54 + CRX56 |
| 1102 | GH122 + GH56 + CRX06 + CRX57 | 1001 | CRX34 +CRX56 |
| | | DR"LY10" | GH102 + CRX23 + CRX57 |

The horseradish peroxidase-conjugated SSOs of the invention, called "HRP-SSOs," allow detection methods that employ chromogenic or chemiluminescent substrates that are easy to use and produce detectable signals rapidly (typically 1 to 10 minutes). The HRP-SSOs are stable for over two years without detectable loss of activity when stored at 4°C. See the article entitled "Nonisotopically labeled probes and primers" by Levenson and Chang, 1989, in PCR Protocols: A Guide to Methods and Applications (Innis, Gelfand, Sninsky and White ed., Academic Press, Inc. San Diego). Radiolabelled probes can be employed but are not necessary for excellent sensitivity, an important benefit provided by the present invention.

The dot-blot format for detection enables the rapid typing of a large number of samples and will be useful in determining the allele frequencies of HLA DRB. A recently developed alternative for PCR/SSO DRbeta typing is the immobilized reverse dot-blot format. See the article entitled "Genetic analysis of amplified DNA with immobilized sequence-specific oligonucleotide probes" by Saiki et al., 1989, Proc. Natl. Acad. Sci. USA 86:6230. In this procedure, the SSO probes are applied and fixed to the filter (rather than the amplified DNA applied and fixed to the filter), hence the term "reverse dot blot."

The reverse dot blot procedure allows a single sample to be analyzed in a single hybridization with a membrane containing an array of immobilized probes. The conventional dot blot format is useful when the number of samples exceeds the number of probes used (e.g., patient versus control or population genetics studies). The reverse dot blot format is valuable for clinical, diagnostic, and forensic analyses. The reverse dot blot format is described in more detail in Example 8.

The following examples show illustrative preferred embodiments of the present invention. The examples show that the present invention provides, in a preferred embodiment, a nonisotopic PCR/SSO system for HLA DRB typing that is simple, rapid, and capable of precise DRB typing for a variety of samples from different sources.

### Example 1

### Amplification and Detection Methods

For typing samples with the first panel (Table 4) of probes, 0.5 µg of human genomic DNA was amplified using reaction constituents as described in the article entitled "Primer-directed enzymatic amplification of DNA with a thermostable DNA polymerase," by Saiki et al., 1988, Science 239:487 and in Scharf et al., 1988, Hum. Immunol. 22:61, and Scharf et al., 1989, Proc. Natl. Acad. Sci. USA 86:6215, incorporated herein by reference.

The HLA DRB generic PCR primers are GH46 and GH50. The sequences of these primers are shown below. GH46 SEQ ID NO: 67 5'-CCGGATCCTTCGTGTCCCCACAGCACG GH50 SEQ ID NO: 68 5'-CTCCCCAACCCCGTAGTTGTGTCTGCA The primers were present in the reaction mixture at 500 nM. These primers produce a 272 base-pair (bp) fragment and contain sequences for BamHI and PstI restriction sites for cloning the PCR product Digestion of the amplified DNA with BamHI and PstI produces a 248 bp product due to an internal PstI site.

The DRB1 alleles from all haplotypes (except DR2, DR7, and DR9) were specifically amplified by the PCR primers GH46 and CRX37. The sequence of the CRX37 primer is shown below. CRX37 SEQ ID NO: 73 5'-GAATTCCCGCGCCGCGCT Amplification with the GH46/CRX37 primer pair produces a 297 bp fragment. Primer CRX37 incorporates an EcoRI restriction endonuclease recognition sequence at the 5'-end to facilitate cloning and, in contrast to the primer pair GH46/GH50, amplification and BamHI/EcoRI digestion with this primer pair allows isolation and analysis of the full-length PCR product.

Such isolation and analysis frequently involved the determination of the nucleotide sequence of the PCR product. For sequencing the HLA DRB alleles, 1 µg of purified human genomic DNA was amplified using the primer pairs GH46/GH50 and GH46/CRX37. The amplified DNA was cloned into M13mp10 by the methods described in Scharf et al., 1988, Hum. Immunol. 22:61, and Scharf et al., 1986, Hum. Immunol. 233:1076. The inserts were then sequenced by the dideoxy chain-termination procedure described in Sanger et al., 1977, Proc. Natl. Acad. Sci. USA. 74:5463.

The sequences shown in Tables 1 and 2 above were generated by genomic cloning (see Horn et al., 1988, Hum. Immunol. 21: 249 , PCR amplification (see Erlich et al., 1989, in Immunobiology of HLA (du Pont ed., Springer-Verlag, New York) and Scharf et al., 1989, Proc. Natl. Acad. Sci. USA 86: 6215), or from the literature (see WHO Nomenclature Committee, 1990, Immunogenetics 31:131 and Gregersen et al., 1989, "First domain diversity of DR and DQ subregion alleles" in Immunobiology of HLA (du Pont, ed., Springer-Verlag. New York)).

Samples were amplified for 32 cycles (unless otherwise noted) using the reaction conditions described above, except that 1.25 units (rather than 2.5 units) of Taq polymerase (PECI, Norwalk, CT) were added per 100 µl of reaction volume. The cDNAs for the bladder carcinoma patients were amplified for the HLA-DRB loci as described (see Kawasaki, 1989, "Amplification of RNA" in PCR Protocols: A Guide to Methods and Applications (Innis et al., eds., Academic Press. San Diego)). All samples were overlaid with 100 µl of high grade mineral oil (Sigma, St. Louis, MO) to prevent evaporation. After amplification, the oil overlay was extracted with 100 µl chloroform.

The thermal profile for each cycle comprised incubations at the following temperatures for the indicated times: 45 seconds at 94°C (for denaturation of the DNA strands), 45 seconds at 55°C (for annealing of the primers), and 45 seconds at 72°C (for extension of the primed templates). After the last cycle, the PECI Thermal Cycler (Perkin Elmer Cetus Instruments, Norwalk, CT) was programmed to incubate the samples at 72°C for 10 minutes to ensure that the final extension was complete. Care should be taken to avoid cross-contamination of samples; one must particularly guard against.allowing the product of one PCR to contaminate an unamplified sample, e.g. by methods to prevent amplification of PCR product that has been "carried over" to an unamplified sample.

After amplification, a small portion of the amplified DNA was denatured and applied to and crosslinked to a series of nylon filters; each filter was then hybridized to one of the labelled probes. Each SSO probe was covalently conjugated to horseradish peroxidase (HRP) and provides a means of nonisotopic detection in the presence of a chromogenic or chemiluminescent substrate. The nucleotide sequence, the encoded amino acids (or potential epitope), and the identified DR type, as well as the wash conditions for each probe are listed in Tables 4 and 5.

Thus, 5 µl of each amplified DNA sample were mixed with 100 µl of a mixture composed of 0.4 M NaOH and 25 mM EDTA, and the resulting mixture applied to BioDyne B nylon filters (Pall Corp., Glen Cove, NY) using a dot-blot manifold (Bio Rad, Richmond, CA). The filters, still in the dot-blot manifold, were rinsed with a mixture of 10 mM Tris-HCl and 0.1 mM EDTA, at pH 8.0, and dried on Whatman 3MM paper. The DNA was immobilized on the nylon filter by ultraviolet irradiation at a flux of 55 mJ/cm² with a Stratalinker™ (Stratagene, La Jolla, CA) UV light box.

Unless otherwise noted, all filters were hybridized in 2X SSPE (saline sodium phosphate EDTA), 5X Denhardt's solution, and 0.5% SDS with 2 pmoles of HRP-SSO probe per ml of hybridization solution for 15 min. at 42°C. Horseradish peroxidase conjugated oligonucleotides were prepared as described by Levenson and Chang, 1989, in PCR Protocols: A Guide to Methods and Applications (Innis et al., eds., Academic Press, Inc. San Diego) and Saiki et al., 1988, N. Eng. J. Med. 319:537. Filters for each probe were washed in 25 ml of the SSPE solutions at the temperatures listed in Tables 4 and 5 for 15 minutes (unless noted otherwise).

After washing, filters to be developed with a chromogenic dye substrate were rinsed in PBS at room temperature for 30 minutes, then placed in 100 mM sodium citrate, pH 5.0, containing 0.1 mg/ml of 3,3',5,5'-tetramethylbenzidine (TMB) per milliliter (Fluka) and 0.0015 percent hydrogen peroxide, and incubated with gentle agitation for 5 to 15 minutes at room temperature. Developed filters were rinsed in PBS and immediately photographed. Filters that were developed with the chemiluminescent detection system (ECL; Amersham, Arlington Heights, IL) were rinsed in PBS for 5 minutes and placed in the ECL solution for 1 minute with gentle agitation. Filters were then exposed to X-ray film at room temperature for 1 to 5 minutes.

### Example 2

### DRB 1 Specific Amplification

Several polymorphic sequences of alleles of the DRB 1 locus that distinguish the various DRB alleles are also present on alleles of the other DRB loci (see Table III). An example is the nucleotide sequence encoding the epitope "K-GR" (codons 71 to 74) on DR3 DRB 1 alleles, where a probe to this region (CRX50) can distinguish DR3 from DRw11 and DRw6 alleles. However, this epitope is also encoded by the DRB3 allele DRw52a (DRB3*0101) and is present on some DRw6 haplotypes and some DR3 haplotypes as well. Because the PCR primers GH46/GH50 amplify all of the DRB loci, a DRw6 sample that was DRw52a at the DRB3 locus would be impossible to distinguish from a DR3 sample using this probe.

The present invention solves this problem by providing PCR primers that specifically amplify only the DRB 1 locus. One of these primers hybridizes to a region in the intron immediately downstream from the second exon. The intron contains sequences that distinguish the DRB1 and DRB3 loci. The primer (CRX37) hybridizes specifically to the DRB 1 intron sequences, and combining this primer with GH46 permits DRB 1 specific amplification for most haplotypes.

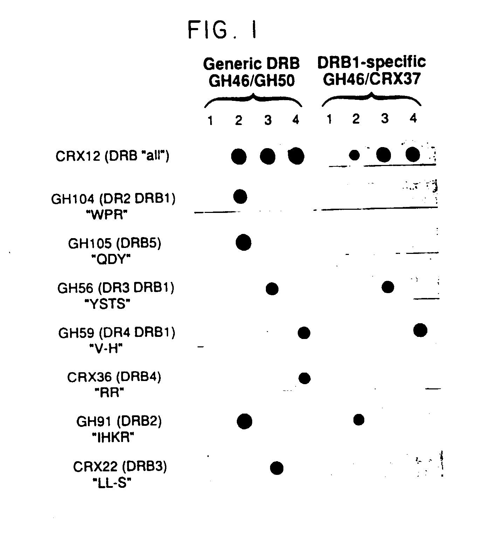

To determine if the DRB 1 locus is the only locus amplified with these putative DRB 1 specific primers, DR2, DR3, and DR4 HTC (homozygous typing cells) DNA was amplified and analyzed with these primers and SSO probes for all the DRB loci. In addition to the DRB1 locus, the DR2 haplotype has a DRB2 and DRB5 locus, the DR3 haplotype has a DRB3 locus, and the DR4 haplotype has a DRB4 locus. The results are shown in Figure 1.

To generate these results, about 200 ng of HTC DNA were amplified by the generic DRB primers GH46/GH50 or by the DRB 1 specific primers GH46/CRX37 and applied to filters as described in Example 1. Each filter containing amplified cell line DNA (samples 1 to 4) was hybridized with the probes shown in Figure 1. The locus to which each probe hybridizes is shown in parentheses. CRX36 hybridizes to the DRB4 locus of DR4, DR7, and DR9. CRX22 hybridizes to the DRB3 allele DRw52b.

The sequence of the SSOs: CRX36 SEQ ID NO: 72 5'–CCCGCCTCCGCTCCA CRX22 SEQ ID NO: 65 5'-GAGCTGCTTAAGTCT is described by Scharf et al., 1989, Proc. Natl. Acad. Sci. USA 86:6215. Probe GH91 (SEQ ID NO: 88, 5'-CTCCCGTTTATGGATGTAT) hybridizes specifically to the DRB2 locus. This probe was hybridized as described in Example 1 and washed in 1X SSPE, 0.1% SDS for 15 minutes at 42°C. The samples amplified are: (1) No DNA (negative control); (2) DR2 HTC "SCHU"; (3) DR3 HTC "QBL"; and (4 ) DR4 HTC "BSM." After the hybridization and wash steps, the probes were detected by enhanced chemiluminescence.

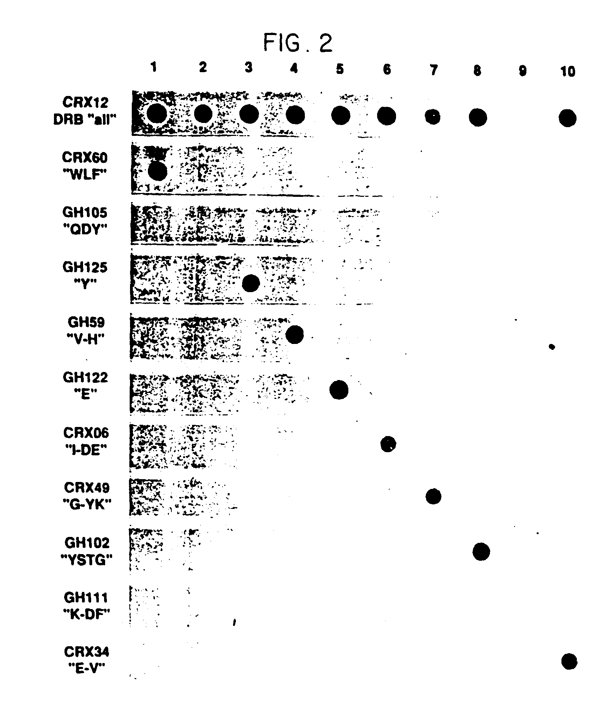

Figure 1 shows that all of the DRB loci for all three HTCs are amplified with the generic DRB primers, while the DRB 1 specific primers only amplify the DRB 1 loci from the DR3 and DR4 HTCs. Interestingly, for the DR2 HTC, the DRB2 locus is amplified, albeit weakly, with the DRB1 specific primers. The DRB1 specific primers amplify DRB1 sequences efficiently from all DR haplotypes tested except DR2, DR7, and DR9, as shown in Figures 1 and 2. To generate the results shown in Figure 2, about 0.5 µg of genomic DNA from HTCs was amplified for HLA DRB with the DRB 1 specific primers GH46/CRX37. Amplification reactions and filters were prepared as described in Example 1. Each filter strip was hybridized to one of the probes shown under the appropriate conditions of hybridization and wash stringency (see Tables 4, 5, and 7). After the hybridization and wash steps, filters were developed in the chromogenic substrate TMB. The samples are: (1) DR1 HTC "KAS9003"; (2) DR2 HTC "SCHU"; (3) DR3 HTC "QBL"; (4) DR4 HTC "BSM"; (5) DR5 (DRw11) HTC "SPOO1O"; (6) DRw6 HTC "OMW"; (7) DR7 HTC "MOU"; (8) DRw8 HTC "SPACH"; (9) DR9 HTC "DKB"; and (10) DRw10 HTC "SHY".

The results in Figure 2 indicate that the DR7 probe CRX49 ("G-YK") does hybridize to the weakly amplified DR7 HTC in this experiment. DRB 1 amplification of DR7 from heterozygous samples is typically weak. These results demonstrate that the generic and DRB1 specific primers can be used for complete DRB1 typing.

### Example 3

### Typing for the Serological DR Types 1 Through 10

About 0.5 µg of genomic DNA from HTCs was amplified for HLA DRB. All samples except for nos. 3 and 6 were amplified with the generic primers GH46/GH50. Sample nos. 3 and 6 were amplified with the DRB1 specific primers GH46/CRX37. Amplification reactions and filters were prepared as described in Example 1. Each filter strip was hybridized to one of the probes shown under the appropriate conditions of hybridization and wash stringency (see Tables 4, 5, and 7). After probing and washing, filters were developed in the chromogenic substrate TMB. The samples are: (1)DR1 HTC "KAS9003"; (2) DR2 HTC "SCHU"; (3) DR3 HTC "QBL"; (4) DR4 HTC "BSM"; (5) DR5 (DRw11) HTC "SPOO1O"; (6) DRw6 HTC "OMW"; (7) DR7 HTC "MOU"; (8) DRw8 HTC "SPACH"; (9) DR9 HTC "DKB"; and (10) DRw10 HTC "SHY".

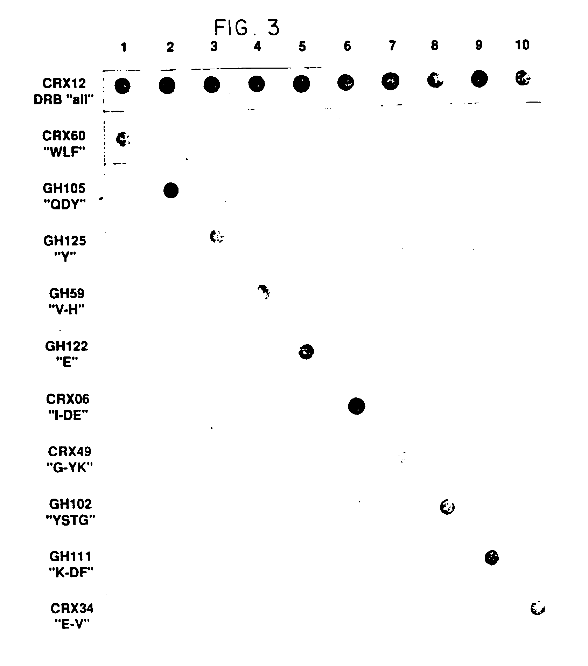

Figure 3 demonstrates the results of DR typing for the classical, serologically defined DR types 1 through 10 on a panel of HTCs. A single probe can be used to detect each of the classical DR types, but two different PCR primer pairs were required. For all but the DR3 and DRw6 samples, the generic PCR primers GH46/GH50 can be used. The DRB 1 specific primer pair GH46/CRX37 was used for amplifying the DNA of the DR3 and DRw6 samples in this array, because the probe used to detect the "Y" epitope (GH125; codon 26) on the DR3 HTC "QBL" (DRw17) is also present on the DRB3 allele (DRw52a) of the DRw6 sample. If the standard DRB primers had been used, then the "Y" probe would have hybridized to the DRw6 sample as well.

### Example 4

### General DRB1 Typing Strategy

For typing a particular sample, the generic primers should be used for the first amplification, followed by probing of the amplified DNA with the first panel of probes (Tables 4 and 7). This panel of probes will identify the majority of the serological types; although GH56 ("YSTS") will not unequivocally distinguish DR3 from DRw6.

For samples that are positive with GH56, or for subtyping particular DR types (e.g., the Dw types of DR4 or the subtypes of DRw8), the second panel (Table 5) of probes should be used. As discussed above, some of the probes in the second panel require amplification with the DRB 1 specific pair of PCR primers. By combining the results of the probings from the first and second panels, one can distinguish 31 of the 34 DRB1 alleles in the 1989 allele set. The combinations of SSO probes used to identify these alleles is shown in Table 8. The DR2 subtypes (DRB1*1501, *1502, *1601, and *1602) are not distinguished by these primers and probes, and the DRB1*0403 and DRB1*0406 alleles are not distinguished by these probes.

One DR2 probe of the invention comprises sequences that hybridize to the first hypervariable region ("Q-D-Y") of the DRB5 locus. Because the DRB5 locus is present on all known DR2 haplotypes and only on those haplotypes, such a probe can be used reliably to type for DR2 in an amplification with generic DRB primers. Probe GH104 ('W-P-R") hybridizes to the first hypervariable region of the DR2 DRB1 locus and hybridizes specifically to DR2 DNA when amplified with the generic primers (Figure 1) but not with the DRB1 specific primers. These two probes are identical indicators of DR2 in an amplification with generic primers.

### Example 5

### Subtyping DR4

The subtyping of the DR4 specificity cannot be carried out serologically. The subtypes have been revealed by two dimensional protein gel electrophoresis and cellular typing; both methods are cumbersome and time-consuming. Dw4, Dw10, Dw13, and Dw14 differ from each other at positions 70 to 74 of the third hypervariable region of the gene. Dwl5 also has a serine ("S") at position 57. Dw13 also has the same arginine residue at position 71 as Dw14 and Dw15 but is distinguished by a glutamic acid ("E") as position 74.

As a result, the SSO probe CRX04 ("R") probe hybridizes to the Dw13, Dw14, and Dw15 alleles; these alleles have to be distinguished from each other by additional probes. The SSO probe CRX15 ("R-E") specifically distinguishes Dw13 from Dw14 and Dw15, and CRX61 ("S") specifically distinguishes Dw15 from Dw14. The "G" versus "V" polymorphism at position 86 that distinguishes various DRB1 alleles (e.g., Dw14.1 or DRB1*0404 from Dw14.2 or DRB1*0408) is detected using the CRX56 probe ("G") and the CRX57 probe ("V") (see Table 5).

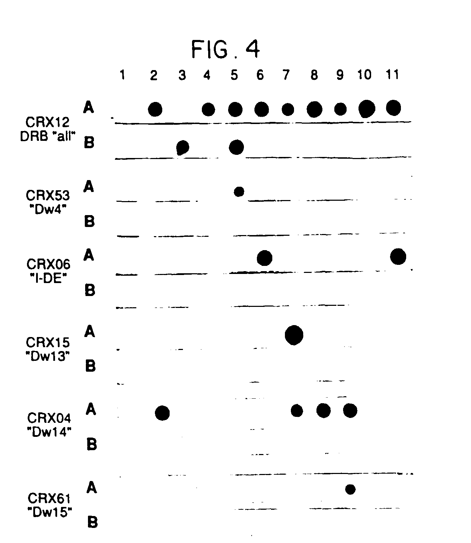

HTCs with DR types 1 through 10 and the five DR4 Dw subtypes were amplified with the DRB1 specific primers GH46/CRX37, applied to six identical nylon filters, and hybridized with HRP-SSO's specific for the Dw type. The results are shown in Figure 4.

To generate the results shown in Figure 4, about 500 ng of HTC genomic DNA were amplified with the DRB 1 specific primers GH46/CRX37. Each pair of filters, A and B, were hybridized with the probes shown in Figure 4. The samples are: Row A: (1) No DNA control; (2) DRI HTC "KAS9003"; (3) DR2 HTC "SCHU"; (4) DR3 HTC "QBL"; (5) DR4 Dw4 HTC "BSM"; (6) DR4 Dw10 HTC "YAR"; (7) DR4 Dw13 HTC "JHA"; (8) DR4 Dw14 HTC "BM92"; (9) DR4 Dw15 HTC "LKT3"; (10) DR5 HTC "SPOO1O"; and (11) DRw6 HTC "OMW"; Row B: (1) No DNA control; (2) DR7 HTC "MOU"; (3) DRw8 HTC "SPACH"; (4) DR9 HTC "DKB"; and (5) DRw10 HTC "SHY".

After the hybridization and wash steps, the probes were detected by enhanced chemiluminescence. Panel A shows that all but the DR2, DR7, and DRw9 HTCs hybridized to CRX12.

Panel B shows that CRX53 hybridizes specifically to the Dw4 HTC BSM. An alternative probe for typing DR4 Dw4 is DRB163 (SEQ ID NO: 239), using the hybridization and wash conditions given in Example 9. Another alternative probe for typing DR4 Dw4 that gives a much stronger signal at lower stringencies (2X SSPE, 5X Denhardt's, 0.5% SDS at 42°C for 15 minutes hybridization and 2X SSPE, 0.1% SDS at 42°C for 15 minutes wash) is probe CRX64, shown below. CRX64 SEQ ID NO: 83 5'-HRP-GAGGAIAAGCIGGCC-3' HRP is horseradish peroxidase. I is inosine, which slightly destabilizes the probe.

Panel C shows detection of the Dw10 HTC "YAR" with the SSO CRX06 ("I-DE"). This probe also hybridizes to the HTC "OMW", which is DRw13, and shares this polymorphism.

Panel D shows specific hybridization of CRX15, which distinguishes the Dw13 sample, JHA, from the Dw14 sample BM92 and the Dw15 sample LKT3.

Panel E shows hybridization of CRX04 to JHA, BM92, and LKT3, which are Dw13, Dw14, and Dw15, respectively. CRX04 also hybridizes to the DR1 HTC KAS9003, which is DRB1*0101, and shares this polymorphism ("R") with these three Dw-types. The DR4 Dw14 type is inferred from the probe hybridization pattern in which samples do not hybridize to either CRX15 ("R-E") or CRX61 ("S"; panel F), but do hybridize to CRX04 ("R"). Panel F shows hybridization of CRX61 to the Dw15 HTC "LKT3".

Figure 4 shows that, in general, the present invention enables one to distinguish samples that share polymorphic regions recognized by these probes by the pattern of hybridization to the first panel of probes. For example, DR4 Dw types which are positive for the "R" epitope can be distinguished from DR1 by being positive for GH59 ("V-H") and negative for CRX60 ("W-L-F"); DR4 Dw10 can be distinguished from DRw13 by being positive for GH59 and negative for GH56 ("YSTS").

Table 8 shows that the two DR4 subtypes that this system is not capable of distinguishing are the DRB1*0403 and DRB1*0406 alleles. These two alleles will give the same pattern of hybridization with the two panels of probes. DRB1*0406 is identical to DRB1*0403, except for a serine residue at position 37, where DRB1*0403 has a tyrosine residue. The illustrative panels of probes shown above are not capable of detecting this polymorphism, but the use of additional probes provides complete discrimination.

### Example 6

### Subtyping DR3, DR5, DRw6, and DRw8

The subtypes for the "splits" of DR3, DR5, DRw6, and DRw8 can be identified by using probes that distinguish between the various alleles of each haplotype. For example, both DRw17 and DRw18 hybridize to the "K-GR" probe CRX50, but only DRw17 hybridizes to the "Y" probe GH125, which distinguishes DRw17 from DRw8. Likewise, the DRw11 and the DRw12 alleles (which used to be grouped as DR5) can be distinguished from each other and from the three DRw8 alleles by using a different combination of probes.

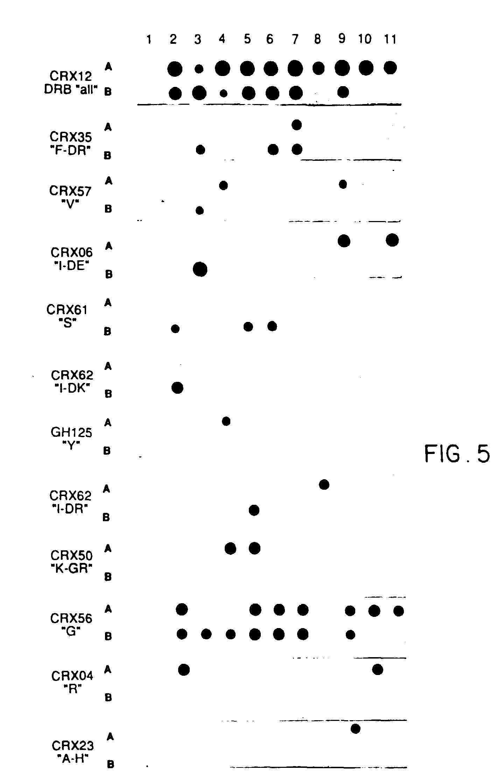

DNA amplified with the DRB1 specific PCR primers CRX37/GH46 from HTCs of the "general" serological DR types 1 through 10 along with HTCs of the DR3, DR5, DRw6, DRw8 subtypes, was applied to 12 filters. Because the serological DR types of these samples were already known, only the probes necessary to determine the subtypes of these alleles were used to illustrate this aspect of the invention. The results are shown in Figure 5.

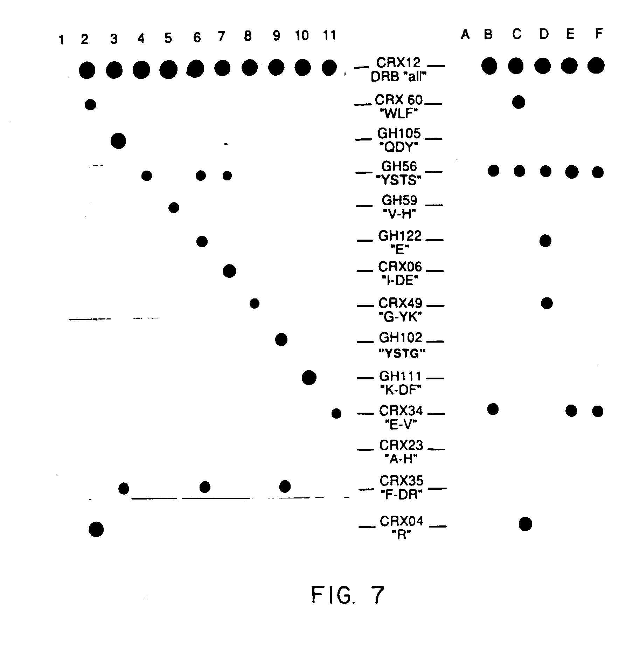

To generate the results shown in Figure 5, about 500 ng of HTC genomic DNA were amplified with the DRB 1 specific primers GH46/CRX37. Each pair of filters, A and B, was hybridized with the probes shown. The samples are: Row A: (1) No DNA control; (2) DR1 HTC "KAS9003"; (3) DR2 HTC "SCHU"; (4) DRw17 HTC "QBL"; (5) DRw18 HTC "RSH"; (6) DR4 HTC "BSM"; (7) DRw11HTC"SPOO1O"; (8) DRw12 HTC "HERLUF"; (9) DRw13/DRw14 "KOSE"; (10) DRw14 HTC "AMALA"; (11)DRw13 HTC "SLE"; Row B: (1) No DNA control; (2) DRw13 HTC "HAG"; (3) DR PEV "BAR P"; (4) DR7 HTC "MOU"; (5) DRw8.3 HTC "TAB"; (6) DRw8.1 HTC "ARC"; (7) DRw8.2 HTC "SPL"; (8) DR9 HTC "DKB"; and (9) DRw10 HTC "SHY". The data for the first panel of probes, which shows that GH56 ("YSTS") hybridizes to DR3, DR5, and DRw6, and GH102 ("YSTG") hybridizes to DRw8 is shown in Figure 7 (see Example 7).

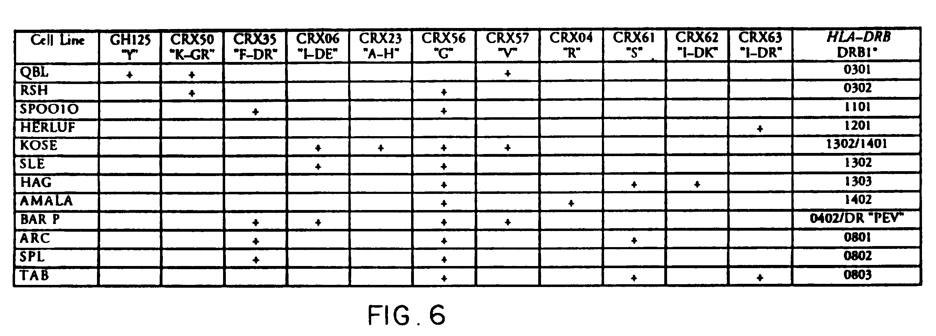

After the hybridization and wash steps, the bound probes were detected by enhanced chemiluminescence. By determining the pattern of probes that hybridize to each sample (see Table 8), the samples can be subtyped for specific alleles, as shown in Figure 6. In Figure 6, samples which hybridized to probes are shown with a "+" symbol. Blank cells indicate samples which did not hybridize to a particular probe. Except for "KOSE and BAR P", all the samples were homozygous typing cells.

The hybridization data and DR types for the DR3, DR5, DRw6 and DRw8 samples are shown in Figure 6. The sample "KOSE" was typed serologically as homozygous for the DRw6 haplotype (though this sample has been shown to be Dw9 and Dw19); however, it types as DRB1*1302 (CRX06 + CRX56) and DRB1*1401(CRX23 + CRX57).

The sample "BAR P" is typed by serology and MLC as DR4 Dw10 and DRw6. Consequently, "BAR P" hybridizes to the probes CRX35 and CRX56 ("F-DR" and "G"), which types it as the newly discovered "DR PEV" DRw6 allele, and to the probes CRX06 and CRX57 ("I-DE" and "V"), reflecting the presence of the DR4 Dw10 allele (DRB1*0402).

### Example 7

### DR Typing of Cell Lines

Many established cell lines do not express the Class II molecules, making it impossible to DR type them serologically. DNA samples from five Class II negative cell lines coded as a blind panel were amplified with the generic primer pair GH46/GH50 and probed with the first panel of probes (Table 4) to establish the general serological DR types for the samples (Figure 7).

For DR typing these and other cell lines, approximately 100 ng of cell line DNA were amplified for 30 cycles with the generic HLA DRB primers GH46/GH50. Each filter containing amplified cell line DNA (samples A through F) was hybridized simultaneously with a control filter (samples 1 through 11) containing HTC DNAs amplified with the generic HLA DRB primers. All filters were prepared as described in Example 1. The results are shown in Figure 7. Each pair of filters was hybridized to the SSO probes shown. The samples are: (1) No DNA control; (2) DR1 HTC "KAS9003"; (3) DR2 HTC "SCHU"; (4) DR3 HTC "QBL"; (5) DR4 HTC "BSM"; (6) DR5 (DRw11) HTC "SPOO1O"; (7) DRw6 HTC "OMW"; (8) DR7 HTC "MOU"; (9) DRw8 HTC "SPACH"; (10) DR9 HTC "DKB"; (11) DRw10 HTC "SHY;" (A) No DNA control; (B) Raji; (C) 616; (D) Beguit; (E) RM3; and (F) RS225.

The signal intensity for the CRX12 probe demonstrates that all of the samples were equally well amplified. Raji, RM3, and RS225 hybridize to the probe CRX34, which is specific for DRw10. Sample 616 hybridizes to the probes CRX60 ("WLF") and CRX04 ("R"), which is consistent with this sample having the DR1 allele.

The cell line Beguit hybridizes to the DR7 probe CRX49 ("G-YK") and to two other probes, GH56 ("YSTS") and GH122 ("E"), suggesting that it is DR7 and DRw11. However, the putative DRw11 allele from this sample would be expected to hybridize either to CRX35 ("F-DR"; DRB1*1101) or CRX06 ("I-DE"; DRB1*1102), but it does not, suggesting that it may be a new variant of DRw11, as discussed further below.

This sample, derived from an individual with bare lymphocyte syndrome, was cloned and sequenced to determine the nature of the polymorphism at this position. Sequencing of the allele showed that there is indeed a sequence polymorphism at the third hypervariable region that codes for the epitope "F-DE"; this allele types as DRB1*1103 (see sequence "Beguit" in Table 3). Probe CRX68 hybridizes specifically to this polymorphism in the third hypervariable region (see Table 4).

All of the other samples also hybridize to the GH56 "YSTS" probe, so they could be DR3, DRw11, or DRw6. Because these other samples do not hybridize to the "E" probe, it is likely they are DR3 or DRw6. However, the samples do not hybridize to the probes CRX06 ("I-DE") or CRX23 ("A-H"), which recognize sequences present in three common DRw6 alleles, DRB1*1301, DRB1*1302, and DRB1*1401; this suggests that they are not DRw6, but DR3.

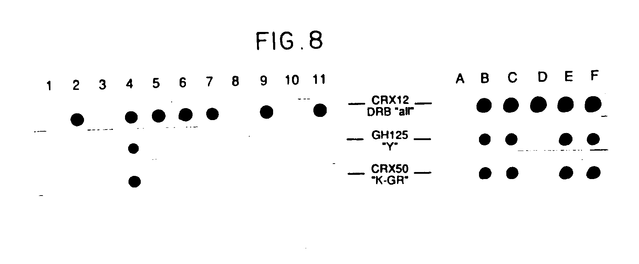

To confirm this, the samples were amplified with the DRB 1 specific primers GH46/CRX37 and probed with the SSOs for DR3, GH125 ("Y") and CRX50 ("K-GR"). About 100 ng of cell line genomic DNA were amplified with the DRB1 specific primers GH46/CRX37 as described in Example 1. Each filter containing amplified cell line DNA (samples A through F) was hybridized simultaneously with a control filter (samples 1 through 11) containing HTC DNAs amplified with the DRB specific primers. All filters were prepared as described in Example 1. The results are shown in Figure 8.

Each pair of filters was hybridized the SSO probes shown. The samples are: (1) No DNA control; (2) DR1 HTC "KAS9003"; (3) DR2 HTC "SCHU"; (4) DR3 HTC "QBL"; (5) DR4 HTC "BSM"; (6) DR5 (DRw11) ) HTC "SPOO1O"; (7) DRw6 HTC "OMW"; (8) DR7 HTC "MOU"; (9) DRw8 HTC "SPACH"; (10) DR9 HTC "DKB"; (11) DRw10HTC "SHY"; (A) No DNA control; (B) Raji; (C) 616; (D) Beguit; (E) RM3; and (F) RS225. After the hybridization and wash steps, the bound probes were detected by enhanced chemiluminescence.

As Figure 8 shows, Raji, 616, RM3, and RS225 hybridize to both the "Y" and "K-GR", which types them as DR3 (DRw17). In summary, Raji, RM3, and RS225 type as DRw10/DRw17. RM3 and RS225 are cell lines derived from Raji, so it is not surprising that they have the same DRB type. Cell line 616 types as DR1/DRw17, and Beguit types as DR7/DRw11 but, as noted above, the Beguit cell line contains the DRB1*1103 allele, which has the "F-DE" epitope. Previously, most DRw11 typing cells were observed to contain DRB1 alleles with the "F-DR" epitope. Thus, the Beguit cell line contains an unusual DRB1 allele for the DRw11 type.

In addition to cell line samples, DNA from three different sources was amplified and typed. One source is purified genomic DNA from a set of unrelated, heterozygous individuals from the Center for Study of Human Polymorphism (CEPH, Paris, France; samples 555 to 863). Another source was cDNA made from mRNA of cancer tissue from patients with bladder carcinoma (samples 2426, 2446, 2540, 2671, 2755). Normal human bladder cells do not express Class II molecules, but various carcinoma cells have been reported as expressing Class II molecules.

The remaining sample (PSW) is a buccal swab amplified for HLA DRB directly without DNA purification. For complete DR typing, the samples were amplified with both the generic DRB primers GH46/50 and the DRB 1 specific PCR primers GH46/CRX37. Filter strips containing DNA samples amplified with the general probes were probed with the first panel of probes (Table 4), and filter strips containing DNA samples amplified specifically for DRB1 were probed with the second panel of probes (Table 5).

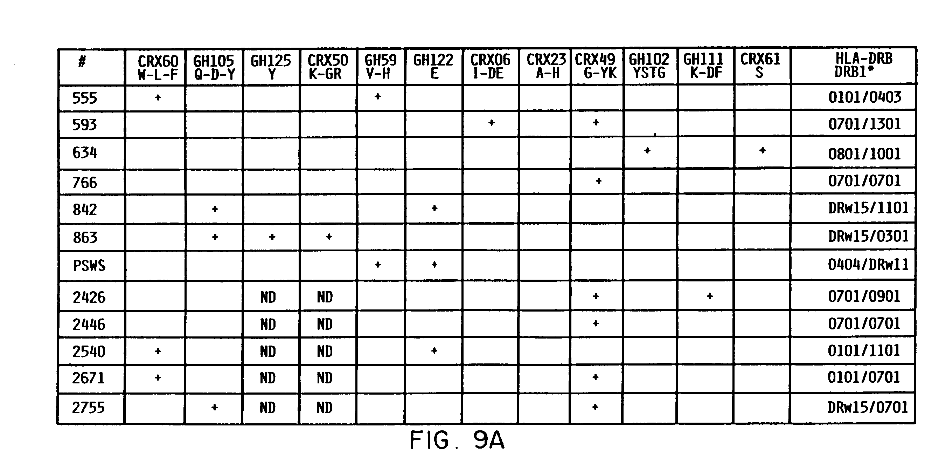

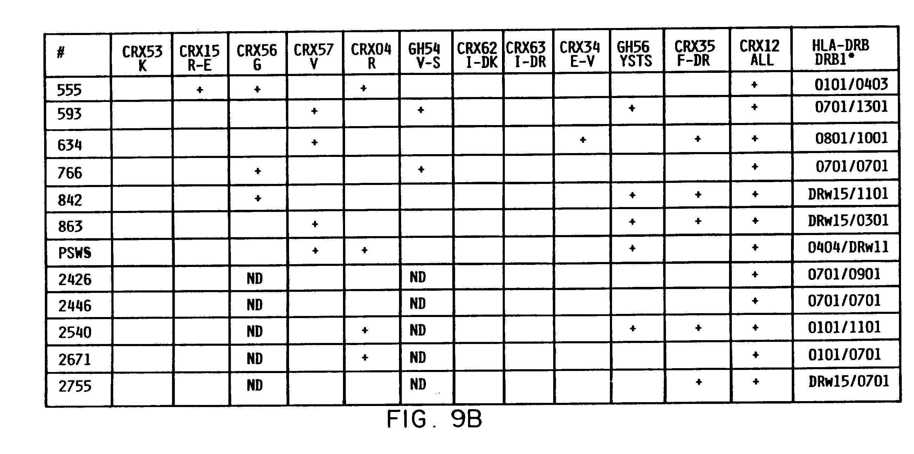

Comparing the pattern of hybridization with Table 3 and Table 8, one can unambiguously derive the DRB-type of the samples. The results for the typing of the heterozygous samples are shown in Figure 9. In Figure 9, samples which hybridized to a probe are shown with a "+" symbol. Blank cells indicate samples which did not hybridize to a particular probe.

Samples 555 to 863 are pure genomic DNA provided by CEPH. Sample "PSW" is a sample amplified directly from a buccal swab. This sample was heated for 5 minutes at 95°C in 200 µl of 5% Chelex (Singer-Sam et al., 1989, Amplifications 3:11). About 50 µl of this solution were amplified directly in 200 µl of reaction volume, and generic DRB and DRB 1 specific amplification reactions were conducted for the sample for 40 cycles.

Samples 2426, 2446, 2540, 2671, and 2755 were amplified from bladder carcinoma cDNA preparations. The bladder carcinoma samples 2426, 2446, 2540, 2671, and 2755 were not analyzed with the SSOs GH125, CRX50, CRX56, and GH54, because these probes require amplification with the DRB1 specific primers GH46/CRX37. Because CRX37 is derived from intron sequence, it cannot be used to amplify cDNA. ND is not determined.

The buccal swab sample, PSW, is DR4 Dw14 (DRB1*0404) and DRw11.

These data show that the system is capable of typing heterozygous DNAs from a variety of sources, from standard purified genomic DNA, from cDNAs synthesized from RNA, and from unusual sources, such as a buccal swab or single hair.

### Example 8

### DRB Typing - Reverse Dot Blot Format

In this embodiment of the invention, the DRB probes are fixed to a membrane, and the amplified target DNA is hybridized to the membrane-bound probe. The set of typing probes is designed so that each probe will hybridize to a specific target sequence at the same temperature and salt concentration (and stay hybridized under the same wash conditions) as all other probes in the set The PCR primers used in the amplification are biotinylated, as described in the book PCR Protocols, incorporated herein by reference, so that any amplified DNA that hybridizes to the membrane-bound probes can be easily detected.

In one embodiment, detection is carried out by reacting streptavidin (SA)-conjugated horseradish peroxidase with any biotinylated, amplified DNA hybridized to the membrane-bound probe. The HRP thus becomes bound, through the SA-biotin interaction, to the amplified DNA and can be used to generate a signal by a variety of well known means, such as the generation of a colored compound, e.g., by the oxidation of tetramethylbenzidine (see U.S. Patent No. 4,789,630).

Although the probes can be fixed to the membrane by any means, a preferred method involves "tailing" an oligonucleotide probe about 13 to 25 nucleotides in length with a much longer sequence of poly-dT. The resulting poly-dT "tail" can then be reacted with amine groups on the membrane to fix the probe covalently to the membrane. This reaction can be facilitated by UV irradiation.

Terminal deoxyribonucleotidyl transferase (TdT, Ratliff Biochemicals; for the reactions below assume a concentration of abut 120 Units/µl, which is 100 pmol/µl) can be used to create a poly-dT tail on a probe, although one can also synthesize the tailed probe on a commercially available DNA synthesizer. When one uses a DNA synthesizer to make the tailed probe, however, one should place the tail on the 5' end of the probe, so that undesired premature chain termination occurs primarily in the tail region.

TdT reactions should be carried out in volume of about 100 µl containing 1X TdT salts, 200 pmol of oligonucleotide, 800 µM dTT, and 60 units of TdT. 10X TdT salts is 1,000 mM K-cacodylate, 10 mM CoCl₂, 2 mM dithiothreitol, 250 mM Tris-Cl, pH 7.6, and is prepared as described by Roychoudhury and Wu, Meth. Enzymol. 65:43-62. A 10X stock solution of 8 mM dTTP can be prepared (neutralized to pH 7 with NaOH) for convenience.

The TdT reaction should be carried out at 37°C for two hours and then stopped by the addition of 100 µl of 10 mM EDTA, pH 8. The final concentration of tailed oligonucleotide is 1 µM (1 pmol/µl), and the length of the homopolymer tail is about 400 residues. Tail length can be changed by adjusting the molar ratio of dTTP to oligonucleotide. The tailed probes can be stored at -20°C until use.

Two types of nylon membrane are preferred for the reverse dot blot format: Biodyne™ nylon membrane, 0.45 micron pore size, manufactured by Pall; and Biotrans™ nylon membrane, 0.45 micron pore size, manufactured by ICN. The probes can be spotted onto the membrane very conveniently with the Bio-Dot™ dot blot apparatus manufactured by BioRad. Each probe is spotted onto a unique, discrete location onto the membrane. About 5 to 10 picomoles of each tailed probe is premixed with 60-100 µl of TE buffer before application to the dot blot apparatus. After dot blotting, the membrane is briefly placed on absorbent paper to draw off excess liquid.

The membrane is then placed inside a UV light box, such as the Stratalinker™ light box manufactured by Stratagene, and exposed to 50 to 60 millijoules of flux to fix the tailed probe to the nylon membrane. After a brief rinse (for about 15 minutes in hybridization solution) to remove unbound probe, the membrane is then ready for hybridization with biotinylated PCR product. One-half to one picomole (one-quarter to one-half of a typical, 100 µl PCR mixture) of PCR product is added to each probe panel for hybridization. About 50 µl of streptavidin-horseradish peroxidase (SA-HRP, commercially available from PECI; see the instruction manual for the AmpliType™ DQα DNA Typing Kit) conjugate can be added at this time for convenience, but better signals will result if a separate SA-HRP incubation and wash, at room temperature, is performed after the stringency wash.

Hybridization is typically carried out at 50°C for 30 minutes in a water bath and with hybridization buffer composed of 0.5% SDS and 3X to 5X SSPE, most commonly 4X. Stringency washing is carried out at 50°C for 15 minutes in a water bath and with wash solution composed of 0.1% SDS and 1X SSPE. A post-wash of 1X PBS at room temperature for 30 minutes can enhance signal quality.

The biotinylated primers for the reverse dot blot method and other useful primers of the invention are shown below. Note, however, that one or both of the primers can be biotinylated in an amplification and that the primers can be used for amplification with any detection format.

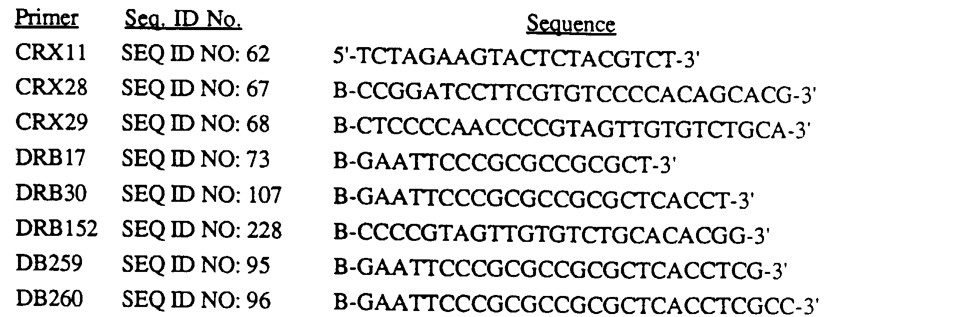

B is biotin. CRX11 is a left-end primer designed for use with GH50 to amplify the DRB1 second exon of DR3, DR5, and DRw6 (13 and 14) haplotypes. CRX28 is biotinylated left-end primer GH46; CRX29 is biotinylated right-end primer GH50; and DRB17 is biotinylated right-end primer CRX37. DRB30 is a right-end primer that includes the CRX37 sequence and has the same DRB 1 range as CRX37 except that, unlike CRX37, DRB30 can amplify DR7 DRB1 sequences. DRB152 is a group specific right-end primer for DR7 and DR9 alleles. DB259 and DB260 are also right-end primers designed to extend the range of DRB1 specific amplification to DR2 and DR9.

The hybridizing regions of the tailed probes for use in the reverse dot blot method are shown below. X is inosine. Where two probe names are shown, i.e., DRB01/CRX60, the first name designates the hybridizing region (shown) of the tailed probe, the second designates the HRP-labeled, untailed probe.

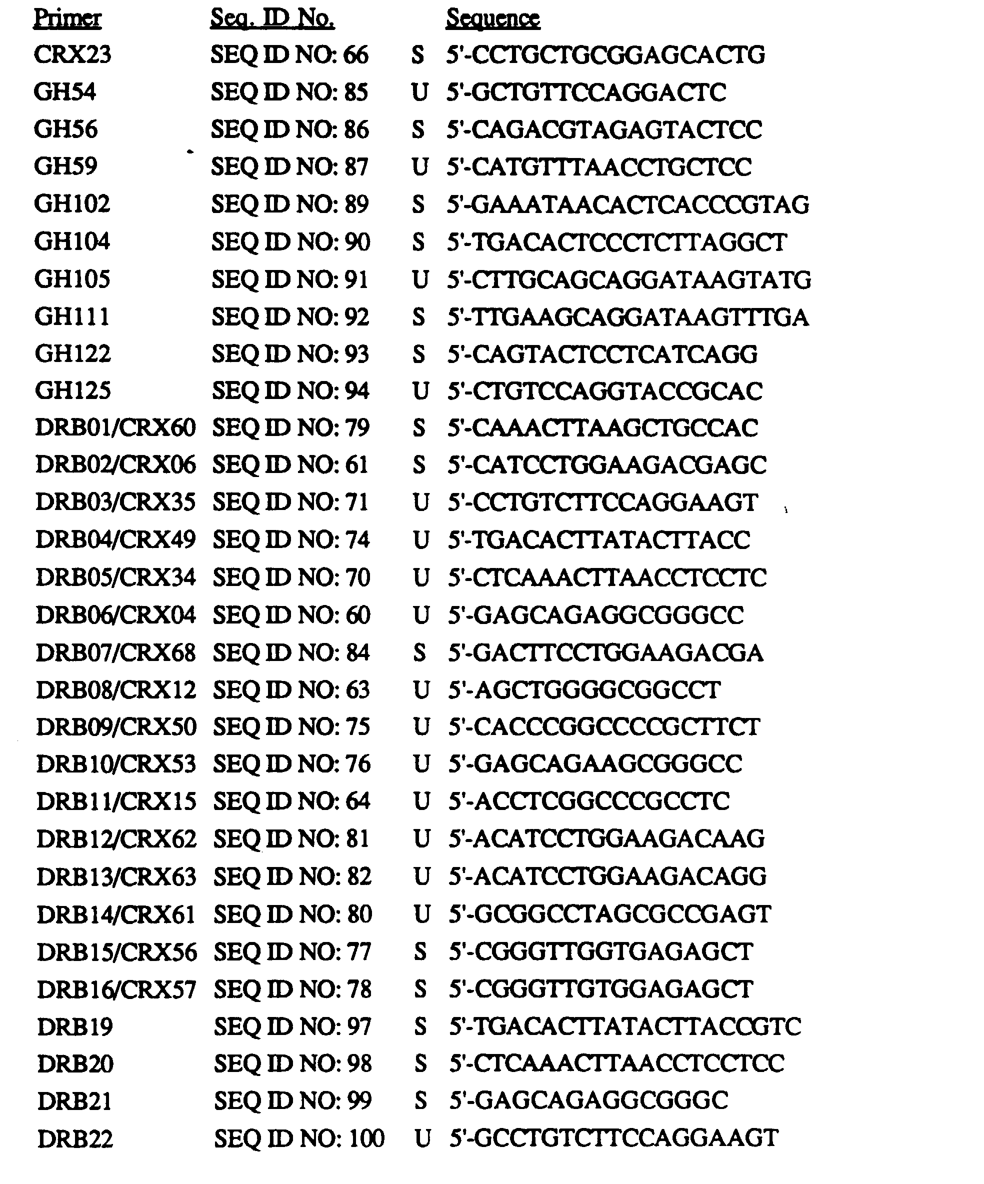

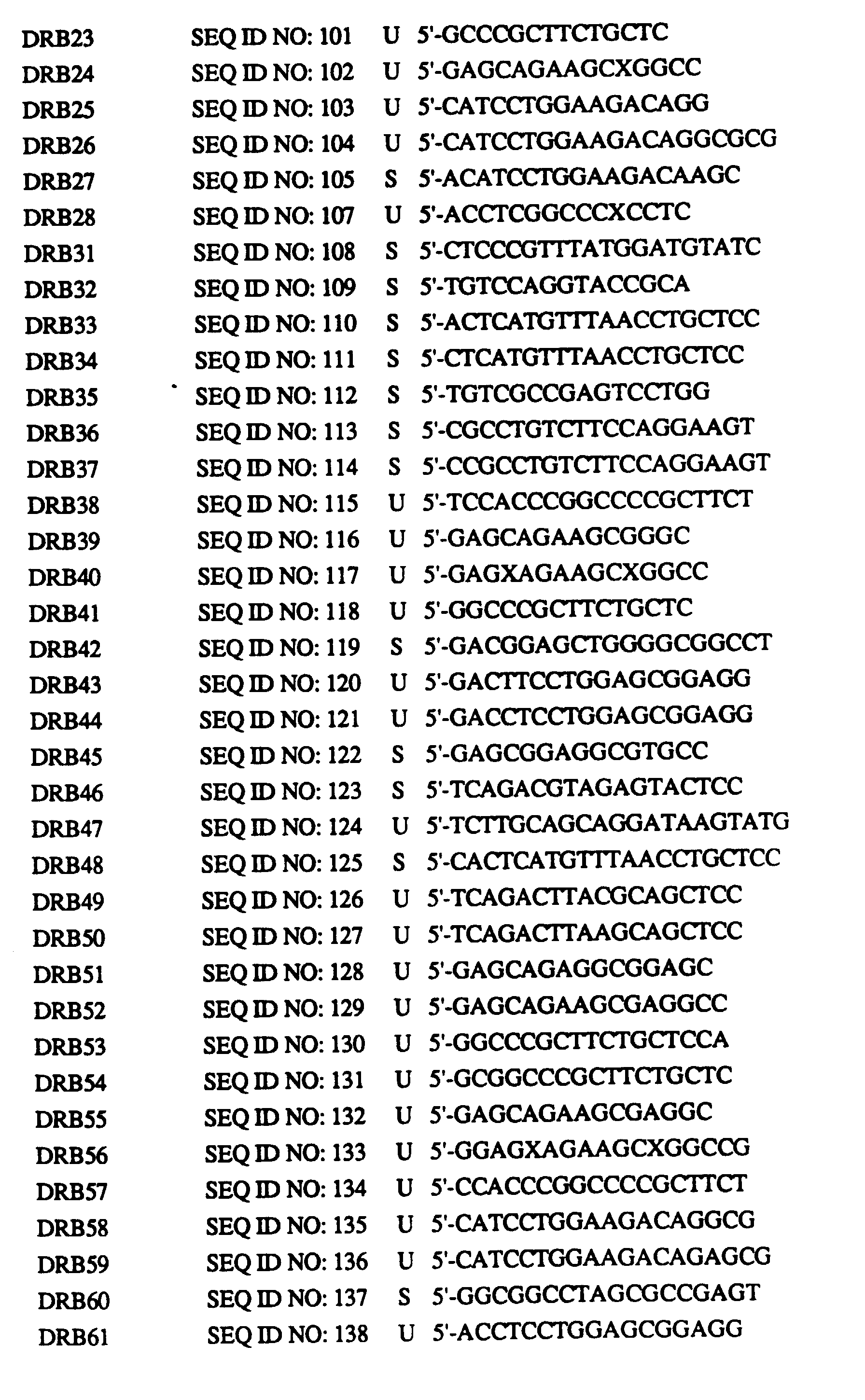

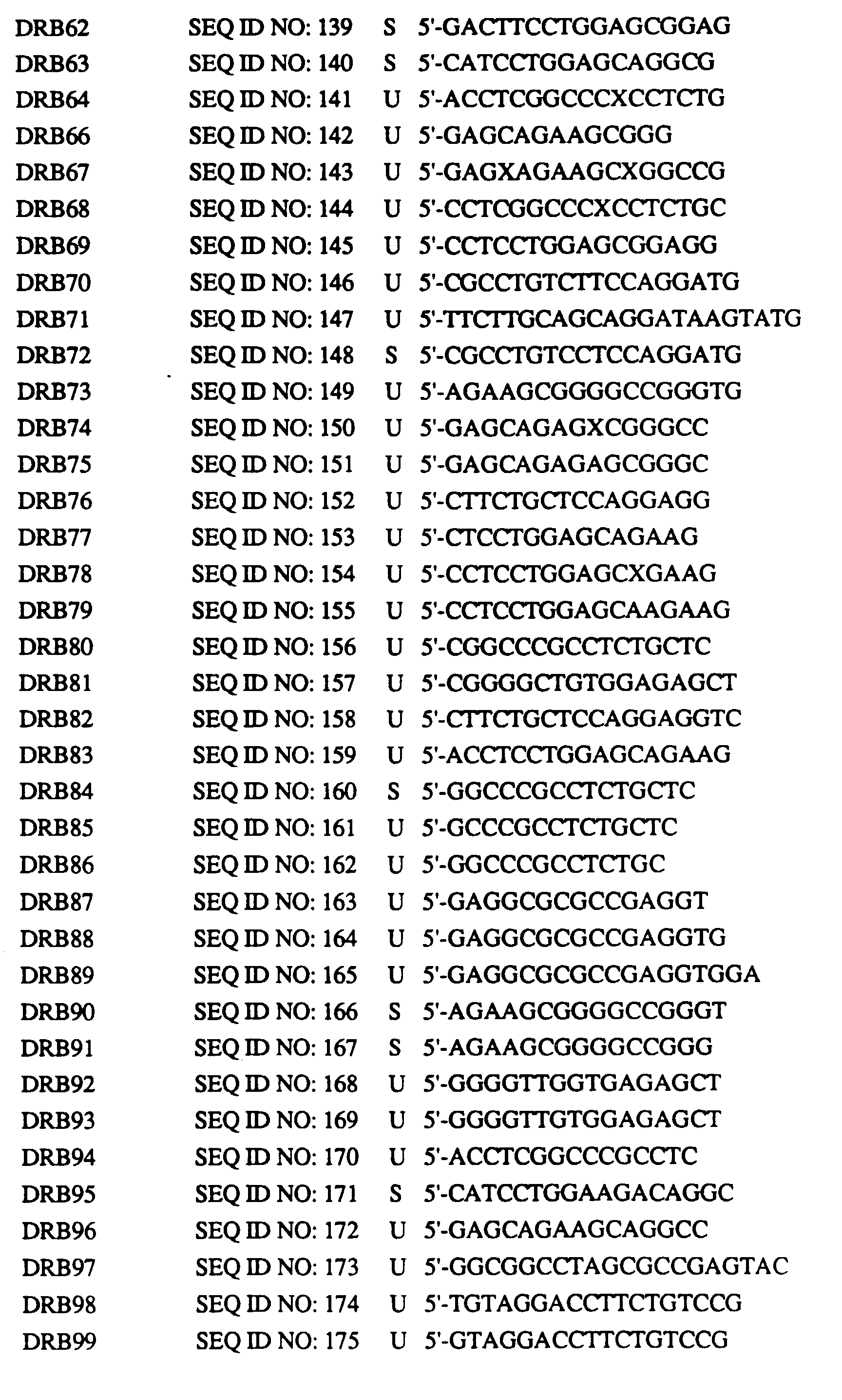

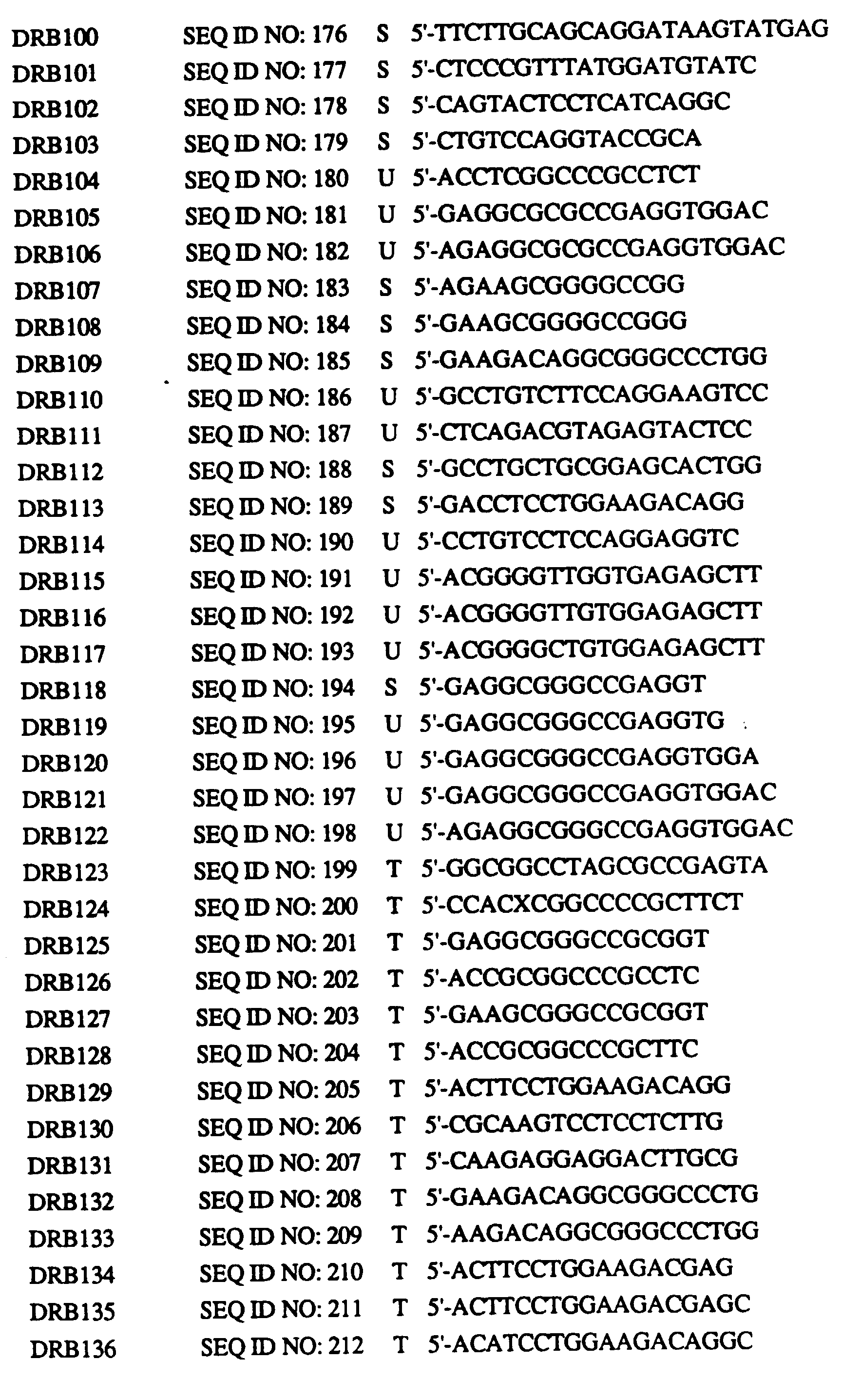

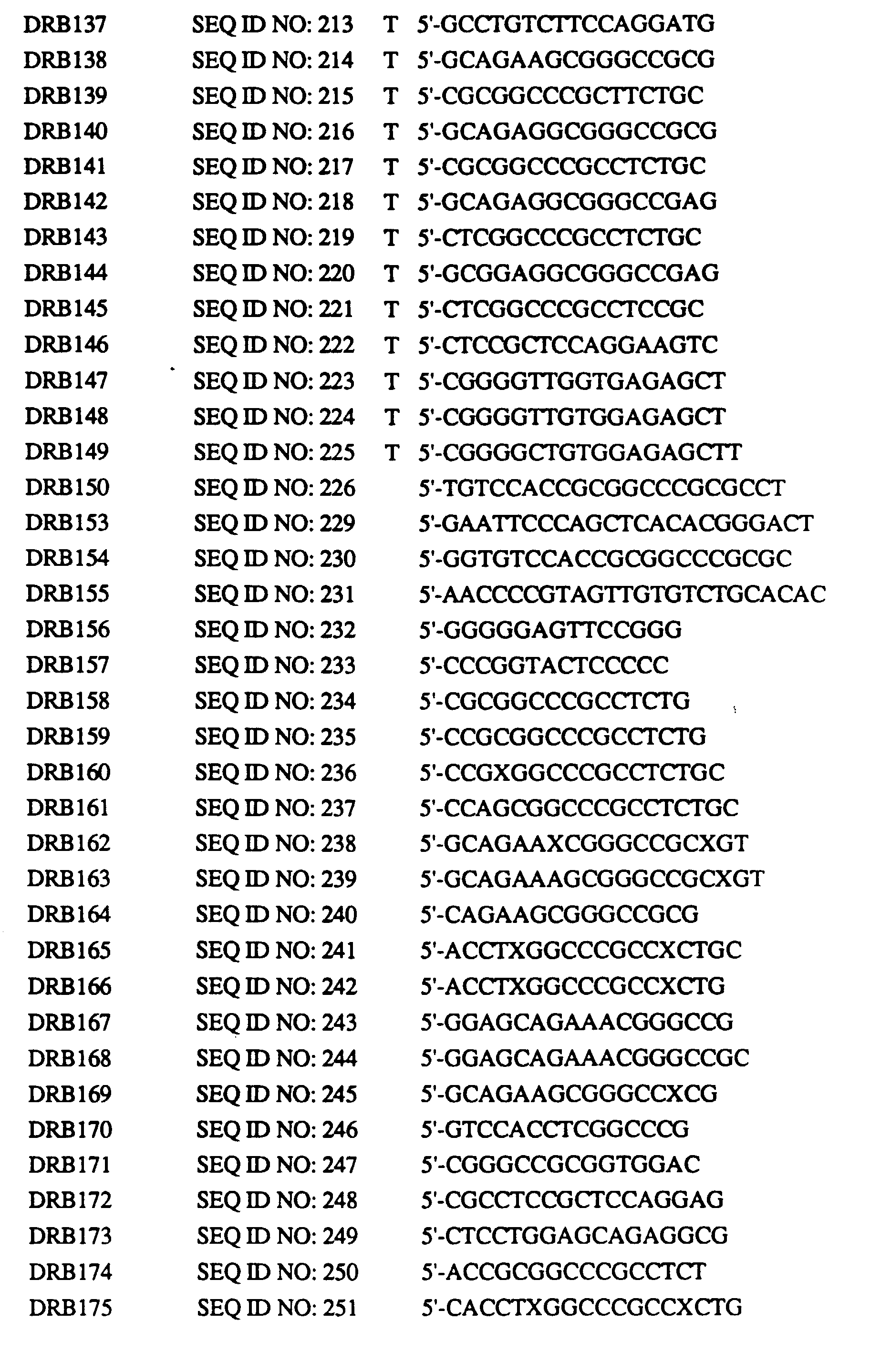

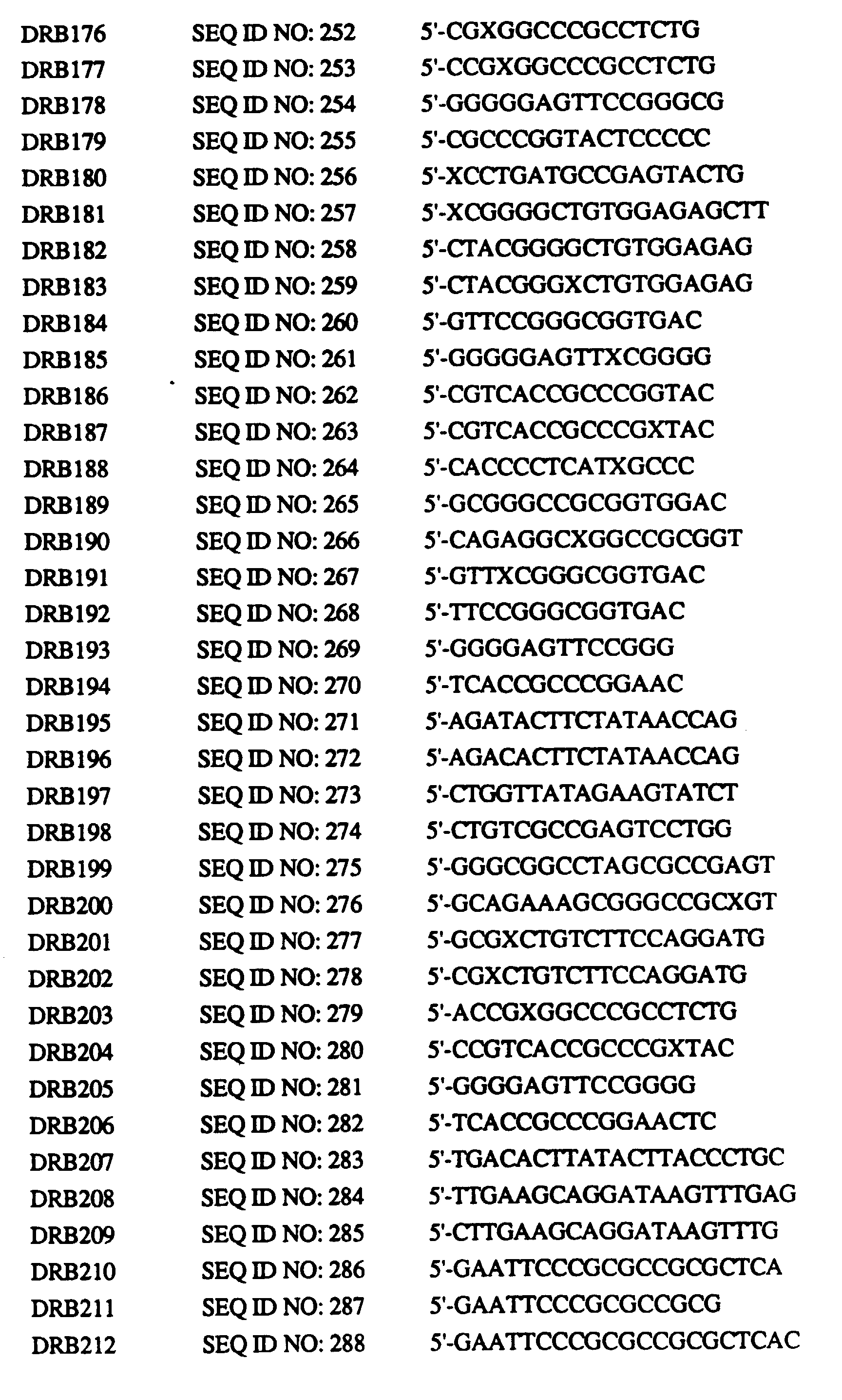

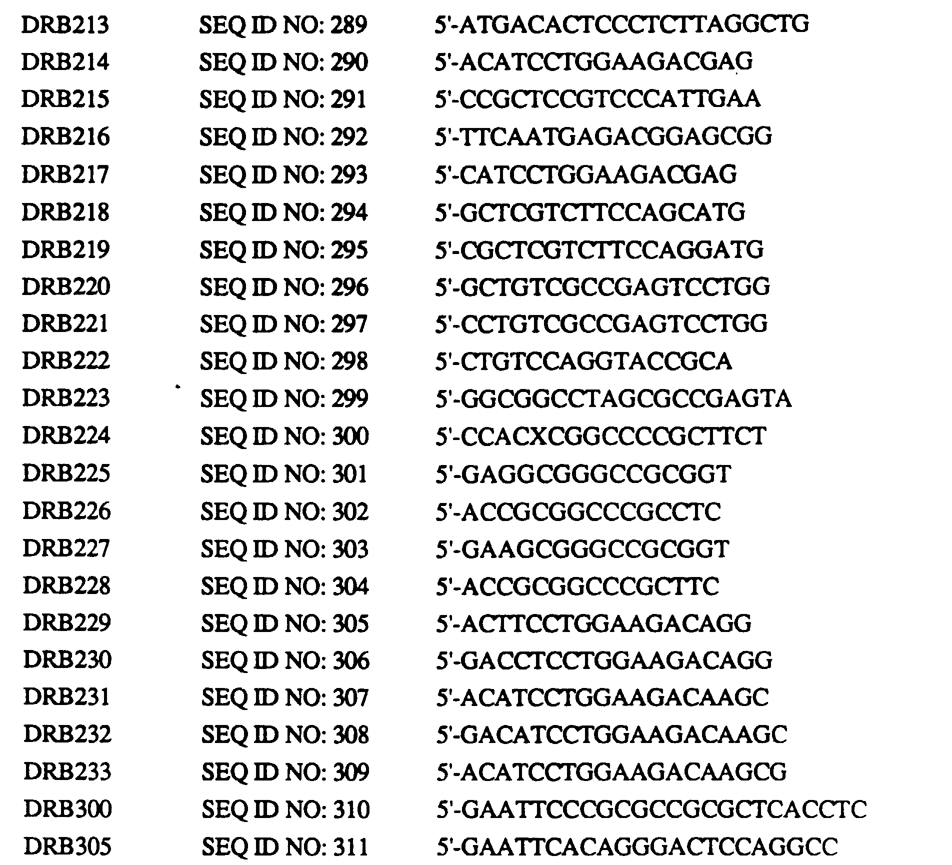

Preliminary testing shows that some probes, marked "S" above are more preferred than other probes, marked "U" above. Other probes, marked "T" above, have not yet been tested.

The hybridization patterns and specificity (1989 allele set) for the preferred reverse dot blot probes are show below. Where an "X" is used in the "specificity" column, the "X" designates inclusivity of all alleles indicated by the first two digits after the "*".

| Epitope | Name | Specificity |
|---|---|---|
| W-L-F | CRX60\DRB01 | DRB1*0101, 0102, 0103 |
| W-P-R | GH104 | DRB1*1501, 1502, 1601, 1602 |
| QDY | DRB100 | DRB5*0101, 0102, 0201, 0202 |
| K-D-F | GH111 | DRB1*0901 |
| YSTS | DRB46 | DRB1*030X, 110X, 130X, 140X |
| YSTG | GH102 | DRB1*080X, 1201 |
| V--H | DRB48 | DRB1*040X |
| G-YK | DRB19 | DRB1*070X |
| EV | DRB20 | DRB1*1001 |
| LR-S | GH57 | DRB3*0101 |
| LL-S | GH58 | DRB3*0201, 0202, 0301 |
| F--DR | DRB37/DRB109 | DRB1*0801, 0802, 1101, 1104, 1601, PEV; DRB5*0101, 0102 |
| F--DE | CRX68 | DRB1*1103 |
| I--DE | CRXO6/DRB02 | DRB1*0103, 0402, 1102, 1301, 1302 |
| I--DK | DRB27 | DRB1*1303 |
| RR | DRB45 | DRB1*1001 |
| F--RR-E | DRB62 | DRB1*0901 |
| I--A | DRB63 | DRB1*1501, 1502; DRB5*0201, 0202 |
| Y | DRB103 | DRB1*0301,DRB3*0101 |
| E | DRB102 | DRB1*110X |
| S | DRB60 | DRB1*0405,0801,0803,1303 |
| A--H | DRB112 | DRB1*1401,LY10 |
| V--S | DRB35 | DRB1*070X, 0901, 1201; DRB3*0101, 0301 |
| R | ---- | DRB1*0101, 0102, 0404, 0405, 0408, 1402 |
| K | ---- | DRB1*0401 |
| R--E | ---- | DRB1*0403, 0406, 0407 |
| RR--E | ---- | DRB1*1401, LY10; DRB4*0101 |
| I--DR | DRB72 | DRB1*0701, 0702 |
| I--DR | DRB95 | DRB1*0803, 1201 |
| K--GR | ---- | DRB1*0301, 0302; DRB3*0101 |
| DR | DRB113 | DRB1*1602 |
| G (pos. 86) | ---- | See Table 5 |
| V (pos. 86) | ---- | See Table 5 |

| Epitope | Name | Specificity |
|---|---|---|
| AV (pos. 86) | ---- | DRB1*0102, 1201; DRB5 |
| WNIIN | GH51 | DRB4*0101 |
| IHKR | DRB101 | DRB2*0101 |

### Example 9

### Reverse Dot-Blot Typing Kit

HLA DRB typing kits incorporating a rapid and simple reverse dot blot hybridization format were designed to provide a simple and fast prescreening of samples before proceeding to fine subtyping involving allele specific amplification. The kits contain the amplification reagents, DNA for use as a positive control, nylon strips on which the probes have been immobilized, colorimetric detection reagents, reaction tubes, and instructions.

Typing was carried out using two amplification reactions, one using DRB general primers and the other using DRB1 specific primers. The primer pairs amplify under the same thermocycler conditions so that the two reactions could be carried out concurrently. Two panels of probes, one specific for each amplification reaction, were used in the reverse dot blot hybridization format Each panel of probes was immobilized on a single nylon strip for ease of handling.

Biotinylated primers were used in the amplification reactions to allow for later detection using a colorimetric assay as described in Example 8, above. The DRB amplification primers used were CRX28 (SEQ ID NO: 67) and CRX29 (SEQ ID NO: 68). The DRB1 amplification primers were CRX28 and CRX 37 (SEQ ID NO: 73). Both sets of primers are described in Example 8, above.

The two probe panels, one for hybridizing with the amplification products of the DRB amplification and the other for hybridizing with the amplification product from the DRB 1 -specific amplification, are shown below. The nucleotide sequence for each probe is found in the Sequence Listing section; the SEQ ID NO: for each probe is provided below.

| Probe Panel for the DRB Amplification | | | | |
|---|---|---|---|---|
| | Probe | Seq. ID No. | AA Sequence | Reactivity |
| 1 | DRB01 | SEQ ID NO: 79 | WLF | DR1 |
| 2 | GH104 | SEQ ID NO: 90 | WPR | DR2 |
| 3 | DRB46 | SEQ ID NO: 123 | YSTS | DR3, 11, 13, 14 |
| 4 | DRB48 | SEQ ID NO: 125 | V-H | DR4 |
| 5 | DRB207 | SEQ ID NO: 283 | G-YK | DR7 |
| 6 | GH102 | SEQ ID NO: 89 | YSTG | DR8, 12, 1404 |
| 7 | DRB209 | SEQ ID NO: 285 | K-D-F | DR9 |
| 8 | DRB20 | SEQ ID NO: 98 | EV | DR10 |
| 9 | DRB102 | SEQ ID NO: 178 | E | DR11 |
| 10 | DRB112 | SEQ ID NO: 188 | A-H | 1401, 1404 |
| 11 | DRB07 | SEQ ID NO: 84 | F-DE | 1103 |
| C | DRB42 | SEQ ID NO: 119 | TELGRP | ALL |

| Probe Panel for the DRB1 Amplificafion | | | | |
|---|---|---|---|---|
| | Probe | Seq. ID No. | AA Sequence | Reactivity |
| 12 | DRB223 | SEQ ID NO: 299 | S | * |
| 13 | DRB37 | SEQ ID NO: 114 | F-DR | * |
| 14 | DRB203 | SEQ ID NO: 279 | R | * |
| 15 | DRB163 | SEQ ID NO: 239 | K | 0401, 0409 |
| 16 | DRB118 | SEQ ID NO: 194 | R-E/RR-E | * |
| 17 | DRB02 | SEQ ID NO: 61 | I-DE | * |
| 18 | DRB38 | SEQ ID NO: 115 | K-GR | DR3 |
| 19 | DRB222 | SEQ ID NO: 298 | Y | 0301 |
| 20 | DRB232 | SEQ ID NO: 308 | I-DK | 1303 |
| 21 | DRB136 | SEQ ID NO: 212 | I-DR | 0803, 1201 (not DR7) |
| 22 | DRB198 | SEQ ID NO: 274 | V-S | DR7, 0803, 1201 |
| C | DRB42 | SEQ ID NO: 119 | TELGRP | ALL |

| | | | | |
|---|---|---|---|---|
| * sequence is found on a number of different alleles (see DRB1 amino acid alignment). | | | | |

DRB probes 1-8 are specific to the region about amino acids 9-13; probe 9 is specific for amino acid 58; probe 10 is specific for amino acids 57-60; and probe 11 is specific for amino acids 67-74. DRB1 probes 13-18, 20, and 21 are specific to amino acids 67-74; probe 12 is specific to amino acid 57; probe 20 is specific to amino acid 26; and probe 22 is specific for amino acids 57-60. The control probe is specific for amino acids 51-56.

It should be noted that the probes shown in the above panels can also be labeled and used in the DRB typing methods described in Example 7. The same hybridization and wash conditions would be used. Interpretation of the hybridization pattern would be as described below.

The kits were packaged as two boxes: one box contained the DRB reagents and the other box contained the DRB1 reagents. Packaged in each box was either DRB or DRB1 PCR mix, DNA control, and typing strips; 8 mM magnesium chloride solution; mineral oil; SA-HRP conjugate; chromogen (TMB); reaction tubes; and instructions. PCR contains the reagents necessary for a PCR with the exception of the magnesium chloride and the template DNA. Other reagents and equipment needed to perform the methods described were supplied by the kit user; all are commonly available commercially.

Several sample preparation procedures suitable for use in PCR amplifications are known in the art. A preferred procedure is the Chelex extraction method described in Singer-Sam et al., 1989, Amplifications 3:11, and Walsh et al., 1991, BioTechniques 10(4):506-513. For examples of other techniques for extracting nucleic acids from biological samples, see those described in Sambrook et al., Molecular Cloning: A Laboratory Manual (New York, Cold Spring Harbor Laboratory, 1989); Arrand, Preparation of Nucleic Acid Probes, in pp. 18-30, Nucleic Acid Hybridization: A Practical Approach (Ed Hames and Higgins, IRL Press, 1985); or, in PCR Protocols, Chapters 18-20 (Innis et al., ed., Academic Press, 1990).

Primer pairs for amplifying all DRB sequences and for amplifying specifically DRB1 sequences are used in two separate PCR reactions. Amplification reactions are essentially as described in Saiki et al., 1989, Proc. Natl, Acad. Sci. USA 86:6230-6234, with the modifications described below. Reactions are carried out using 25 µl of sample in a total volume of 100 µl, each reaction containing 50 mM KCL, 10 mM TrisHCl (pH 8.4), 1.5 mM MgCl₂, 10 µg Gelatin, 200 µM each dATP, dCTP, dGTP, and dTTP, 0.2 µM each biotinylated amplification primer, and 2.5 units of Thermus aquaticus DNA polymerase (PECI).

The same temperature profile is used for both amplification reaction, allowing both reaction to be carried out concurrently in the same thermocycler. The thermocycler is programmed for 35 cycles of the following temperature profile: denature at 95°C for 60 seconds, anneal at 60°C for 30 seconds, extend at 72°C for 60 seconds. The thermocycler is programmed to incubate the sample for an additional 7 minutes at 72°C following the last cycle.

The amplified DNA from the PCR is in double stranded form and must be denatured to allow hybridization with the oligonucleotide probes. The amplified DNA is denatured at 95°C for 5 to 10 minutes in the thermocycler and maintained at that temperature until used. Alternatively, amplified DNA which has been denatured can be transferred directly from the 95°C temperature into an ice bath. The rapid cooling stabilizes the DNA in a denatured form. The amplified DNA is then kept in the ice bath until it is required for the hybridization.

Twelve probes are affixed to each of two nylon membrane strips; one strip contains the probes to be used for hybridization with the DRB amplification product and the other strip contains the probes for hybridization with the DRB1-specific amplification product. Hybridization reactions are carried out in trays which hold each probe-containing strip in a separate well. The hybridization trays are commercially available from PECI and are typically supplied with the kit. The hybridization conditions described below are specific for the DRB hybridization. The DRB1 DNA hybridization protocol differs from the DRB protocol in that the DRB1 hybridization is at 55°C instead of the 50°C. All other aspects of the protocol are identical.

In each well containing a strip, 3 ml of pre-warmed hybridization solution (4X SSPE and 0.5% weight/volume SDS) are added followed by 25 µl of amplified DNA. After careful mixing of the tray contents, the tray is placed in a 50°C shaking water bath and incubated at 50°C for 20 minutes at about 50 rpm.

The strips are washed initially in 10 ml of wash solution (1.0X SSPE and 0.1% weight/volume SDS) at room temperature for several seconds prior to the stringent wash and each well is then aspirated. The temperature and timing of the stringent wash are critical. Pre-warmed wash solution (10 ml) are added to each well and the tray is incubated in a 50°C shaking water bath for 12 (± 2) minutes at about 50 rpm. After aspirating each well, 10 ml of wash solution are added to each well, the tray is incubated at room temperature for 5 minutes on an orbital shaker at about 50 rpm_{'} and the wells are again aspirated.

To each well containing a strip are added 3 ml of wash/enzyme conjugate solution (from a solution of 3.3 ml of wash solution and 27 µl of enzyme conjugate prepared within 15 minutes of use). The tray is incubated at room temperature for 20 minutes on an orbital shaker at approximately 50 rpm. The solution is aspirated from each well and the strips are washed in 10 ml of wash solution at room temperature for 5 minutes on an orbital shaker at approximately 50 rpm. Finally, the wash solution is aspirated from each well and the strips are ready for the color development step.

The color development steps are identical for both DRB and DRB1. To each well is added 10 ml of citrate buffer (100 mM Sodium Citrate, pH = 5.0) and the tray is place on an orbital shaker for 5 minutes at approximately 50 rpm. Preferably, the color development solution is prepared during this time (no more than 10 minutes before use). The color development solution for each well is made from 10 ml of citrate buffer, 10 µl of 3% hydrogen peroxide, and 0.5 ml TMB chromogen solution (commercially available from PECI) mixed gently (do not vonex).

The tray is removed from the orbital shaker, the citrate buffer is aspirated, and 10 ml of the freshly-made color development solution is added to each well. The tray is shielded from light beginning with the color development step using an aluminum foil covering. The strips are developed at room temperature for up to 30 minutes on an orbital shaker at about 50 rpm. The development can be terminated as soon as the desired signal intensity has been reached.

After the desired signal intensity has been reached, the tray is removed from the shaker and the contents of each well are aspirated. The color development is stopped by washing with 10 ml of deionized water in each well. The water is added, the tray is then shaken at room temperature for 5 minutes on an orbital shaker at about 50 rpm, and the contents of each well are slowly poured off. At least three washes should be performed.

The strips can be stored protected from light in the typing trays at between 2°C and 8°C for 2 to 3 days. The strips should be photographed wet for a permanent record Strips can also be dried and stored in the dark, though some fading can occur.

Results are interpreted by reading the pattern of blue dots on the DNA Probe strips; each blue dot indicates that a biotinylated amplified product hybridized with the immobilized probe. The intemal control probe (DRB42), which detects all of the DRB alleles, is designed to produce a dot of intensity equal to or less than the intensity of other positive dots on the strip. This provides a guide as to the minimum dot intensity that should be scored as a positive.

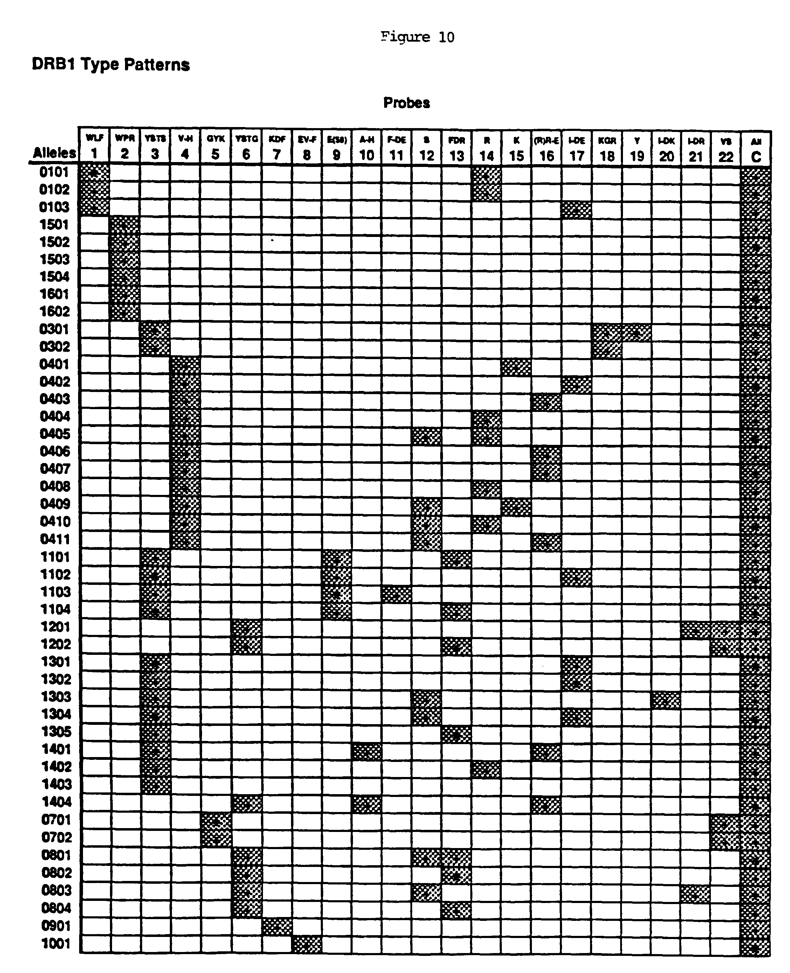

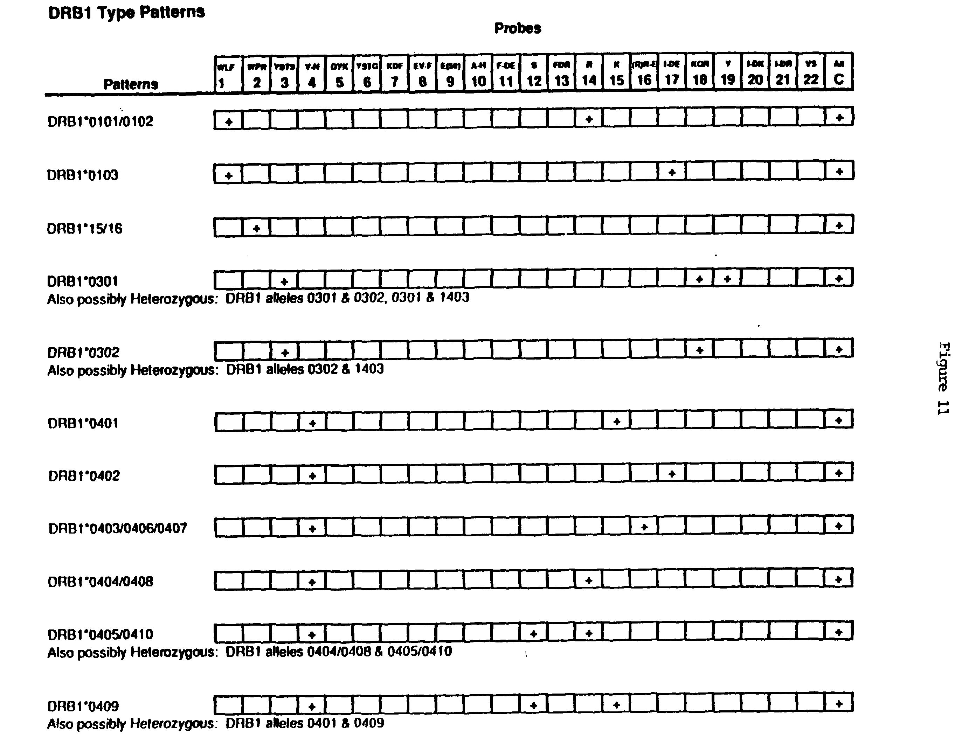

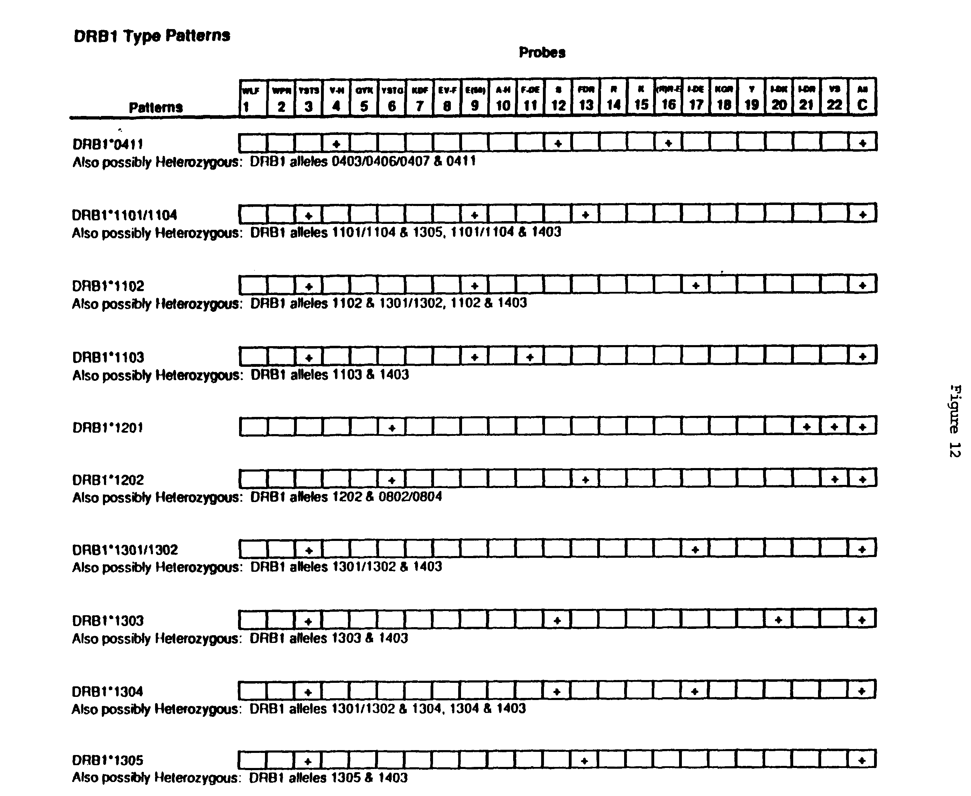

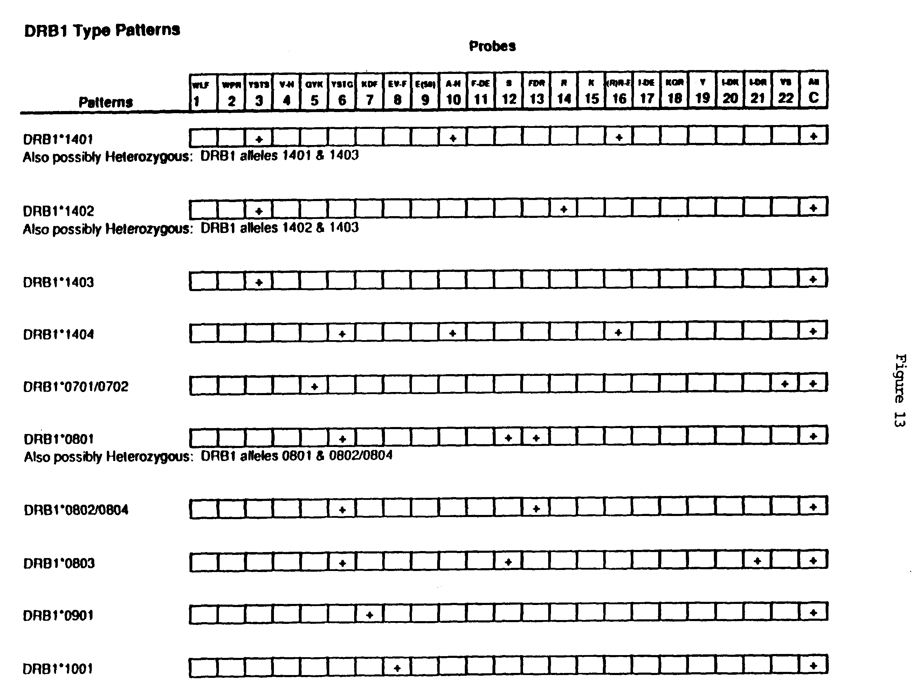

The probes of the above kit distinguish 31 HLA DRB 1 types. Some of these probe-defined types are sets of related alleles. Figure 10 shows the probe hybridization pattern for each allele of the 1990 allele set. Figures 11, 12, and 13 provide the possible interpretations for each probe hybridization pattern. Strip results can easily be interpreted directly using Figures 11-13.

A convenient alternative to manual interpretation of results is provided by a computer program which accepts the probe hybridization pattern as input, matches the input pattern to known allele patterns, and provides the possible interpretations. Suitable algorithms for matching the probe hybridization pattern to the possible allele combinations include simple table lookup and decision tree algorithms. Program input can be entered either manually or from an automated strip reader that detects the intensity of the blue hybridization dots.

As noted above, this reverse dot blot typing system does not fully descriminate among all possible types. For example, the system does not subtype DR2 serotypes. Certain heterozygous combinations also cannot be fully resolved. All of these can be resolved with additional primer pairs for allele-specific amplifications and probes, as discussed in Example 12, below.

Because of the hybridization positions of primers CRX28 and CRX29, it is not possible to detect amino acid variations which occur at position 86 when amplifying with this primer pair. Consequently, alleles which differ only at position 86 cannot be distinguished. There are seven pairs of alleles known which differ only in the present of a glycine or a valine at position 86; these allele pairs are listed below.

| Allele pairs differing only at amino acid position 86 |
|---|
| DRB1*1501 and DRB1*1502 |
| DRB1*0302 and DRB1*0303 |
| DRB1*0403 and DRB1*0407 |
| DRB1*0404 and DRB1*0408 |
| DRB1*1101 and DRB1*1104 |
| DRB1*1301 and DRB1*1302 |
| DRB1*0802 and DRB1*0804 |

### Example 10

### General DRB1 Typing Strategy II

Example 4 describes one strategy for typing DRB 1 alleles; an alternate strategy is described here. In order to obtain complete discrimination of all 45+ alleles at the DRB 1 locus, a two stage assay is used. The first stage involves amplification of all samples with the DRB general primers, GH46 and GH50. The resulting PCR product is immobilized and probed (as in Example 4) with a first panel of probes to determine which allele specific amplifications, if any, need to be performed and the amplification products screened in the second stage. The amplification and hybridization protocols are essentially as described in Example 4. Interpretation of the hybridization pattern by reference to the allele specificities listed with each probe panel.

### First Stage Typing

The first panel of probes is a similar to the probe panel shown in Table 4, differing by only a few probes. In the allele specificities shown in the probe panels below, an "X" as the last digit of an allele designation indicates that all alleles beginning with the specified number are recognized. For example, 030X is equivalent to 0301, 0302, and 0303.

| First Panel of HLA DRB Typing SSO Probes | | | | |
|---|---|---|---|---|
| Probe | SEQ ID NO: | Epitope | Alleles | Wash (SSPE, °C.) |
| CRX33 | SEQ ID NO: 69 | "W-L-F" | 010X | 0.4X, 42 |
| GH104 | SEQ ID NO: 90 | "W-P-R" | 150X, 160X | 0.2X, 42 |
| GH56 | SEQ ID NO: 86 | "YSTS" | 030X, 110X, 130X, 1401, 1402 | 0.2X, 42 |
| GH59 | SEQ ID NO: 87 | "V-H" | 040X | 0.2X, 42, 20 |
| CRX49 | SEQ ID NO: 74 | "G-YK" | 070X | 1.0X, 42 |
| GH102 | SEQ ID NO: 89 | "YSTG" | 080X, 120X, 1404 | 0.1X, 42 |
| GH111 | SEQ ID NO: 92 | "K-D-F" | 0901 | 0.4X, 42 |
| CRX34 | SEQ ID NO: 70 | "EV" | 1001 | 0.4X, 42 |
| GH122 | SEQ ID NO: 93 | "E" | 110X | 0.2X, 42 |
| CRX61 | SEQ ID NO: 80 | "S" | 0405, 0409, 0410, 0411, 0801, 0803, 1304, 1305 | 0.1X, 42 |
| CRX23 | SEQ ID NO: 66 | "A-H" | 1401, 1404 | 0.1X, 42 |
| CRX06 | SEQ ID NO: 61 | "I-DE" | 0103, 0402, 1102, 1301, 1302, 1304 | 0.1X, 42 |
| CRX35 | SEQ ID NO: 71 | "F-DR" | 1601, 1101, 1104, 1305, 0801, 0802, 0804, 1202 | 0.2X, 42 |
| CRX68 | SEQ ID NO: 84 | "F-DE" | 1103 | 0.2X, 42 |
| CRX62 | SEQ ID NO: 81 | "I-DK" | 1303 | 0.2X, 42 |
| CRX12 | SEQ ID NO: 63 | DRB "ALL" | All | 0.2X, 42 |

The probes are hybridized and then washed for 15 minutes at 42°C, except for GH59, which is washed for 20 minutes at 42°C. All SSPE wash solutions contain 0.1% SDS. Each probe is conjugated to HRP at the 5'-end. Based on the hybridization pattern obtained from the first probe panel, a subtyping step may be necessary using allele-specific amplification.

Each of the types, DR7, DR9, and DR10 have only one identifiable allele and are identified directly from the first stage probe panel. There are actually two alleles specifying the DR7 type, DRB1*0701 and DRB1*0702. However, these two alleles differ only in the third exon and cannot be distinguished by the present methods which amplify and detect sequences from the second exon. Therefore, only one distinct allele is identifiable by the methods of the present invention.

### Second Stage Subtyping

### DR2

Samples that contained a positive signal for W-P-R (GH104), which all type as DR2 serologically, are amplified with a primer pair specific for alleles with the W-P-R epitope, AB83 and AB60. The resulting product is probed with the following panel.

| Panel of HLA DR2 Typing SSO Probes | | | | |
|---|---|---|---|---|
| Probe | SEQ ID NO: | Epitope | Alleles | Wash (SSPE, °C.) |
| DRB63 | SEQ ID NO: 140 | "I-A" | 150X | 0.1X, 42 |
| DRB113 | SEQ ID NO: 189 | "-DR" | 1602 | 0.1X, 42 or 0.4X, 50 |
| CRX35 | SEQ ID NO: 71 | "F-DR" | 1601 | 0.2X, 42 |
| CRX57 | SEQ ID NO: 78 | "V" | 1501, 1502 | 0.2X, 42 |
| CRX56 | SEQ ID NO: 77 | "G" | 1502, 160X | 0.2X, 42 |
| DRB196 | SEQ ID NO: 272 | "H" (30) | 1503 | 1.0X, 42 |
| DRB197 | SEQ ID NO: 273 | "Y"(30) | 1501, 1502, 160X | 1.0X, 42 |
| GH104 | SEQ ID NO: 90 | "W-P-R" | DR2 | 0.2X, 42 |
| CRX12 | SEQ ID NO: 63 | DRB "ALL" | All | 0.2X, 42 |

The probes shown are hybridized and then washed for 15 minutes at 42°C. All SSPE wash solutions contain 0.1% SDS. Each probe is conjugated to HRP at the 5'-end.

### DR3, DR5, DRw6

Samples that contained a positive signal for the YSTS epitope with probe GH56 are either DR3, DR5, or DRw6. These alleles are amplified specifically with AB82 and AB60. The alleles can then be distinguished using the probe panel shown below.

| Panel of HLA DR3, 5, w6 Typing SSO Probes | | | | |
|---|---|---|---|---|
| Probe | SEQ ID NO: | Epitope | Alleles | Wash (SSPE, °C.) |
| CRX50 | SEQ ID NO: 75 | KGR | 030X | 0.2X, 50 |
| GH125 | SEQ ID NO: 94 | Y | 0301 | 0.2X, 50 |
| DRB180 | SEQ ID NO: 256 | A | 030X, 1301, 1302, 1305, 1402, 1403, 1405 | 0.2X, 42 |
| GH122 | SEQ ID NO: 93 | E | 110X | 0.2X, 42 |
| CRX61 | SEQ ID NO: 80 | S | 1303, 1304 | 0.2X, 42 |
| CRX23 | SEQ ID NO: 66 | A-H | 1401 | 0.1X, 42 |
| CRX06 | SEQ ID NO: 61 | I-DE | 1102, 1301-02 | 0.2X, 42 |
| CRX62 | SEQ ID NO: 81 | I-DK | 1303 | 0.2X, 42 |
| CRX68 | SEQ ID NO: 84 | FDE | 1103 | 0.2X, 42 |
| CRX35 | SEQ ID NO: 71 | FDR | 1101, 1104, 1305 | 0.2X, 42 |

| Probe | SEQ ID NO: | Epitope | Alleles | Wash (SSPE, °C.) |
|---|---|---|---|---|
| CRX04 | SEQ ID NO: 60 | R | 1402 | 0.1X, 42 |
| CRX57 | SEQ ID NO: 78 | V | 0301, 0303, 1102, 1104, 1301, 1304, 1305, 1405 | 0.2X, 42 |
| CRX56 | SEQ ID NO: 77 | G | 0302, 1101, 1302, 1303, 1305, 1402, 1403 | 0.2X, 42 |
| GH56 | SEQ ID NO: 86 | YSTS | DR3, DR5, DRw6 | 0.2X, 42 |
| CRX12 | SEQ ID NO: 63 | DRB "ALL" | ALL | 0.2X, 42 |

The probes shown are hybridized and then washed for 15 minutes at 42°C. All SSPE wash solutions contain 0.1% SDS. Each probe is conjugated to HRP at the 5'-end.

### DR4

Samples that contained a positive signal for the VH epitope with probe GH59 are DR4. These alleles are amplified specifically with AB54 and AB60. The alleles can then be distinguished using the probe panel shown below.

| Probe | Epitope | Allele | Wash |
|---|---|---|---|
| CRX61 | S | 0405, 0409, 0410, 0411 | 0.2X, 42 |
| CRX64 | K | 0401, 0409 | 2X, 40 |
| CRX04 | R | 0403, 0404, 0405, 0406, 0407, 0408, 0410, 0411 | 0.1X, 42 |
| CRX15 | R-E | 0403, 0406, 0407, 0411 | 0.4X, 55 |
| CRX06 | IDE | 0402 | |
| CRX57 | V | 0402, 0403, 0404, 0406, 0410, 0411 | 0.2X, 42 |
| CRX56 | G | 0401, 0405, 0407, 0408, 0409 | 0.2X, 42 |
| GH59 | V-H | DR4 | 0.2X, 42 |
| CRX12 | DRB"ALL" | ALL | 0.2X, 42 |

The probes shown are hybridized and then washed for 15 minutes at 42°C with the exception of GH59, which is washed for 20 minutes. All SSPE wash solutions contain 0.1% SDS. Each probe is conjugated to HRP at the 5'-end.

### DR1, DRw8, and DRw12

Samples that contained a positive signal for the WLF epitope with probe CRX33 or positive for the YSTG epitope with probe GH102 are amplified with the DRB1-specific primers, GH46 and CRX37. This also amplifies all DRB1 alleles except DR2, DR7, and DR9. The alleles can then be distinguished using the probe panel shown below.

| Probe | Epitope | Allele | Wash (SSPE, °C) |
|---|---|---|---|
| CRX33 | WLF | 010X | 0.4X, 42 |
| CRX04 | R | 0101, 0102, 0403, 0404, 0405, 0406, 0407, 0408, 0410, 0411 | 0.1X, 42 |
| CRX06 | IDE | 0103, 1102, 1301, 1302 | 0.2X, 42 |
| CRX57 | V | 0301, 0402, 0403, 0404, 0406, 0410, 0411, 1102, 1103, 1104, 1301, 1304, 1401, 1404, 1405, 0804 | 0.2X, 42 |
| DRB181 | AV | 0102, 1201, 1202 | 0.1X, 42 |
| CRX56 | G | 0101, 0103, 0302, 0401, 0405, 0407, 0408, 0409, 1101, 1302, 1303, 0801, 0802, 0803, 1305, 1402, 1403 | 0.2X, 42 |
| GH102 | YSTG | 0801, 0802, 0803, 0804, 1201, 1202, 1404 | 0.1X, 42 |
| GH54 | V-S | 1201, 1202 | 0.4X, 42 |
| CRX63 | I-DR | 1201, 0803 | 0.2X, 42 |
| CRX35 | F-DR | 0801, 0802, 1202, 1101, 1104, 1305 | 0.2X, 42 |
| CRX12 | DRB "ALL" | ALL | 0.2X, 42 |

The probes shown are hybridized and then washed for 15 minutes at 42°C. All SSPE wash solutions contain 0.1% SDS. Each probe is conjugated to HRP at the 5'-end.

Since some epitopes recognized by the probes are shared in many alleles (such as FDR), the results obtained from the hybridization pattern with the above probe panel are compared to other allele specific amplifications to determine if the probe is associated with a DR1, DRw8, or DRw12, or is associated with another allele also amplified with the DRB1-specific primers. The design of primers specific for the WLF and YSTG epitopes will simplify the interpretation of the typing results.

### Example 11

### Allele Subtyging in Heterozygotes

The pattern of hybridization with the probe panels of Example 10 alone may not be sufficient to unambiguously determine which alleles are present in certain heterozygous individuals. The probe hybridization pattern indicates which allele epitopes are present in the sample, but to determine the specific alleles present, it may be necessary to know which epitopes occur on which allele. Most frequently, such ambiguity does not arise because the epitope origin can usually be inferred from the limited possibilities of allelic combinations. The few cases which do arise can be resolved using allele-specific amplification.

In some DR5/DRw6 heterozygotes, the following probe pattern can result: 
Probe:  GH56 + GH122 + DRB180 + CRX06 + CRX35 + CRX56 + CRX57
Epitope: YSTS   E       DA       I-DE    F-DR    G       V
 Three different DR5/DRw6 heterozygote allele combinations produce this probe hybridization pattern; these combinations are listed below. These heterozygote allele combinations cannot be distinguished by any other probe.
DRB1*1101 and DRB1*1301
DRB1*1104 and DRB1*1302
DRB1*1102 and DRB1*1305

To distinguish these possibilities, additional primers were designed to take advantage of the unique dimorphism at position 86. All DRB1 alleles contain either a valine or a glycine at position 86. In each of the heterozygotes listed above, one of the alleles contains a valine at position 86 and the other contains a glycine. DRB1*1301, DRB1*1104, and DRB1*1102 each contain a valine at position 86; DRB1*1101, DRB1*1302, and DRB1*1305 each contain a glycine. Amplification using either a V or G specific primer in combination with a group specific primer (W-PR, VH, or YSTS) allows single allele amplification based on the polymorphism at position 86. In this manner, one of the two alleles present in each heterozygote can be selectively amplified and determined by direct probe hybridization. The second allele can be inferred from knowledge of the first allele based on the possible allelic combinations.

Subtyping DR5/DRw6 heterozygotes by single allele amplification was achieved using the PCR primer pairs AB82/RAP05 and AB82/RAP06. Amplification was carried out as in Example 10 except that the PCR buffer contained a final concentration of 1.0 mM MgCl₂. The thermocycler was programmed for 35 cycles, each with the following temperature profile: ramp to 94°C; 30 seconds denaturation at 94°C; 30 seconds annealing and extension at 70°C. Probe hybridization was carried out as before. The sequences of the V-specific (RAP06) and G-specific (RAP05) primers are shown below and in the sequence listing section. Although each was used in combination with AB82, other group specific primers would be suitable.

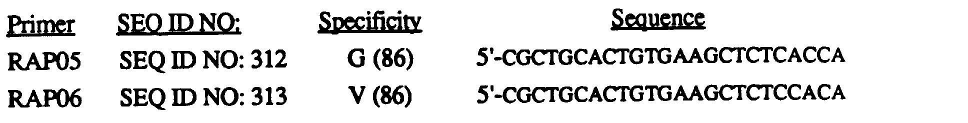

### Example 12

### DRB Two-Stage Typing Kit

The typing kit described here is designed to uniquely identify the greatest number of types possible. The primers and probes are designed to provide rapid yet complete typing for the DRB1 and DRB3 loci in a reverse dot blot format. In order to obtain complete discrimination of all 45+ alleles at the DRB1 locus and the 3 alleles at the DRB3 locus, a two stage assay is used. The first stage involves amplification of all samples with the DRB general primers, DRB27 (SEQ ID NO: 105) and DRB28 (SEQ ID NO: 106). The resulting PCR product is screened against a strip with 13 probes to determine which allele specific amplifications, if any, need to be done and screened in the second stage.

Amplification and hybridization are carried out as in Example 9. The amplification product from the DRB general amplification is screened against a panel of probes containing eight probes specific for the first region of variability in the DRBllocus, four probes specific for the three DRB3 alleles (52a, 52b, 52c), and one control probe. Additional probes can be added to this strip to type for the DRB5 locus which has three alleles and is found on DR2 haplotypes. The specific hybridization regions and types recognized are listed below.

| | Type recognized | Sequence Probed |
|---|---|---|
| 1 | DR1 | WLF |
| 2 | DR2 | WPR |
| 3 | DR3, 5, 6 | YSTS |
| 4 | DR4 | VH |
| 5 | DR7 | GYK |
| 6 | DR8, 12 | YSTG |
| 7 | DR9 | KDF |
| 8 | DR10 | EV |
| 9 | 52a | LRS |
| 10 | 52b, 52c | LLS |
| 11 | 52b | to be designed |
| 12 | 52c | to be designed |
| 13 | All | TELGRP |

The DRB3 type is determined from the results of this assay and, based on the results of this assay, it is determined which allele-specific amplifications (ASAs), if any, need to be done. Primers for five different ASAs are provided, though the most needed for typing any one sample is two. The same 3' primer, AB60, for example, can be used for all five ASAs and typically would be included as a component of the PCR reaction mix provided in a kit. Amplification specificity is conferred by the 5' primers. The DRB type amplified and the epitope recognized by each of the 5' primers are listed below. Primers 2, 3, and 4 have been designed and tested extensively and are discussed in the examples, above.

| | Type Amplified | Hybridization Sequence |
|---|---|---|
| 1 | DR1 | WLF |
| 2 | DR2 | WPR |
| 3 | DR3, 5, 6 | YSTS |
| 4 | DR4 | VH |
| 5 | DR8, 12 | YSTG |

The second stage involves a series of probes which permit fine typing of the amplified products from the ASAs. In one embodiment, all probes are attached to a single strip and this single strip is used to type the product from all ASAs. Probes specific to the following regions are sufficient for typing the results of the ASAs.

| Region | Epitopes |
|---|---|
| 57-60 | DAE, S, E, A-H, V-S |
| 67-74 | R, I-DE, I-A, F-DR, DR, KGR, K, R-E/RR-E, F-DE, I-DK, I-DR, DR-L |
| 85-86 G, V, AV | |
| Other | E (a.a. 20), H (a.a. 30), Y (a.a. 26), S (a.a. 37) |

The "other" region polymorphisms are very rare in Caucasian populations. However, an important goal of the present method is to be able to type non-Caucasian populations. Additional probes at these positions can be included to deal with possible ambiguities in recognizing heterozygotes versus homozygotes.

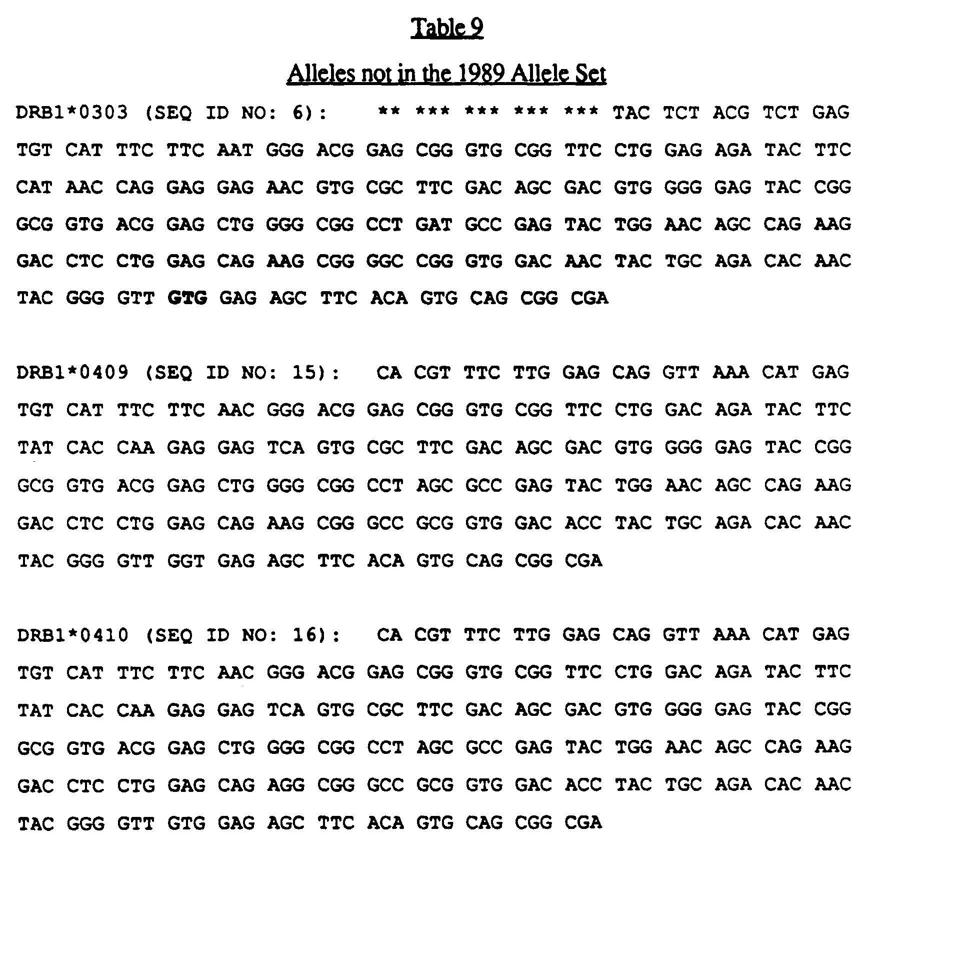

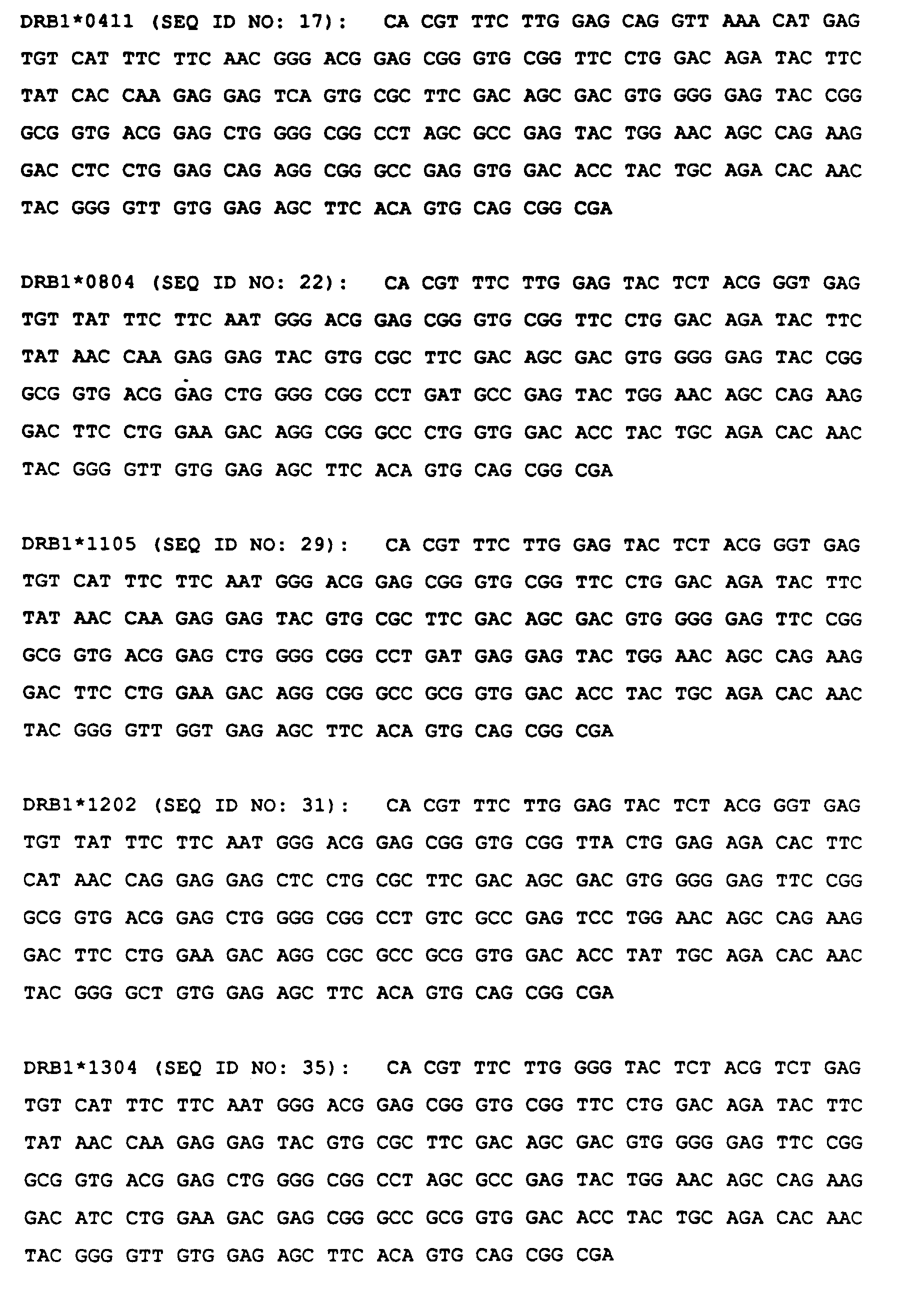

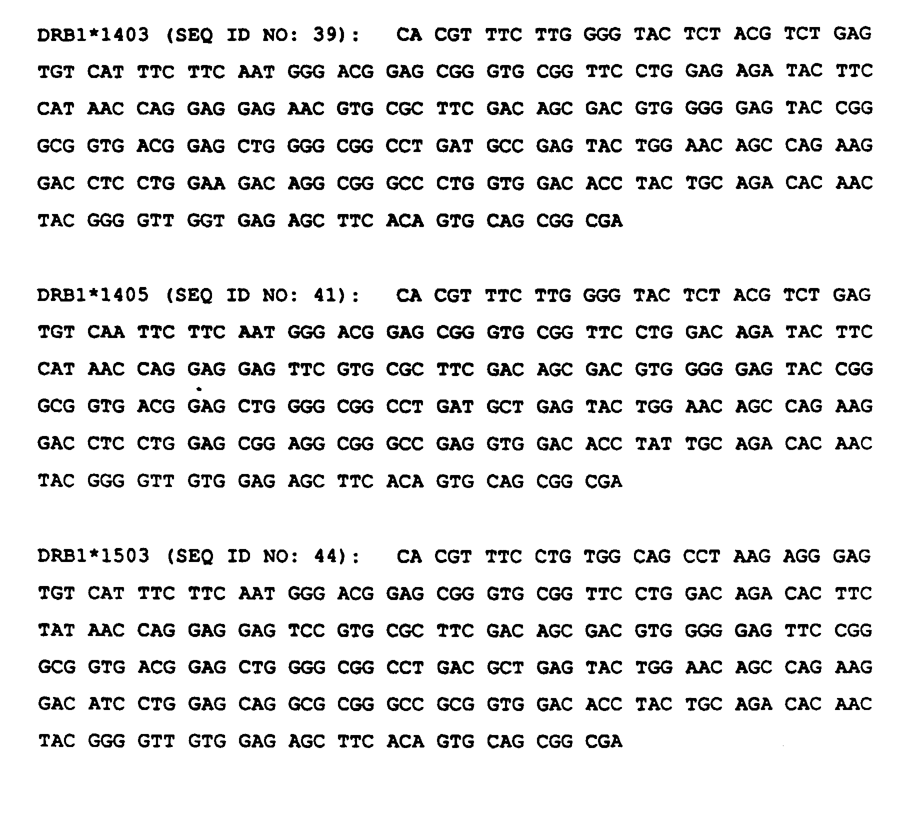

## Claims

1. A method for determining the DRbeta DNA type of nucleic acid in a sample, which method comprises:
(a) amplifying any nucleic acids in the sample that contain a DRbeta gene second exon using the primer pair GH46 (SEQ ID NO:67) and GH50 (SEQ ID NO:68);
(b) hybridizing said nucleic acid amplified in step (a) to a first panel of oligonucleotide probes under conditions such that said probes hybridize only to exactly complementary sequences greater than ten nucleotides in length;
(c) amplifying a specific subset of nucleic acids in the sample that contain DRbeta gene second exon sequences using the primer pairs GH46 (SEQ ID NO:67) and CRX37 (SEQ ID NO:73) or GH46 (SEQ ID NO:67) and DRB30 (SEQ ID NO:107);
(d) hybridizing said nucleic acid amplified in step (c) with a second panel of oligonucleotide probes under conditions such that said probes hybridize only to exactly complementary sequences greater than ten nucleotides in length; and
(e) determining from the pattern of probe hybridization in steps (b) and (d) the DRbeta alleles from which the DRbeta DNA in said sample originates.

2. The method of claim 1, wherein the amplifying of steps (a) and (c) is carried out by a polymerase chain reaction.

3. The method of claims 1 or 2, wherein said first and second panel of oligonucleotide probes each comprise two or more probes, and wherein said probes can hybridize to DRbeta gene second exon sequences, which sequences are selected from the group consisting of sequences that encode amino acid residues 9 to 16, amino acid residues 25 to 34, amino acid residues 67 to 74, and amino acid residue 86.

4. The method of claim 3, wherein the first panel of oligonucleotide probes comprises two or more probes selected from the group consisting of probes CRX60 (SEQ ID NO: 79), GH105 (SEQ ID NO: 91), GH104 (SEQ ID NO: 90), GH59 (SEQ ID NO: 57), CRX06 (SEQ ID NO: 61), GH122 (SEQ ID NO: 93), CRX23 (SEQ ID NO: 66), CRX35 (SEQ ID NO: 71), CRX49 (SEQ ID NO: 74), GH102 (SEQ ID NO:89), GH111(SEQ ID NO: 92), CRX34 (SEQ ID NO: 70), CRX04 (SEQ ID NO: 60), GH56 (SEQ ID NO: 86), CRX68 (SEQ ID NO: 84), and CRX12 (SEQ ID NO: 63).

5. The method of claim 3, wherein the second panel of oligonucleotide probes comprises two or more probes selected from the group consisting of probes GH125 (SEQ ID NO: 94), CRX50 (SEQ ID NO: 68), CRX53 (SEQ ID NO: 76), CRX15 (SEQ ID NO: 64), CRX62 (SEQ ID NO: 81), CRX63 (SEQ ID NO: 82), CRX61(SEQ ID NO: 80), GH54 (SEQ ID NO: 85), CRX56 (SEQ ID NO: 77), CRX57 (SEQ ID NO: 78), and CRX12 (SEQ ID NO: 63).

6. The method of claim 3, wherein the primers used in step (a) are GH46 (SEQ ID NO: 67) and GH50 (SEQ ID NO: 68), the primers used in step (c) are GH46 and CRX37 (SEQ ID NO: 73), the first panel of oligonucleotide probes comprises probes CRX60 (SEQ ID NO: 79), GH105 (SEQ ID NO: 91), GH104 (SEQ ID NO: 90), GH59 (SEQ ID NO: 57), CRX06 (SEQ ID NO: 61), GH122 (SEQ ID NO: 93), CRX23 (SEQ ID NO: 66), CRX35 (SEQ ID NO: 71), CRX49 (SEQ ID NO: 74), GH102 (SEQ ID NO: 89), GH111 (SEQ ID NO: 92), CRX34 (SEQ ID NO: 70), CRX04 (SEQ ID NO: 60), GH56 (SEQ ID NO: 86), CRX68 (SEQ ID NO: 84), and CRX12 (SEQ ID NO: 63), and the second panel of oligonucleotide probes comprises probes GH125 (SEQ ID NO: 94), CRX50 (SEQ ID NO: 68), CRX53 (SEQ ID NO: 76), CRX15 (SEQ ID NO: 64), CRX62 (SEQ ID NO: 81), CRX63 (SEQ ID NO: 82), CRX61 (SEQ ID NO: 80), GH54 (SEQ ID NO: 85), CRX56 (SEQ ID NO: 77), CRX57 (SEQ ID NO: 78), and CRX12 (SEQ ID NO: 63).

7. The method of claim 1, wherein said first panel of probes comprises two or more probes selected from the group consisting of probes DRB01 (SEQ ID NO: 79), GH104 (SEQ ID NO: 90), DRB46 (SEQ ID NO: 123), DRB48 (SEQ ID NO: 125), DRB207 (SEQ ID NO: 283), GH102 (SEQ ID NO: 89), DRB209 (SEQ ID NO: 285), DRB20 (SEQ ID NO: 98), DRB102 (SEQ ID NO: 178), DRB112 (SEQ ID NO: 188), DRB07 (SEQ ID NO: 84), and DRB42 (SEQ ID NO: 119).

8. The method of claim 1, wherein said second panel of probes comprises two or more probes selected from the group consisting of probes DRB223 (SEQ ID NO: 299), DRB37 (SEQ ID NO: 144), DRB203 (SEQ ID NO: 279), DRB163 (SEQ ID NO: 239), DRB118 (SEQ ID NO: 194), DRB02 (SEQ ID NO: 61), DRB38 (SEQ ID NO: 115), DRB222 (SEQ ID NO: 298), DRB232 (SEQ ID NO: 308), DRB 136 (SEQ ID NO: 212), DRB198 (SEQ ID NO: 274), and DRB42 (SEQ ID NO: 119).

9. The method of claim 2, wherein the primers used in step (a) are CRX28 (SEQ ID NO: 67) and CRX29 (SEQ ID NO: 68), the primers used in step (c) are CRX28 and CRX37 (SEQ ID NO: 73), the first panel of oligonucleotide probes comprises probes DRB01 (SEQ ID NO: 79), GH104 (SEQ ID NO: 90), DRB46 (SEQ ID NO: 123), DRB48 (SEQ ID NO: 125), DRB207 (SEQ ID NO: 283), GH102 (SEQ ID NO: 89), DRB209 (SEQ ID NO: 285), DRB20 (SEQ ID NO: 98), DRB 102 (SEQ ID NO: 178), DRB112 (SEQ ID NO: 188), DRB07 (SEQ ID NO: 84), and DRB42 (SEQ ID NO: 119), and the second panel of oligonucleotide probes comprises probes DRB223 (SEQ ID NO: 299), DRB37 (SEQ ID NO: 144), DRB203 (SEQ ID NO: 279), DRB163 (SEQ ID NO: 239), DRB118 (SEQ ID NO: 194), DRB02 (SEQ ID NO: 61), DRB38 (SEQ ID NO: 115), DRB222 (SEQ ID NO: 298), DRB232 (SEQ ID NO: 308), DRB136 (SEQ ID NO: 212), DRB198 (SEQ ID NO: 274), and DRB42 (SEQ ID NO: 119).

10. The method of claim 1, wherein the second panel of probes comprises two or more probes selected from a third panel of probes, wherein said third panel is selected from the group consisting of panels GH104 (SEQ ID NO: 90), DRB63 (SEQ ID NO: 140), DRB113 (SEQ ID NO: 189), CRX35 (SEQ ID NO: 71), CRX57 (SEQ ID NO: 78), CRX56 (SEQ ID NO: 77), DRB196 (SEQ ID NO: 272), DRB197 (SEQ ID NO: 273), DRB215 (SEQ ID NO: 291), and DRB216 (SEQ ID NO: 292); CRX50 (SEQ ID NO: 75), GH125 (SEQ ID NO: 94), DRB180 (SEQ ID NO: 256), GH122 (SEQ ID NO: 93), CRX61 (SEQ ID NO: 80), CRX23 (SEQ ID NO: 66), CRX06 (SEQ ID NO: 61), CRX62 (SEQ ID NO: 81), CRX68 (SEQ ID NO: 84), CRX35 (SEQ ID NO: 71), CRX04 (SEQ ID NO: 60), CRX57 (SEQ ID NO: 78), CRX56 (SEQ ID NO: 77), and GH56 (SEQ ID NO: 86); CRX61 (SEQ ID NO: 80), CRX64 (SEQ ID NO: 83), CRX04 (SEQ ID NO: 60), CRX15 (SEQ ID NO: 64), CRX06 (SEQ ID NO: 61), CRX57 (SEQ ID NO: 78), CRX56 (SEQ ID NO: 77), GH59 (SEQ ID NO: 87); and CRX33 (SEQ ID NO: 69), CRX04 (SEQ ID NO: 60), CRX06 (SEQ ID NO: 61), CRX57 (SEQ ID NO: 78), DRB181 (SEQ ID NO: 257), CRX56 (SEQ ID NO: 77), GH102 (SEQ ID NO: 89), GH54 (SEQ ID NO: 85), CRX63 (SEQ ID NO: 82), CRX35 (SEQ ID NO: 71).

11. The method of claim 2, wherein the primers used in step (a) are GH46 (SEQ ID NO: 67) and GH50 (SEQ ID NO: 68); the primers used in step (c) are selected from the group consisting of AB83 (SEQ ID NO: 59) and AB60 (SEQ ID NO: 57), AB82 (SEQ ID NO: 58) and AB60, AB54 (SEQ ID NO: 56) and AB60, and GH46 (SEQ ID NO: 67) and CRX37 (SEQ ID NO: 73); the first panel of oligonucleotide probes comprises two or more probes selected from the group consisting of probes CRX33 (SEQ ID NO: 69), GH104 (SEQ ID NO: 90), GH56 (SEQ ID NO: 86), GH59 (SEQ ID NO: 87), CRX49 (SEQ ID NO: 74), GH102 (SEQ ID NO: 89), GH111 (SEQ ID NO: 92), CRX34 (SEQ ID NO: 70), GH122 (SEQ ID NO: 93), CRX61 (SEQ ID NO: 80), CRX23 (SEQ ID NO: 66), CRX06 (SEQ ID NO: 61), CRX35 (SEQ ID NO: 71), CRX68 (SEQ ID NO: 84), and CRX62 (SEQ ID NO: 81); and the second panel of probes comprises two or more probes selected from a third panel of probes, wherein said third panel is selected from the group consisting of panels GH104 (SEQ ID NO: 90), DRB63 (SEQ ID NO: 140), DRB113 (SEQ ID NO: 189), CRX35 (SEQ ID NO: 71), CRX57 (SEQ ID NO: 78), CRX56 (SEQ ID NO: 77), DRB196 (SEQ ID NO: 272), DRB197 (SEQ ID NO: 273), DRB215 (SEQ ID NO: 291), and DRB216 (SEQ ID NO: 292); CRX50 (SEQ ID NO: 75), GH125 (SEQ ID NO: 94), DRB 180 (SEQ ID NO: 256), GH122 (SEQ ID NO: 93), CRX61(SEQ ID NO: 80), CRX23 (SEQ ID NO: 66), CRX06 (SEQ ID NO: 61), CRX62 (SEQ ID NO: 81), CRX68 (SEQ ID NO: 84), CRX35 (SEQ ID NO: 71), CRX04 (SEQ ID NO: 60), CRX57 (SEQ ID NO: 78), CRX56 (SEQ ID NO: 77), and GH56 (SEQ ID NO: 86); CRX61 (SEQ ID NO: 80), CRX64 (SEQ ID NO: 83), CRX04 (SEQ ID NO: 60), CRX15 (SEQ ID NO: 64), CRX06 (SEQ ID NO: 61), CRX57 (SEQ ID NO: 78), CRX56 (SEQ ID NO: 77), GH59 (SEQ ID NO: 87); and CRX33 (SEQ ID NO: 69), CRX04 (SEQ ID NO: 60), CRX06 (SEQ ID NO: 61), CRX57 (SEQ ID NO: 78), DRB181 (SEQ ID NO: 257), CRX56 (SEQ ID NO: 77), GH102 (SEQ ID NO: 89), GH54 (SEQ ID NO: 85), CRX63 (SEQ ID NO: 82), CRX35 (SEQ ID NO: 71).

12. The method of claim 2, wherein the primers used in step (a) are GH46 (SEQ ID NO: 67) and GH50 (SEQ ID NO: 68); the primers used in step (c) are selected from the group consisting of AB83 (SEQ ID NO: 59) and AB60 (SEQ ID NO: 57), AB82 (SEQ ID NO: 58) and AB60, AB54 (SEQ ID NO: 56) and AB60, and GH46 (SEQ ID NO: 67) and CRX37 (SEQ ID NO: 73); the first panel of oligonucleotide probes consists of probes CRX33 (SEQ ID NO: 69), GH104 (SEQ ID NO: 90). GH56 (SEQ ID NO: 86), GH59 (SEQ ID NO: 87), CRX49 (SEQ ID NO: 74), GH102 (SEQ ID NO: 89), GH111(SEQ ID NO: 92), CRX34 (SEQ ID NO: 70), GH122 (SEQ ID NO: 93), CRX61(SEQ ID NO: 80), CRX23 (SEQ ID NO: 66), CRX06 (SEQ ID NO: 61), CRX35 (SEQ ID NO: 71), CRX68 (SEQ ID NO: 84), and CRX62 (SEQ ID NO: 81); and the second panel is selected from the group consisting of panels GH104 (SEQ ID NO: 90), DRB63 (SEQ ID NO: 140), DRB113 (SEQ ID NO: 189), CRX35 (SEQ ID NO: 71), CRX57 (SEQ ID NO: 78), CRX56 (SEQ ID NO: 77), DRB196 (SEQ ID NO: 272), DRB197 (SEQ ID NO: 273), DRB215 (SEQ ID NO: 291), and DRB216 (SEQ ID NO: 292); CRX50 (SEQ ID NO: 75), GH125 (SEQ ID NO: 94), DRB180 (SEQ ID NO: 256), GH122 (SEQ ID NO: 93), CRX61 (SEQ ID NO: 80), CRX23 (SEQ ID NO: 66), CRX06 (SEQ ID NO: 61), CRX62 (SEQ ID NO: 81), CRX68 (SEQ ID NO: 84), CRX35 (SEQ ID NO: 71), CRX04 (SEQ ID NO: 60), CRX57 (SEQ ID NO: 78), CRX56 (SEQ ID NO: 77), and GH56 (SEQ ID NO: 86); CRX61 (SEQ ID NO: 80), CRX64 (SEQ ID NO: 83), CRX04 (SEQ ID NO: 60), CRX15 (SEQ ID NO: 64), CRX06 (SEQ ID NO: 61), CRX57 (SEQ ID NO: 78), CRX56 (SEQ ID NO: 77), GH59 (SEQ ID NO: 87); and CRX33 (SEQ ID NO: 69), CRX04 (SEQ ID NO: 60), CRX06 (SEQ ID NO: 61), CRX57 (SEQ ID NO: 78), DRB181 (SEQ ID NO: 257), CRX56 (SEQ ID NO: 77), GH102 (SEQ ID NO 89), GH54 (SEQ ID NO: 85), CRX63 (SEQ ID NO: 82), CRX35 (SEQ ID NO: 71).

13. The method of anyone of claims 1 to 12, wherein said first and second panels of oligonucleotide probes are fixed to a solid support, each probe located in a discrete and distinct location on said solid support.

14. A kit for performing the method of claim 2, which kit comprises the primers GH46 (SEQ ID NO: 67) and GH50 (SEQ ID NO: 68) used in step (a) and the primer pairs GH46 (SEQ ID NO: 67) and CRX37 (SEQ ID NO: 73) or GH46 (SEQ ID NO: 67) and DRB30 (SEQ ID NO: 107) used in step (c).

15. The kit of claim 14, which kit comprises the primers GH46 (SEQ ID NO: 67), GH50 (SEQ ID NO: 68), and CRX37 (SEQ ID NO: 73), two or more oligonucleotide probes selected from the group consisting of probes CRX60 (SEQ ID NO: 79), GH105 (SEQ ID NO: 91), GH104 (SEQ ID NO: 90), GH59 (SEQ ID NO: 57), CRX06 (SEQ ID NO: 61), GH122 (SEQ ID NO: 93), CRX23 (SEQ ID NO: 66), CRX35 (SEQ ID NO: 71), CRX49 (SEQ ID NO: 74), GH102 (SEQ ID NO: 89), GH111(SEQ ID NO: 92), CRX34 (SEQ ID NO: 70), CRX04 (SEQ ID NO: 60), GH56 (SEQ ID NO: 86), CRX68 (SEQ ID NO: 84), and CRX12 (SEQ ID NO: 63), and two or more probes selected from the group consisting of probes GH125 (SEQ ID NO: 94), CRX50 (SEQ ID NO: 68), CRX53 (SEQ ID NO: 76), CRX15 (SEQ ID NO: 64), CRX62 (SEQ ID NO: 81), CRX63 (SEQ ID NO: 82), CRX61 (SEQ ID NO: 80), GH54 (SEQ ID NO: 85), CRX56 (SEQ ID NO: 77), CRX57 (SEQ ID NO: 78), and CRX12 (SEQ ID NO: 63).

16. The kit of claim 15, which kit comprises the primers GH46 (SEQ ID NO: 67), GH50 (SEQ ID NO: 68), and CRX37 (SEQ ID NO: 73), a first panel of oligonucleotide probes comprising probes CRX60 (SEQ ID NO: 79), GH105 (SEQ ID NO: 91), GH104 (SEQ ID NO: 90), GH59 (SEQ ID NO: 57), CRX06 (SEQ ID NO: 61), GH122 (SEQ ID NO: 93), CRX23 (SEQ ID NO: 66), CRX35 (SEQ ID NO: 71), CRX49 (SEQ ID NO: 74), GH102 (SEQ ID NO: 89), GH111 (SEQ ID NO: 92), CRX34 (SEQ ID NO: 70), CRX04 (SEQ ID NO: 60), GH56 (SEQ ID NO: 86), CRX68 (SEQ ID NO: 84), and CRX12 (SEQ ID NO: 63), and a second panel of oligonucleotide probes comprising probes GH125 (SEQ ID NO: 94), CRX50 (SEQ ID NO: 68), CRX53 (SEQ ID NO: 76), CRX15 (SEQ ID NO: 64), CRX62 (SEQ ID NO: 81), CRX63 (SEQ ID NO: 82), CRX61 (SEQ ID NO: 80), GH54 (SEQ ID NO: 85), CRX56 (SEQ ID NO: 77), CRX57 (SEQ ID NO: 78), and CRX12 (SEQ ID NO: 63).

## Patentansprüche

1. Eine Methode zur Bestimmung der Nukleinsäuren vom Typ DRbeta DNA in einer Probe, wobei die Methode umfasst:
(a) Amplifikation aller Nukleinsäuren in der Probe, die ein zweites Exon des DRbeta-Gens enthalten unter Verwendung des Primerpaares GH46 (SEQ ID NO:67) und GH50 (SEQ ID NO:68);
(b) Hybridisierung der gemäss Schritt (a) amplifizierten Nukleinsäure an eine erste Auswahl von Oligonukleotidsonden unter Bedingungen bei denen die genannten Sonden nur zu exakt komplementären Sequenzen hybridisieren, die mehr als zehn Nukleotide lang sind;
(c) Amplifikation einer spezifischen Untergruppe von Nukleinsäuren in der Probe, die die zweite Exonsequenz des DRbeta Genes enthalten unter Verwendung der Primerpaare GH46 (SEQ ID NO:67) und CRX37 (SEQ ID NO:73) oder GH46 (SEQ ID NO:67 und DRB30 (SEQ ID NO:107);
(d) Hybridisierung der in Schritt (c) amplifizierten Nukleinsäure mit einer zweiten Auswahl von Oligonukleotidsonden unter Bedingungen bei denen die genannten Sonden nur zu exakt komplementären Sequenzen hybridisieren, die mehr als zehn Nukleotide lang sind, und
(e) Bestimmung der DRbeta Allele von denen die DRbeta DNA in der Probe herstammt aus dem Muster der Sondenhybridisierung in den Schritten (b) und (d).

2. Die Methode gemäss Anspruch 1, worin die Amplifikation in den Schritten (a) und (c) mittels einer Polymerase-Kettenreaktion ausgeführt wird.

3. Die Methode gemäss Anspruch 1 oder 2, worin die erste und die zweite Auswahl von Oligonukleotidsonden je zwei oder mehrere Sonden umfasst und worin die Sonden an die zweite Exonsequenz des DRbeta Genes hybridisieren können, wobei die Sequenzen ausgewählt sind aus der Gruppe bestehend aus den Sequenzen, die die Aminosäurereste 9 bis 16, die Aminosäurereste 25 bis 34, die Aminosäurereste 67 bis 74 und den Aminosäurerest 86 kodieren.

4. Die Methode gemäss Anspruch 3, worin die erste Auswahl von Oligonukleotidsonden zwei oder mehrere Sonden umfasst, die ausgewählt sind aus der Gruppe bestehend aus den Sonden CRX60 (SEQ ID NO:79), GH105 (SEQ ID NO:91), GH104 (SEQ ID NO:90), GH59 (SEQ ID NO:57), CRX06 (SEQ ID NO:61), GH122 (SEQ ID NO:93), CRX23 (SEQ ID NO:66), CRX35 (SEQ ID NO:71), CRX49 (SEQ ID NO:74), GH102 (SEQ ID NO:89), GH111 (SEQ ID NO:92), CRX34 (SEQ ID NO:70), CRX04 (SEQ ID NO:60), GH56 (SEQ ID NO:86), CRX68 (SEQ ID NO:84) und CRX12 (SEQ ID NO:63).

5. Die Methode gemäss Anspruch 3, worin die zweite Auswahl von Oligonukleotidsonden zwei oder mehrere Sonden umfasst, die ausgewählt sind aus der Gruppe bestehend aus den Sonden GH125 (SEQ ID NO:94), CRX50 (SEQ ID NO:68), CRX53 (SEQ ID NO:76), CRX15 (SEQ ID NO:64), CRX62 (SEQ ID NO:81), CRX63 (SEQ ID NO:82), CRX61 (SEQ ID NO:80), GH54 (SEQ ID NO:85), CRX56 (SEQ ID NO:77), CRX57 (SEQ ID NO:78) und CRX12 (SEQ ID NO:63).

6. Die Methode gemäss Anspruch 3, worin die Primer die in Schritt (a) verwendet werden GH46 (SEQ ID NO:67) und GH50 (SEQ ID NO:68) sind, die Primer die in Schritt (c) verwendet werden GH46 und CRX37 (SEQ ID NO:73) sind, die erste Auswahl von Oligonukleotidsonden die Sonden CRX60 (SEQ ID NO:79), GH105 (SEQ ID NO:91), GH104 (SEQ ID NO:90), GH59 (SEQ ID NO:57), CRX06 (SEQ ID NO:61), GH122 (SEQ ID NO:93), CRX23 (SEQ ID NO:66), CRX35 (SEQ ID NO:71), CRX49 (SEQ ID NO:74), GH102 (SEQ ID NO:89), GH111 (SEQ ID NO:92), CRX34 (SEQ ID NO:70), CRX04 (SEQ ID NO:60), GH56 (SEQ ID NO: 86), CRX68 (SEQ ID NO:84) und CRX12 (SEQ ID NO:63) umfasst und die zweite Auswahl von Oligonukleotidsonden die Sonden GH125 (SEQ ID NO:94), CRX50 (SEQ ID NO:68), CRX53 (SEQ ID NO:76), CRX15 (SEQ ID NO:64), CRX62 (SEQ ID NO:81), CRX63 (SEQ ID NO:82), CRX61 (SEQ ID NO:80), GH54 (SEQ ID NO:85), CRX56 (SEQ ID NO:77), CRX57 (SEQ ID NO:78) und CRX12 (SEQ ID NO:63) umfasst.

7. Die Methode gemäss Anspruch 1, worin, die genannte erste Auswahl von Sonden zwei oder mehrere Sonden umfasst, die ausgewählt sind aus der Gruppe bestehend aus den Sonden DRB01 (SEQ ID NO:79), GH104 (SEQ ID NO:90), DRB46 (SEQ ID NO:123), DRB48 (SEQ ID NO:125), DRB207 (SEQ ID NO:283), GH102 (SEQ ID NO:89), DRB209 (SEQ ID NO:285), DRB20 (SEQ ID NO:98), DRB102 (SEQ ID NO:178), DRB112 (SEQ ID NO:188), DRB07 (SEQ ID NO:84) und DRB42 (SEQ ID NO:119).

8. Die Methode gemäss Anspruch 1, worin, die genannte zweite Auswahl von Sonden zwei oder mehrere Sonden umfasst, die ausgewählt sind aus der Gruppe bestehend aus den Sonden DRB223 (SEQ ID NO:299), DRB37 (SEQ ID NO:144), DRB203 (SEQ ID NO:279), DRB163 (SEQ ID NO:239), DRB118 (SEQ ID NO:194), DRB02 (SEQ ID NO:61), DRB38 (SEQ ID NO:115), DRB222 (SEQ ID NO:298), DRB232 (SEQ ID NO:308), DRB136 (SEQ ID NO:212), DRB198 (SEQ ID NO:274) und DRB42 (SEQ ID NO:119).

9. Die Methode gemäss Anspruch 2, worin die Primer, die in Schritt (a) verwendet werden CRX28 (SEQ ID NO:67) und CRX29 (SEQ ID NO:68) sind, die Primer, die in Schritt (c) verwendet werden CRX28 und CRX37 (SEQ ID NO:73) sind, die erste Auswahl von Oligonukleotidsonden die Sonden DRB01 (SEQ ID NO:79), GH104 (SEQ ID NO:90), DRB46 (SEQ ID NO:123), DRB48 (SEQ ID NO:125), DRB207 (SEQ ID NO:283), GH102 (SEQ ID NO:89), DRB209 (SEQ ID NO:285), DRB20 (SEQ ID NO:98), DRB102 (SEQ ID NO:178), DRB112 (SEQ ID NO:188), DRB07 (SEQ ID NO:84) und DRB42 (SEQ ID NO:119) umfasst und die zweite Auswahl von Oligonukleotidsonden die Sonden DRB223 (SEQ ID NO:299), DRB37 (SEQ ID NO:144), DRB203 (SEQ ID NO:279), DRB163 (SEQ ID NO:239), DRB118 (SEQ ID NO:194), DRB02 (SEQ ID NO:61), DRB38 (SEQ ID NO:115), DRB222 (SEQ ID NO:298), DRB232 (SEQ ID NO:308), DRB136 (SEQ ID NO:212), DRB198 (SEQ ID NO:274) und DRB42 (SEQ ID NO:119) umfasst.

10. Die Methode gemäss Anspruch 1, worin die zweite Auswahl der Sonden zwei oder mehrere Sonden umfasst, die ausgewählt sind aus einer dritten Auswahl von Sonden, wobei die genannte dritte Auswahl ausgewählt ist aus der Gruppe bestehend aus den Auswahlen GH104 (SEQ ID NO:90), DRB63 (SEQ ID NO:140), DRB113 (SEQ ID NO:189), CRX35 (SEQ ID NO:71), CRX57 (SEQ ID NO:78), CRX56 (SEQ ID NO:77), DRB196 (SEQ ID NO:272), DRB197 (SEQ ID NO:273), DRB215 (SEQ ID NO:291) und DRB216 (SEQ ID NO:292); CRX50 (SEQ ID NO:75), GH125 (SEQ ID NO:94), DRB180 (SEQ ID NO:256), GH122 (SEQ ID NO:93), CRX61 (SEQ ID NO:80), CRX23 (SEQ ID NO:66), CRX06 (SEQ ID NO:61), CRX62 (SEQ ID NO:81), CRX68 (SEQ ID NO:84), CRX35 (SEQ ID NO:71), CRX04 (SEQ ID NO:60), CRX57 (SEQ ID NO:78), CRX56 (SEQ ID NO:77) und GH56 (SEQ ID NO:86); CRX61 (SEQ ID NO:80), CRX64 (SEQ ID NO:83), CRX04 (SEQ ID NO:60), CRX15 (SEQ ID NO:64), CRX06 (SEQ ID NO:61), CRX57 (SEQ ID NO:78), CRX56 (SEQ ID NO:77), GH59 (SEQ ID NO:87); und CRX33 (SEQ ID NO:69), CRX04 (SEQ ID NO:60), CRX06 (SEQ ID NO:61), CRX57 (SEQ ID NO:78), DRB181 (SEQ ID NO:257), CRX56 (SEQ ID NO:77), GH102 (SEQ ID NO:89), GH54 (SEQ ID NO:85), CRX63 (SEQ ID NO:82), CRX35 (SEQ ID NO:71).

11. Die Methode gemäss Anspruch 2, worin die Primer, die in Schritt (a) verwendet werden, GH46 (SEQ ID NO:67) und GH50 (SEQ ID NO:68) sind; die Primer, die in Schritt (c) verwendet werden, aus der Gruppe bestehend aus AB83 (SEQ ID NO:59) und AB60 (SEQ ID NO:57), AB82 (SEQ ID NO:58) und AB60, AB54 (SEQ ID NO :56) und AB60 und GH46 (SEQ ID NO:67) und CRX37 (SEQ ID NO:73) ausgewählt sind; die erste Auswahl der Oligonukleotidsonden zwei oder mehrere Sonden umfasst, die ausgewählt sind aus der Gruppe bestehend aus den Sonden CRX33 (SEQ ID NO:69), GH104 (SEQ ID NO:90), GH56 (SEQ ID NO:86), GH59 (SEQ ID NO:87); CRX49 (SEQ ID NO:74), GH102 (SEQ ID NO:89), GH111 (SEQ ID NO:92), CRX34 (SEQ ID NO:70), GH122 (SEQ ID NO:93), CRX61 (SEQ ID NO:80), CRX23 (SEQ ID NO:66), CRX06 (SEQ ID NO:61), CRX35 (SEQ ID NO:71), CRX68 (SEQ ID NO:84) und CRX62 (SEQ ID NO:81); und die zweite Auswahl der Sonden zwei oder mehrere Sonden umfasst, die ausgewählt sind aus einer dritten Auswahl von Sonden, wobei die genannte dritte Auswahl ausgewählt ist aus der Gruppe bestehend aus den Auswahlen GH104 (SEQ ID NO: 90), DRB63 (SEQ ID NO: 140), DRB113 (SEQ ID NO: 189), CRX35 (SEQ ID NO: 71), CRX57 (SEQ ID NO: 78), CRX56 (SEQ ID NO: 77), DRB196 (SEQ ID NO: 272), DRB197 (SEQ ID NO: 273), DRB215 (SEQ ID NO: 291), und DRB216 (SEQ ID NO: 292); CRX50 (SEQ ID NO: 75), GH125 (SEQ ID NO: 94), DRB 180 (SEQ ID NO: 256), GH122 (SEQ ID NO: 93), CRX61 (SEQ ID NO: 80), CRX23 (SEQ ID NO: 66), CRX06 (SEQ ID NO: 61), CRX62 (SEQ ID NO: 81), CRX68 (SEQ ID NO: 84), CRX35 (SEQ ID NO: 71), CRX04 (SEQ ID NO: 60), CRX57 (SEQ ID NO: 78), CRX56 (SEQ ID NO: 77), und GH56 (SEQ ID NO: 86); CRX61 (SEQ ID NO: 80), CRX64 (SEQ ID NO: 83), CRX04 (SEQ ID NO: 60), CRX15 (SEQ ID NO: 64), CRX06 (SEQ ID NO: 61), CRX57 (SEQ ID NO: 78), CRX56 (SEQ ID NO: 77), GH59 (SEQ ID NO: 87); und CRX33 (SEQ ID NO: 69), CRX04 (SEQ ID NO: 60), CRX06 (SEQ ID NO: 61), CRX57 (SEQ ID NO: 78), DRB181 (SEQ ID NO: 257), CRX56 (SEQ ID NO: 77), GH102 (SEQ ID NO: 89), GH54 (SEQ ID NO: 85), CRX63 (SEQ ID NO: 82), CRX35 (SEQ ID NO: 71).

12. Die Methode gemäss Anspruch 2, worin die Primer, die in Schritt (a) verwendet werden GH46 (SEQ ID NO:67) und GH50 (SEQ ID NO:68) sind; die Primer, die in Schritt (c) verwendet werden, aus der Gruppe bestehend aus AB83 (SEQ ID NO:59) und AB60 (SEQ ID NO:57), AB82 (SEQ ID NO:58) und AB60, AB54 (SEQ ID NO:56) und AB60 und GH46 (SEQ ID NO:67) und CRX37 (SEQ ID NO:73) ausgewählt sind; die erste Auswahl von Oligonukleotidsonden aus den Proben CRX33 (SEQ ID NO:69), GH104 (SEQ ID NO:90), GH56 (SEQ ID NO:86), GH59 (SEQ ID NO:87), CRX49 (SEQ ID NO:74), GH102 (SEQ ID NO:89), GH111 (SEQ ID NO:92), CRX34 (SEQ ID NO:70), GH122 (SEQ ID NO:93 ), CRX61 (SEQ ID NO:80), CRX23 (SEQ ID NO.66), CRX06 (SEQ ID NO:61), CRX35 (SEQ ID NO:71), CRX68 (SEQ ID NO:84) und CRX62 (SEQ ID NO:81) besteht; und die zweite Auswahl ausgewählt ist aus der Gruppe bestehend aus den Auswahlen GH104 (SEQ ID NO:90), DRB63 (SEQ ID NO:140), DRB113 (SEQ ID NO:189); CRX35 (SEQ ID NO:71), CRX57 (SEQ ID NO:78), CRX56 (SEQ ID NO:77), DRB196 (SEQ ID NO:272), DRB197 (SEQ ID NO:273), DRB215 (SEQ ID NO:291) und DRB216 (SEQ ID NO:292); CRX50 (SEQ ID NO:75), GH125 (SEQ ID NO:94), DRB180 (SEQ ID NO:256), GH122 (SEQ ID NO:93), CRX61 (SEQ ID NO:80), CRX23 (SEQ ID NO:66), CRX06 (SEQ ID NO:61), CRX62 (SEQ ID NO:81), CRX68 (SEQ ID NO:84), CRX35 (SEQ ID NO:71), CRX04 (SEQ ID NO:60), CRX57 (SEQ ID NO:78), CRX56 (SEQ ID NO:77) und GH56 (SEQ ID NO:86); CRX61 (SEQ ID NO:80), CRX64 (SEQ ID NO:83), CRX04 (SEQ ID NO:60), CRX15 (SEQ ID NO:64), CRX06 (SEQ ID NO:61), CRX57 (SEQ ID NO:78); CRX56 (SEQ ID NO:77), GH59 (SEQ ID NO:87); und CRX33 (SEQ ID NO:69), CRX04 (SEQ ID NO:60), CRX06 (SEQ ID NO:61), CRX57 (SEQ ID NO:78), DRB181 (SEQ ID NO:257), CRX56 (SEQ ID NO:77), GH102 (SEQ ID NO:89), GH54 (SEQ ID NO:85), CRX63 (SEQ ID NO:82), CRX35 (SEQ ID NO:71).

13. Die Methode gemäss einem der Ansprüche 1 bis 12, worin die genannten erste und zweite Auswahl von Sonden an einem festen Träger gebunden sind, wobei jede Sonde an einer bestimmten unterscheidbaren Stelle auf den genannten festen Träger fixiert ist.

14. Ein Kit zur Durchführung der Methode gemäss Anspruch 2, wobei das Kit die Primer GH46 (SEQ ID NO:67) und GH50 (SEQ ID NO:68), die in Schritt (a) gebraucht werden, und die Primerpaare GH46 (SEQ ID NO:67) und CRX37 (SEQ ID NO:73) oder GH46 (SEQ ID NO:67) und DRB30 (SEQ ID NO:107), die in Schritt (c) gebraucht werden, umfasst.

15. Das Kit gemäss Anspruch 14, wobei das Kit die Primer GH46 (SEQ ID NO:67), GH50 (SEQ ID NO:68) und CRX37 (SEQ ID NO:73) umfasst, sowie zwei oder mehrere Oligonukleotidsonden, die ausgewählt sind aus der Gruppe bestehend aus den Sonden CRX60 (SEQ ID NO:79), GH105 (SEQ ID NO:91), GH104 (SEQ ID NO:90), GH59 (SEQ ID NO:57), CRX06 (SEQ ID NO:61), GH122 (SEQ ID NO:93), CRX23 (SEQ ID NO:66), CRX35 (SEQ ID NO:71), CRX49 (SEQ ID NO:74), GH102 (SEQ ID NO:89); GH111 (SEQ ID NO:92), CRX34 (SEQ ID NO:70), CRX04 (SEQ ID NO:60), GH56 (SEQ ID NO:86), CRX68 (SEQ ID NO:84) und CRX12 (SEQ ID NO:63) und zwei oder mehrere Sonden, die ausgewählt sind aus der Gruppe bestehend aus den Sonden GH125 (SEQ ID NO:94), CRX50 (SEQ ID NO:68), CRX53 (SEQ ID NO:76), CRX15 (SEQ ID NO:64), CRX62 (SEQ ID NO:81), CRX63 (SEQ ID NO:82), CRX61(SEQ ID NO:80), GH54 (SEQ ID NO:85), CRX56 (SEQ ID NO:77), CRX57 (SEQ ID NO:78) und CRX12 (SEQ ID NO:63).

16. Das Kit gemäss Anspruch 15, wobei das Kit die Primer GH46 (SEQ ID NO:67), GH50 (SEQ ID NO:68) und CRX37 (SEQ ID NO:73) umfasst, sowie eine erste Auswahl von Oligonukleotidsonden bestehend aus den Sonden CRX60 (SEQ ID NO:79), GH105 (SEQ ID NO:91), GH104 (SEQ ID NO:90), GH59 (SEQ ID NO:57), CRX06 (SEQ ID NO:61), GH122 (SEQ ID NO:93), CRX23 (SEQ ID NO:66), CRX35 (SEQ ID NO:71), CRX49 (SEQ ID NO:74), GH102 (SEQ ID NO:89), GH111(SEQ ID NO:92), CRX34 (SEQ ID NO:70), CRX04 (SEQ ID NO:60), GH56 (SEQ ID NO:86), CRX68 (SEQ ID NO:84) und CRX12 (SEQ ID NO:63) und eine zweite Auswahl von Oligonukleotidsonden bestehend aus den Sonden GH125 (SEQ ID NO:94), CRX50 (SEQ ID NO:68), CRX53 (SEQ ID NO:76), CRX15 (SEQ ID NO:64), CRX62 (SEQ ID NO:81), CRX63 (SEQ ID NO:82), CRX61 (SEQ ID NO:80), GH54 (SEQ ID NO:85), CRX56 (SEQ ID NO:77), CRX57 (SEQ ID NO:78) und CRX12 (SEQ ID NO:63).

## Revendications

1. Procédé pour déterminer le type d'acide nucléique de l'ADN du DRβ dans un échantillon, lequel procédé consiste :
(a) à amplifier tous acides nucléiques se trouvant dans l'échantillon et qui contiennent un deuxième exon du gène DRβ, par utilisation de la paire d'amorces GH46 (SEQ ID NO:67) et GH50 (SEQ ID NO:68) ;
(b) à hybrider ledit acide nucléique amplifié dans l'étape (a) sur un premier groupe de sondes oligonucléotidiques, dans des conditions telles que lesdites sondes ne s'hybrident qu'à des séquences exactement complémentaires ayant une longueur supérieure à dix nucléotides ;
(c) à amplifier un sous-ensemble spécifique d'acides nucléiques se trouvant dans l'échantillon, qui contiennent les séquences du deuxième exon du gène DRβ, par utilisation des paires d'amorces GH46 (SEQ ID NO:67) et CRX37 (SEQ ID NO:73), ou GH46 (SEQ ID NO:67) et DRB30 (SEQ ID NO:107) ;
(d) à hybrider ledit acide nucléique amplifié dans l'étape (c) avec un deuxième groupe de sondes oligonucléotidiques dans des conditions telles que lesdites sondes ne s'hybrident qu'à des séquences exactement complémentaires ayant une longueur supérieure à dix nucléotides ; et
(e) à déterminer, à partir de la configuration de l'hybridation des sondes des étapes (b) et (d), les allèles DRβ dont provient l'ADN de DRβ dudit échantillon.

2. Procédé selon la revendication 1, dans lequel l'amplification des étapes (a) et (c) est réalisée par l'amplification en chaîne par polymérase.

3. Procédé selon les revendications 1 ou 2, dans lequel ledit premier et ledit deuxième groupes de sondes oligonucléotidiques comprennent chacune deux sondes ou plus, et où lesdites sondes peuvent s'hybrider à des séquences du deuxième exon du gène DRβ, ces séquences étant sélectionnées dans l'ensemble constitué des séquences qui codent les résidus d'acides aminés 9 à 16, les résidus d'acides aminés 25 à 34, les résidus d'acides aminés 67 à 74 et le résidu d'acide aminé 86.

4. Procédé selon la revendication 3, dans lequel le premier groupe de sondes oligonucléotidiques comprend au moins deux sondes choisies parmi l'ensemble constitué des sondes CRX60 (SEQ ID NO:79), GH105 (SEQ ID NO:91), GH104 (SEQ ID NO:90), GH59 (SEQ ID NO:57), CRX06 (SEQ ID NO:61), GH122 (SEQ ID NO:93), CRX23 (SEQ ID NO:66), CRX35 (SEQ ID NO:71), CRX49 (SEQ ID NO:74), GH102 (SEQ ID NO:89), GH111 (SEQ ID NO:92), CRX34 (SEQ ID NO:70), CRX04 (SEQ ID NO:60), GH56 (SEQ ID NO:86), CRX68 (SEQ ID NO:84) et CRX12 (SEQ ID NO:63).

5. Procédé selon la revendication 3, dans lequel le deuxième groupe de sondes oligonucléotidiques comprend au moins deux sondes choisies parmi l'ensemble constitué des sondes GH125 (SEQ ID NO:94), CRX5O (SEQ ID NO:68 ), CRX53 (SEQ ID NO:76), CRX15 (SEQ ID NO:64), CRX62 (SEQ ID NO:81), CRX63 (SEQ ID NO:82), CRX61(SEQ ID NO:80), GH54 (SEQ ID NO:85), CRX56 (SEQ ID NO:77), CRX57 (SEQ IDNO:78) et CRX12 (SEQ ID NO:63).

6. Procédé selon la revendication 3, dans lequel les amorces utilisées dans l'étape (a) sont GH46 (SEQ ID NO:67) et GH50 (SEQ ID NO:68), les amorces utilisées dans l'étape (c) sont GH46 et CRX37 (SEQ ID NO:73), le premier groupe de sondes oligonucléotidiques comprend les sondes CRX60 (SEQ ID NO:79), GH105 (SEQ ID NO:91), GH104 (SEQ ID NO:90), GH59 (SEQ ID NO:57), CRX06 (SEQ ID NO:61), GH122 (SEQ ID NO:93), CRX23 (SEQ ID NO:66), CRX35 (SEQ ID NO:71), CRX49 (SEQ ID NO:74), GH102 (SEQ ID NO:89), GH111 (SEQ ID NO:92), CRX34 (SEQ ID NO:70), CRX04 (SEQ ID NO:60), GH56 (SEQ ID NO:86), CRX68 (SEQ ID NO:84), et CRX12 (SEQ ID NO:63), et le deuxième groupe de sondes oligonucléotidiques comprend les sondes GH125 (SEQ ID NO:94), CRXSO (SEQ ID NO:68), CRX53 (SEQ ID NO:76), CRX15 (SEQ ID NO:64), CRX62 (SEQ ID NO:81), CRX63 (SEQ ID NO:82), CRX61 (SEQ ID NO:80), GH54 (SEQ ID NO:85), CRX56 (SEQ ID NO:77), CRX57 (SEQ ID NO:78), et CRX12 (SEQ ID NO:63).

7. Procédé selon la revendication 1, dans lequel ledit premier groupe de sondes comprend au moins deux sondes choisies parmi l'ensemble constitué des sondes DRB01 (SEQ ID NO:79), GH104 (SEQ ID NO:90), DRB46 (SEQ ID NO:123), DRB48 (SEQ ID NO: 125), DRB207 (SEQ ID NO:283), GH102 (SEQ ID NO:89), DRB209 (SEQ ID NO:285), DRB20 (SEQ ID NO:98), DRB102 (SEQ ID NO:178), DRB112 (SEQ ID NO:188), DRB07 (SEQ ID NO:84), et DRB42 (SEQ ID NO:119).

8. Procédé selon la revendication 1, dans lequel ledit deuxième groupe de sondes comprend au moins deux sondes choisies parmi l'ensemble constitué des sondes DRB223 (SEQ ID NO:299), DRB37 (SEQ ID NO:144), DRB203 (SEQ ID NO:279), DRB163 (SEQ ID NO:239), DRB118 (SEQ ID NO:194), DRB02 (SEQ ID NO:61), DRB38 (SEQ ID NO:115), DRB222 (SEQ ID NO:298), DRB232 (SEQ ID NO:308), DRB136 (SEQ ID NO:212), DRB198 (SEQ ID NO:274) et DRB42 (SEQ ID NO:119).

9. Procédé selon la revendication 2, dans lequel les amorces utilisées dans l'étape (a) sont CRX28 (SEQ ID NO:67) et CRX29 (SEQ ID NO:68), les amorces utilisées dans l'étape (c) sont CRX28 et CRX37 (SEQ ID NO:73), le premier groupe de sondes oligonucléotidiques comprend des sondes DRB01 (SEQ ID NO:79), GH104 (SEQ ID NO:90), DRB46 (SEQ ID NO:123), DRB48 (SEQ ID NO:125), DRB207 (SEQ ID NO:283), GH102 (SEQ ID NO:89), DRB209 (SEQ ID NO:285), DRB20 (SEQ ID NO:98), DRB102 (SEQ ID NO:178), DRB112 (SEQ ID NO:188), DRB07 (SEQ ID NO:84) et DRB42 (SEQ ID NO:119), et le deuxième groupe de sondes oligonucléotidiques comprend les sondes DRB223 (SEQ ID NO:299), DRB37 (SEQ ID NO:144), DRB203 (SEQ ID NO:279), DRB163 (SEQ ID NO:239), DRB118 (SEQ ID NO:194), DRB02 (SEQ ID NO:61), DRB38 (SEQ ID NO:115), DRB222 (SEQ ID NO:298), DRB232 (SEQ ID NO:308), DRB136 (SEQ ID NO:212), DRB198 (SEQ ID NO:274) et DRB42 (SEQ ID NO:119).

10. Procédé selon la revendication 1, dans lequel le deuxième groupe de sondes comprend au moins deux sondes choisies parmi un troisième groupe de sondes, où ledit troisième groupe est choisi parmi l'ensemble constitué des sondes GH104 (SEQ ID NO:90), DRB63 (SEQ ID NO:140), DRB113 (SEQ ID NO:189), CRX35 (SEQ ID NO:71), CRX57 (SEQ ID NO:78), CRX56 (SEQ ID NO:77), DRB196 (SEQ ID NO:272), DRB197 (SEQ ID NO:273), DRB215 (SEQ ID NO:291), et DRB216 (SEQ ID NO:292) ; CRX5O (SEQ ID NO:75), GH125 (SEQ ID NO:94), DRB180 (SEQ ID NO:256), GH122 (SEQ ID NO:93), CRX61 (SEQ ID NO:80), CRX23 (SEQ ID NO:66), CRX06 (SEQ ID NO:61), CRX62 (SEQ ID NO:81), CRX68 (SEQ ID NO:84), CRX35 (SEQ ID NO:71), CRX04 (SEQ ID NO:60), CRX57 (SEQ ID NO:78), CRX56 (SEQ ID NO:77) et GH56 (SEQ ID NO:86) ; CRX61 (SEQ ID NO:80), CRX64 (SEQ ID NO:83), CRX04 (SEQ ID NO:60), CRX15 (SEQ ID NO:64), CRX06 (SEQ ID NO:61), CRX57 (SEQ ID NO:78), CRX56 (SEQ ID NO:77), GH59 (SEQ ID NO:87) ; et CRX33 (SEQ ID NO:69), CRX04 (SEQ ID NO:60), CRX06 (SEQ ID NO:61), CRX57 (SEQ ID NO:78), DRB181 (SEQ ID NO:257), CRX56 (SEQ ID NO:77), GH102 (SEQ ID NO:89), GH54 (SEQ ID NO:85), CRX63 (SEQ ID NO:82), CRX35 (SEQ ID NO:71).

11. Procédé selon la revendication 2, dans lequel les amorces utilisées dans l'étape (a) sont GH46 (SEQ ID NO:67) et GH50 (SEQ ID NO:68) ; les amorces utilisées dans l'étape (c) sont choisies parmi l'ensemble constitué d'AB83 (SEQ ID NO:59) et AB60 (SEQ ID NO:57), AB82 (SEQ ID NO:58) et AB60, AB54 (SEQ ID NO:56) et AB60, et GH46 (SEQ ID NO:67) et CRX37 (SEQ ID NO:73) ; le premier groupe de sondes oligonucléotidiques comprend au moins deux sondes choisies parmi l'ensemble constitué des sondes CRX33 (SEQ ID NO:69), GH104 (SEQ ID NO:90), GH56 (SEQ ID NO:86), GH59 (SEQ ID NO:87), CRX49 (SEQ ID NO:74), GH102 (SEQ ID NO:89), GH111 (SEQ ID NO:92), CRX34 (SEQ ID NO:70), GH122 (SEQ ID NO:93), CRX61 (SEQ ID NO:80), CRX23 (SEQ ID NO:66), CRX06 (SEQ ID NO:61), CRX35 (SEQ ID NO:71), CRX68 (SEQ ID NO:84), et CRX62 (SEQ ID NO:81) ; et le deuxième groupe de sondes comprend au moins deux sondes choisies parmi un troisième groupe de sondes, où ledit troisième groupe est choisi parmi l'ensemble comprenant les sondes GH104 (SEQ ID NO:90), DRB63 (SEQ ID NO:140), DRB113 (SEQ ID NO:189), CRX35 (SEQ ID NO:71), CRX57 (SEQ ID NO:78), CRX56 (SEQ ID NO:77), DRB196 (SEQ ID NO:272), DRB197 (SEQ ID NO:273), DRB215 (SEQ ID NO:291), et DRB216 (SEQ ID NO:292), CRX5O (SEQ ID NO:75), GH125 (SEQ ID NO:94), DRB180 (SEQ ID NO:256), GH122 (SEQ ID NO:93), CRX61 (SEQ ID NO:80), CRX23(SEQ ID NO:66), CRX06 (SEQ ID NO:61), CRX62 (SEQ ID NO:81), CRX68 (SEQ ID NO:84), CRX35 (SEQ ID NO:71), CRX04 (SEQ ID NO:60), CRX57 (SEQ ID NO:78), CRX56 (SEQ ID NO:77) et GH56 (SEQ ID NO:86), CRX61 (SEQ ID NO:80), CRX64 (SEQ ID NO:83), CRX04 (SEQ ID NO:60), CRX15 (SEQ ID NO:64), CRX06 (SEQ ID NO:61), CRX57 (SEQ ID NO:78), CRX56 (SEQ ID NO:77), GH59 (SEQ ID NO:87) ; et CRX33 (SEQ ID NO:69), CRX04 (SEQ ID NO:60), CRX06 (SEQ ID NO:61), CRX57 (SEQ ID NO:78), DRB181 (SEQ ID NO:257), CRX56 (SEQ ID NO:77), GH102 (SEQ ID NO:89), GH54 (SEQ ID NO:85), CRX63 (SEQ ID NO:82), CRX35 (SEQ ID NO:71).

12. Procédé selon la revendication 2, dans lequel les amorces utilisées dans l'étape (a) sont GH46 (SEQ ID NO:67) et GH50 (SEQ ID NO:68) ; les amorces utilisées dans l'étape (c) sont choisies parmi l'ensemble constitué d'AB83 (SEQ ID NO:59) et AB60 (SEQ ID NO:57), AB82 (SEQ ID NO:58) et AB60, AB54 (SEQ ID NO:56) et AB60, et GH46 (SEQ ID NO:67) et CRX37 (SEQ ID NO:73 ) ; le premier groupe de sondes olinucléotidiques est constitué des sondes CRX33 (SEQ ID NO:69), GH104 (SEQ ID NO:90). GH56 (SEQ ID NO:86), GH59 (SEQ ID NO:87), CRX49 (SEQ ID NO:74), GH102 (SEQ ID NO:89), GH111 (SEQ ID NO:92), CRX34 (SEQ ID NO:70), GH122 (SEQ ID NO:93), CRX61 (SEQ ID NO:80), CRX23 (SEQ ID NO:66), CRX06 (SEQ ID NO:61), CRX35 (SEQ ID NO:71), CRX68 (SEQ ID NO:84) et CRX62 (SEQ ID NO:81) ; et le deuxième groupe est choisi parmi l'ensemble constitué des groupes GH104 (SEQ ID NO:90), DRB63 (SEQ ID NO:140), DRB113 (SEQ ID NO:189), CRX (SEQ ID NO:71), CRX57 (SEQ ID NO:78), CRX56 (SEQ ID NO:77), DRB196 (SEQ ID NO:272), DRB197 (SEQ ID NO:273), DRB215 (SEQ ID NO:291), et DRB216 (SEQ ID NO:292) ; CRXSO (SEQ ID NO:75), GH125 (SEQ ID NO:94), DRB180 (SEQ ID NO:256), GH122 (SEQ ID NO:93), CRX61 (SEQ ID NO:80), CRX23 (SEQ ID NO:66), CRX06 (SEQ ID NO:61), CRX62 (SEQ ID NO:81), CRX68 (SEQ ID NO:84), CRX35 (SEQ ID NO:71), CRX04 (SEQ ID NO:60), CRX57 (SEQ ID NO:78), CRX56 (SEQ ID NO:77), et GH56 (SEQ ID NO:86), CRX61 (SEQ ID NO:80), CRX64 (SEQ ID NO:83), CRX04 (SEQ ID NO:60), CRX15 (SEQ ID NO:64), CRX06 (SEQ ID NO:61), CRX57 (SEQ ID NO:78), CRX56 (SEQ ID NO:77), GH59 (SEQ ID NO:87) ; et CRX33 (SEQ ID NO:69), CRX04 (SEQ ID NO:60), CRX06 (SEQ ID NO:61), CRX57 (SEQ ID NO:78), DRB181 (SEQ ID NO:257), CRX56 (SEQ ID NO:77), GH102 (SEQ ID NO:89), GH54 (SEQ ID NO:85), CRX63 (SEQ ID NO:82), CRX35 (SEQ ID NO:71).

13. Procédé selon l'une quelconque des revendications 1 à 12, dans lequel ledit premier et ledit deuxième groupes de sondes oligonucléotidiques sont fixés à un support solide, chaque sonde étant située en un point discret et distinct sur ledit support solide.

14. Coffret pour mettre en oeuvre le procédé selon la revendication 2, lequel coffret comprend les amorces GH46 (SEQ ID NO:67) et GH50 (SEQ ID NO:68) utilisées dans l'étape (a) et les paires d'amorces GH46 (SEQ ID NO:67) et CRX37 (SEQ ID NO:73) ou GH46 (SEQ ID NO:67) et DRB30 (SEQ ID NO:107) utilisées dans l'étape (c).

15. Coffret selon la revendication 14, lequel coffret comprend les amorces GH46 (SEQ ID NO:67), GH50 (SEQ ID NO:68) et CRX37 (SEQ ID NO:73), au moins deux sondes oligonucléotidiques choisies parmi l'ensemble constitué des sondes CRX60 (SEQ ID NO:79), GH105 (SEQ ID NO:91), GH104 (SEQ ID NO:90), GH59 (SEQ ID NO:57), CRX06 (SEQ ID NO:61), GH122 (SEQ ID NO:93), CRX23 (SEQ ID NO:66), CRX35 (SEQ ID NO:71), CRX49 (SEQ ID NO:74), GH102 (SEQ ID NO:89), GH111 (SEQ ID NO:92), CRX34 (SEQ ID NO:70), CRX04 (SEQ ID NO:60), GH56 (SEQ ID NO:86), CRX68 (SEQ ID NO:84), et CRX12 (SEQ ID NO:63), et au moins deux sondes choisies parmi l'ensemble comprenant les sondes GH125 (SEQ ID NO:94), CRX5O (SEQ ID NO:68), CRX53 (SEQ ID NO:76), CRX15 (SEQ ID NO:64), CRX62 (SEQ ID NO:81), CRX63 (SEQ ID NO:82), CRX61 (SEQ ID NO:80), GH54 (SEQ ID NO:85), CRX56 (SEQ ID NO:77), CRX57 (SEQ ID NO:78) et CRX12 (SEQ ID NO:63).

16. Coffret selon la revendication 15, lequel coffret comprend les amorces GH46 (SEQ ID NO:67), GH50 (SEQ ID NO:68) et CRX37 (SEQ ID NO:73), un premier groupe de sondes oligonucléotidiques comprenant les sondes CRX60 (SEQ ID NO:79), GH105 (SEQ ID NO:91), GH104 (SEQ ID NO:90), GH59 (SEQ ID NO:57), CRX06 (SEQ ID NO:61), GH122 (SEQ ID NO:93), CRX23 (SEQ ID NO:66), CRX35 (SEQ ID NO:71), CRX49 (SEQ ID NO:74), GH102 (SEQ ID NO:89), GH111(SEQ ID NO:92), CRX34 (SEQ ID NO:70), CRX04 (SEQ ID NO:60), GH56 (SEQ ID NO:86), CRX68 (SEQ ID NO:84) et CRX12 (SEQ ID NO:63), et un deuxième groupe de sondes oligonucléotidiques comprenant les sondes GH125 (SEQ ID NO:94), CRX5O (SEQ ID NO:68), CRX53 (SEQ ID NO:76), CRX15 (SEQ ID NO:64), CRX62 (SEQ ID NO:81), CRX63 (SEQ ID NO:82), CRX61 (SEQ ID NO:80), GH54 (SEQ ID NO:85), CRX56 (SEQ ID NO:77), CRX57 (SEQ ID NO:78), et CRX12 (SEQ ID NO:63).
